(19) Europäisches Patentamt
European Patent Office
Office européen des brevets

(11) EP 1 270 746 A1

(12) EUROPEAN PATENT APPLICATION

(43) Date of publication:
**02.01.2003 Bulletin 2003/01**

(51) Int Cl.[7]: **C12Q 1/68**, C12N 15/10

(21) Application number: **02076171.4**

(22) Date of filing: **16.07.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **20.07.1998 DK 95698**
**29.07.1998 US 94868 P**

(62) Document number(s) of the earlier application(s) in accordance with Art. 76 EPC:
**99932689.5 / 1 098 991**

(71) Applicant: **Inoxell A/S**
**2970 Hoersholm (DK)**

(72) Inventors:
- **Jensen, Allan**
**3480 Fredensborg (DK)**
- **Halkier, Torben**
**2680 Solroed Strand (DK)**
- **Jespersen, Lene**
**3480 Fredensborg (DK)**

(74) Representative: **Koefoed, Peter**
**Kogle Allé 6**
**2970 Horsholm (DK)**

Remarks:
This application was filed on 26 - 03 - 2002 as a divisional application to the application mentioned under INID code 62.

# (54) Methods for the identification of ligand and target biomolecules

(57) The invention provides for methods for identification of biologically active biomolecules. In one aspect, a biologically active biomolecule such as RNA or a peptide is identified by incorporating random nucleotide sequences in a scaffold constituted by an enzyme activity modulator, transforming substantially identical host cells with the construct obtained thereby and screening the transformed cells to identify those where a preselected phenotypic trait has been altered. The randomized DNA is subsequently isolated from the phenotypically altered cells and the peptide and/or RNA encoded by the random sequence is determined. In turn, interaction partners which are putative drug targets are identified and isolated by use of the peptide and/or RNA as part of affinity reagents. A preferred scaffold is derived from the potato inhibitor I family of protease inhibitors and exemplified is the barley chymotrypsin inhibitor 2A (CI-2A). Another aspect relates to the identification of novel enzyme inhibitors by using substantially the same approach, but screening specifically for changes in target enzyme activity. Also disclosed are methods of producing the relevant transformation and expression vectors as well as methods for identifying lead compounds and drug targets for use in drug development. Finally, the invention also includes within its scope a method for the preparation of a medicinal product.

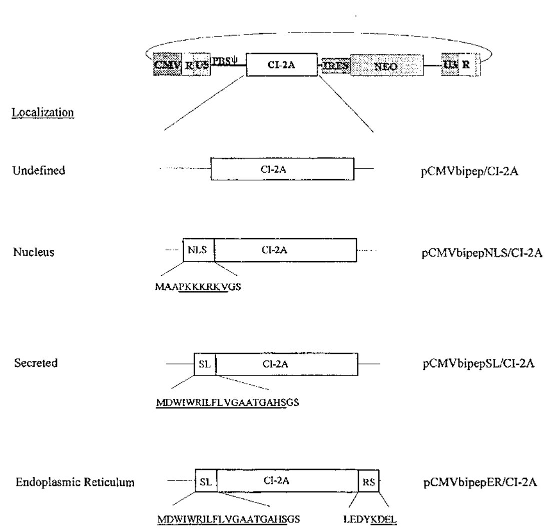


Fig. 1



Printed by Jouve, 75001 PARIS (FR)

## Description

### FIELD OF THE INVENTION

The present invention pertains to a novel method for the identification/preparation of peptides or ribonucleic acids capable of modulating the activity *in vivo* of target enzymes in eukaryotic cells. More specifically, the invention provides a method for identification/preparation of hitherto unknown enhancers as well as inhibitors of *in vivo* enzyme activity in eukaryotic cells. Furthermore, the invention relates to methods for identification of unknown interactions (*i.* e. identification of a target and/or a ligand but also of hitherto unknown interactions between known ligands and known targets). These novel methods employ enzyme inhibitor structures as scaffolds in order to intracellularly display potentially biologically active peptides or ribonucleic acids in a stable form. Also disclosed herein are methods for the preparation of the hitherto unknown ligands or targets as well as methods for the preparation of vectors and transformed cells carrying the genetic information encoding these ligands and targets. Finally, the invention relates to a method for the preparation of a medicinal product which is based on initial identification of targets or ligands according to the present invention.

### BACKGROUND OF THE INVENTION

The CellScreen™ technique is a method which allows for the identification of peptide sequences having biological activity *in vivo* and which is disclosed in WO 96/38553. In short, libraries of random peptides are expressed intracellularly in eukaryotic (eg. mammalian) cells, such that one cell expresses one single or a few heterologous short peptides. Cells that change a preselected phenotype under certain conditions can be isolated and the peptide that they express can hence be identified. The intracellular component with which the peptide interacts (the target molecule) may subsequently be obtained using e.g. affinity columns carrying the immobilized synthetic peptide.

Although the CellScreen™ technology has shown great promise for identifying new drug targets, it is an inherent problem that the intracellular environment is relatively hostile to many heterologous expression products. In other words, interesting peptide or nucleic acid sequences which potentially are capable of interacting with an important target molecule may be degraded or inactivated inside the cell before any effect on phenotype can be detected.

### OBJECT OF THE INVENTION

It is an object of the invention to provide improvements in the CellScreen™ technology by overcoming the above-mentioned problems of potential instability of expressed sequences. Furthermore, it is an object of the invention to expand the utility of the CellScreen™ technology to also encompass screening in prokaryotic cells.

### SUMMARY OF THE INVENTION

A significant number of enzyme activity modulators of plant, microbial and eukaryotic cell origin have been described, cf. below.

Since many of the naturally occurring processes inside cells are regulated by enzymes, the inventors disclose herein a method for expression of large intracellular libraries of such enzyme activity modulators in which the active site of said enzyme activity modulators have been altered by introduction of stretches of randomized amino acid sequences or by introduction of random nucleotides at specific sites in the active site. This creates libraries of putative modulators capable of modulating the activity of an array of different enzymes inside cells. By expressing these modulators in cell lines according to a novel variation of the CellScreen™ technology the enzymatic regulatory mechanisms inside the individual cell in said cell line will be affected differently leading to different phenotypic properties such as e.g. resistance towards hypoglycemia, cytokine killing, toxic compounds, virus infection etc.

The main advantage of using known enzyme activity modulators such as enzyme *inhibitors* as scaffolds is that many of these in their native form are stable in the intracellular environment. The problem of using e.g. antibody fragments as scaffolds for intracellular presentation of random peptides is that many such antibody fragments are susceptible to the proteolytic and reducing intracellular environment and therefore are unsuitable as intracellular scaffolds. On the other hand, enzyme activity modulators can, if carefully tailored, maintain their intracellular stability and at the same time incorporate random sequences which are screened for biological activity. Furthermore, the effectivity of such a screen for biologically active substances will be higher than if using an unstable scaffold (such as a e.g. a coiled coil structure) or no scaffold at all, since none or only a very limited number of the randomized sequences will be degraded before they can exert their effects in the cells. Finally, a majority of such enzyme activity modulators have an active site which is the perfect position in the molecule to modify, since the active site is normally presented in a stable configuration to the environment.

Hence, one aspect of the invention pertains to a method for identifying an *in vivo* active modulator of activity of a target enzyme, the method comprising the steps of (a) preparing a pool of expression vectors, each vector of said pool containing at least one member from a library of randomly modified nucleotide sequences derived from a parent nucleotide sequence encoding a parent peptide or parent ribonucleic acid which modulates the target enzyme activity, (b) transforming a population of substantially identical cells with said vectors of said pool so as to obtain transformed cells, said substantially identical cells being ones which harbour the target enzyme, (c) culturing said transformed cells under conditions facilitating expression of said randomly modified nucleotide sequences, (d) examining said transformed cells and isolating transformed cell(s) wherein the activity of the target enzyme is modulated, and (e) identifying the modulator by determining said randomly modified nucleotide sequence of said vector present in cell(s) isolated in step (d) and/or determining the amino acid sequence or the ribonucleic acid sequence of the expression product encoded by said randomly modified nucleotide sequence.

In this aspect of the invention it is normally preferred that the randomly modified nucleotide sequences consist of 1) an invariable part of the parent nucleotide sequence, and 2) random nucleotides. In line with the above, the invariable part of the parent nucleotide sequence preferably encodes a scaffold portion of the parent peptide or of the parent ribonucleic acid which serves to stabilize said polypeptide fragment or ribonucleic acid fragment.

As mentioned above, the use of enzyme modulator scaffolds also provides for expression of *stable,* biologically active modulators which interact with other biomolecules than enzymes. In other words, in cases where random nucleotides are inserted in a scaffold structure, the outcome will be a stable expression product, the activity of which does not necessarily have anything to do with enzyme activity modulation.

Hence, another part of the invention is a method for identifying a modulator in the form of a biologically active polypeptide fragment or ribonucleic acid fragment which is capable of detectably modulating, *in vivo,* a phenotypic trait in a cell, the method comprising the steps of

(a) preparing a pool of expression vectors, each vector of said pool containing at least one member from a library of randomly modified nucleotide sequences derived from a parent nucleotide sequence encoding a parent peptide or parent ribonucleic acid which *in vivo* modulates activity of a known enzyme, wherein the randomly modified nucleotide sequences comprise

- an invariable part encoding a scaffold portion of the parent peptide or of the parent ribonucleic acid, said scaffold portion serving to stabilize said polypeptide fragment or ribonucleic acid fragment, and
- random nucleotides,

(b) transforming a population of substantially identical cells with said vectors of said pool so as to obtain transformed cells, (c) culturing said transformed cells under conditions facilitating expression of said randomly modified nucleotide sequences, (d) examining said transformed cells and isolating transformed cell(s) wherein the preselected phenotypic trait is modulated by the presence of the expressed randomly modified nucleotide sequence, and (e) identifying the modulator by determining said randomly modified nucleotide sequence of said vector present in cell(s) isolated in step (d) and/or determining the amino acid sequence or the ribonucleic acid sequence of the expression product encoded by said randomly modified nucleotide sequence.

Finally, the invention also pertains to the general use of intracellularly stable scaffold proteins, ribonucleotides, or fragments thereof for the presentation of random sequences in the CellScreen™ technology. As mentioned above, although the concept of using scaffold molecules has been discussed in the prior art, the issue of the *stability* of the scaffold system has not been detailed.

The stability and usefulness of a putative intracellular scaffold is dependent on a number of factors. First of all, it is essential that the relevant cell wherein the scaffold is to be expressed is capable of expressing the scaffold molecule in a functional form; that is, in prokaryotic systems some eukaryotic proteins will not fold correctly, hence rendering the use of such a protein unsuitable as a scaffold in that type of cell. Second, the scaffold should be relatively resistant to the reducing and catalytic environment inside intact cells. However, even when a scaffold molecule is relatively susceptible to the inactivating nature of the intracellular environment, this can be remedied if the production rate of the scaffold molecule is sufficiently high.

In steady state, the intracellular concentration of a scaffold molecule will be a function of the following formula:

$$C_{scaffold} = \frac{R_p}{R_d}$$

-where $R_p$ is the rate of production of the scaffold molecule (moles $\cdot$ $s^{-1}$) and $R_d$ is the inactivation constant for the

scaffold molecule ($1 \cdot s^{-1}$), *i.e.* the rate of inactivation of the scaffold molecule is determined by $\frac{\delta M}{\delta t} = R_d C_{scaffold}$ (which, in steady state, of course equals $R_p$. In other words, when assessing the suitability of a potential scaffold molecule, it should according to the present invention be tested whether the molecule can be kept at a sufficiently high concentration inside the relevant cell wherein the CellScreen™ test is going to be carried out.

Therefore, a very broad aspect of the invention pertains to a method for identifying a modulator in the form of a biologically active polypeptide fragment or ribonucleic acid fragment which is capable of detectably modulating, *in vivo,* a phenotypic trait of a cell, the method comprising the steps of

(a) preparing a pool of expression vectors, each vector of said pool containing at least one member from a library of randomly modified nucleotide sequences derived from a parent nucleotide sequence encoding a parent peptide or parent ribonucleic acid which is stable intracellularly, wherein the randomly modified nucleotide sequences comprise

- an invariable part encoding a scaffold portion of the parent peptide or of the parent ribonucleic acid, said scaffold portion serving to stabilize said polypeptide fragment or ribonucleic acid fragment, and
- random nucleotides,

(b) transforming a population of substantially identical cells with said vectors of said pool so as to obtain transformed cells, (c) culturing said transformed cells under conditions facilitating expression of said randomly modified nucleotide sequences, (d) examining said transformed cells and isolating transformed cell(s) wherein the preselected phenotypic trait is modulated by the presence of the expressed randomly modified nucleotide sequence, and (e) identifying the modulator by determining said randomly modified nucleotide sequence of said vector present in cell(s) isolated in step (d) and/or determining the amino acid sequence or the ribonucleic acid sequence of the expression product encoded by said randomly modified nucleotide sequence. In this aspect of the invention, the expression product of the nucleic acid sequence which encodes the intracellularly stable parent peptide or parent ribonucleic acid is one which, when produced by the substantially identical cells, is present in an effective concentration and in a functional state.

In other words, it is essential that the suitability of the scaffold molecule is evaluated prior to performing the steps of the CellScreen™ technology in order to confirm that the scaffold molecule in unmodified form can be expressed and maintained at a sufficiently high concentration/activity in the cellular system where the method of the invention is to be exercised.

According to WO 96/38553, the isolation of a drug target molecule can be made more efficient if the random peptide sequences are inserted into larger polypeptides functioning as scaffolds for display of the random amino acid sequences. Such scaffolds would probably also lead to higher affinity interaction with the target molecule.

Nothing is, however, mentioned about the use of scaffolds derived from naturally occurring protein inhibitors of enzymes. Inhibition of enzymatic activity by such inhibitors - as opposed to the simple binding of a target protein inside a cell as was suggested in the CellScreen™ technology - is a much more efficient way to affect intracellular biochemical events.

## DETAILED DISCLOSURE OF THE INVENTION

### Definitions

In the following, a number of terms will be defined for the purposes of the present disclosure:

A "modulator" is in the present context a biomolecule which, when expressed *in vivo,* effects the activity of another biomolecule in the cell. Thus, the modulator in essence can inhibit or enhance the activity of the biomolecule. Furthermore, the modulator can interact directly with the biomolecule, but the effect might as well be indirect, *i.e.* the activity change of the biomolecule is brought about by changes in the cell's biochemical machinery, changes which are ultimately the result of the presence of the expression product of the randomized nucleic acid sequence.

A "randomly modified nucleotide sequence" is a nucleotide sequence which in a number of positions has been subjected to insertion or substitution by nucleotides, the nature of which cannot be predicted. In many cases the random nucleotides or nucleotide sequences inserted will be "completely random" (e.g. as a consequence of randomized synthesis or PCR-mediated mutagenesis). However, as will appear from the disclosure below, the random sequences can also include sequences which have a common functional feature (e.g. reactivity with a ligand of the expression product) or the random sequences can be random in the sense that the ultimate expression product is of completely random sequence with e.g. an even distribution of the different amino acids.

"Substantially identical cells" is a term herein intended to designate cells which all exhibit a specific phenotypic trait in such a manner that a change in the expression of said trait in one cell due to an interaction effected by the introduction of random nucleotides according to the inventive methods would also occur in one of the other substantially identical cells had these been transformed with the same vector. In other words, the important parameter to assess when choosing substantially identical cells in the inventive methods is whether an observed change in one cell's exhibition of the phenotypic trait can be taken as an indication that any other cell in the population would have behaved the same way as a consequence of the same change. Hence, substantially identical cells can for instance be clonal cells or cells of a cell line or they can be cells of a cell culture or a tissue culture.

A "phenotypic trait" is the observable result of a certain gene composition in a cell (genotype), *i.e.* a property of a cell (detected by chemical, physical, immunological or any other suitable means) which depends on the presence of one or several genes and the expression rate thereof. Thus, the phenotypic trait can be any of a number of different properties: activity of an enzyme, effects of interaction between receptors and ligands, cell survival rate, presence or absence of an antigen, expression rate, etc.

"Peptide" is in the present context intended to mean both short peptides of from 2 to 10 amino acid residues, oligopeptides of from 11 to 100 amino acid residues, and polypeptides of more than 100 amino acid residues. Furthermore, the term is also intended to include proteins, *i.e.* functional biomolecules comprising at least one polypeptide; when comprising at least two polypeptides, these may form complexes, be covalently linked, or may be non-covalently linked. The polypeptide(s) in a protein can be glycosylated and/or lipidated and/or comprise prosthetic groups.

When using the term "biologically active" to designate a molecule is herein meant that the molecule in question exhibits a detectable effect on *living* cells, i.e. that the molecule interacts with the biology of the living cell so as to produce an effect which can be recognized as a change in the cell's phenotype.

*"In vivo"* is herein mean to designate the environmental conditions inside living cells (*i.e*. cells which are metabolically active and can maintain their vital functions); the living cells may be kept in culture or may be present in a natural habitat (e.g. functioning as part of a larger, multicellular organism). Thus, the term *"in vivo*" also refers to *in vitro* culturing of cells as long as the effect being observed is taking place in the living cell.

"Transformation": A process by which the genetic material carried by an individual cell is altered by incorporation of exogenous DNA into its genome.

"Transfection": The uptake, incorporation, and expression of recombinant DNA by cells.

"Transduction": The transfer of genetic information from one cell to another by way of a viral vector.

The term "effective part" when used in the context of a protein, peptide, or ribonucleic acid is in the present context intended to mean a part (e.g. a subsequence or, in the case of an n-meric protein, a less-than-n-meric molecule) which has retained the desired functionality of the native molecule from which the protein or peptide is derived. For instance, in the case of CI-2A only the truncated form of the molecule seems to be necessary to ensure expression of the active inhibitor intracellularly, and hence the truncated form of that molecule constitutes an effective part of CI-2A; cf. also the Examples herein.

Unless otherwise indicated, nucleotide sequences are presented herein in the 5'→3' direction and amino acid sequences are presented so as to set out with the N-terminus at the left.

General properties of the inventive method

In general, the disclosures in WO 96/38553 and WO 97/27212 relating

to preparation of randomized sequences, to the choice of fusion partners (except for the choice of scaffolds) for the random sequences, to the choice and composition of targeting sequences, to the choice of nucleic acids to be randomly modified, to the choice of randomization method, to the methods of introducing the nucleic acids into the relevant cell type, to the choice of (retroviral) vectors (where applicable), to the method of producing the vectors, the choice of promoters, to the choice of packaging cells (where applicable), to the methods of concentrating infectious virions from the packaging cells (where applicable), to the choice of substantially identical cells to use in the method, to the type of phenotypic changes detected, to the manner in which the change is detected, to methods of isolating the phenotypically changed cells, to the isolation and sequence determination of the randomly modified sequences, to the isolation and characterization of the target for the randomized product, to screening methods, and to the choice of applications of the methods

also are relevant for the purposes of the present invention. Therefore, the disclosures of these two patent applications are hereby incorporated by reference herein. However, since both of these references are focussed on the use of the general principle in higher eukaryotic cells, the present disclosure will also detail on embodiments pertaining to the use in prokaryotic systems, cf. below. However, the more general part of the two above-referenced disclosures which without any difficulty for the skilled person could be applied in the context of a prokaryotic system are also regarded as relevant and important embodiments of the present invention insofar as it relates to the use of the methods in prokaryotic systems.

It is normally preferred that the transformed cells which are being examined in step (d) predominantly carries (and expresses) one single copy of the vector. By ensuring this, the interpretation of a change in phenotype of the cells becomes a much easier task, whereas the interpretation of a phenotype change in cells expressing more than one single randomized sequence renders unclear which of the transforming vectors is responsible for the change.

To ensure that predominantly one vector has transformed each of the cells examined it is e.g. feasible that the transformation step (b) is performed under such conditions that the cells transformed are predominantly or at most transformed with one single vector from said pool (this can e.g. be achieved by adjusting the concentration of infectious virions in embodiments of the invention where the transformation is obtained by means of transduction), or wherein, prior to carrying out step (d), cells being transformed with more than one vector from said pool are substantially excluded from the further steps. This latter option requires that it is possible to quantify the number of transforming vectors and this can be achieved by including a detectable marker in the expression product, e.g. a flourescent probe. Another option is to rescreen cells which exhibit changes in phenotype, thereby ascertaining whether more than one vector has transformed the cell.

It will be understood that the molecule chosen for the purpose of being a scaffold, and wherein the random sequences are ultimately introduced, can be either a peptide sequence or a nucleic acid sequence, such as an RNA fragment interfering (in an antisense-manner) with mRNA, tRNA or with a ribozyme. Alternatively, such an RNA fragment could exhibit ribozyme activity itself, thereby having an indirect influence on the expression rate of other enzymes. At any rate, the resulting product, *i.e.* the randomized expression product, can be a peptide or a ribonucleic acid such as a ribozyme.

One important feature of the scaffold is, as mentioned above, that it is stable towards proteolytic attack and/ or is insensitive to a reducing environment, such as the one which is found intracellularly.

In preferred embodiments of the invention the random nucleotides are introduced in part(s) of the parent nucleotide sequence which encode(s) the active site(s) of the parent peptide or parent ribonucleic acid, or the part(s) which encode(s) structure(s) interfering with the active site(s). As discussed above, the active site (as well as other exposed structures of the scaffold) need to be stably presented to the environment in order to be able to interact with other biomolecules. Hence, preferably the *invariable* part of the nucleotide sequence encodes truncated parts of the parent peptide or parent ribonucleic acid sufficient to maintain stability of the randomized product.

In some embodiments it is preferred that the invariable part of the parent nucleotide sequence encodes a peptide which is free from disulfide bridges. This is due to the fact that disulfide bridges are not formed in the nucleus or in the cytosol. Hence, in cases were the scaffold must be in a functional state when present in the nucleus or the cytosol, it would normally be preferable to use a scaffold which does not contain disulfide bridges or which do not rely on these in order to maintain stability and functionality. On the other hand, in embodiments where it is desired that the randomized expression product is confined to the ER, or to another compartment allowing for the presence of stable disulfide bridges, before the randomized sequence is presented to the environment in a satisfactory manner, it is of course desirable that the invariable part of the parent nucleotide sequence encodes a peptide having disulfide bridges, because the chances of having a correctly folded and functional scaffold outside such compartments is relatively small.

It will be understood that the random nucleotides are preferably introduced in the form of an insertion or a substitution into the parent nucleotide sequence, optionally in combination with deletion(s) in the parent nucleotide sequence. Deleted sequences in the parent polypeptide could e.g. be parts of an active site, the presence of which in unaltered form is toxic or otherwise deleterious to the transformed cells.

The number of random nucleotides introduced can vary to a great degree but normally the number is between 3 and about 100. In this range it is preferred that at least 5, such as at least 7, and better, at least 9-12 random nucleotides are introduces. On the other hand, it is preferred that at most 90, such as at most 70 or 80 random nucleotides are introduced. The most preferred number of introduced randomized nucleotides varies between 15-60, preferably 20-55 or 25-50 nucleotides. Especially preferred numbers of random nucleotides are 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, and 60 nucleotides.

The random nucleotides are introduced in the scaffold in the form of nucleotide sequences and/or in the form of single random nucleotides introduced at specific sites in the parent nucleotide sequence. A variation is to substitute a part of the scaffold sequence with a sequence which retains parts of the scaffold sequence (e.g. those which are essential for stability/functionality) but where other parts are randomized.

The random nucleotides are preferably selected from the group consisting of

- synthetic, completely random deoxyribonucleotides;
- synthetic random DNA sequences, wherein limitation on randomization of some nucleotides is introduced so as to limit the number of available sequences and/or to avoid undesired stop codons and/or to facilitate introduction of post-translational modifications of expressed peptide(s) ;
- synthetic random DNA sequences as in (1) or (2) coupled to a sequence encoding a purification tag; and
- CDR encoding nucleotide sequences isolated from a library of immune-competent cells raised against an antigen (in this embodiment it is preferred that CDR encoding nucleotide sequences encode CDR-3 peptide sequences).

The latter type of "randomization" actually introduces a restriction on randomness which ensures that the sequences introduced encodes an antigen recognizing region. It is, however, well known that a polyclonal immune response against an antigen consist of a large number of immune competent cells which all react with the same antigen (or perhaps even with the same epitope) but the correlation between amino acid sequences of the CDRs and recognition of the epitope(s) is virtually impossible to deduce.

An alternative way to introduce limitations on the randomness of the nucleic acid sequences which are ultimately tested in the substantially identical cells is the following: Upon preparation of the vectors, they are used in a 1$^{st}$ round of phage display, where the phages transformed with the vectors are panned against a library containing a ligand of choice. As for the technique of employing CDR encoding sequences, the result is that the sequences which are ultimately tested in the substantially identical cells are "unpredictable" (and thereby random) but nevertheless selected on the basis of a functional feature. Again, the lack of known correlation between nucleic acid sequences and the interaction in three-dimensional space between the expression product and a ligand of choice has the consequense that the tested subgroup of sequences still is randomized.

In a special embodiment of the above-technique where the method of the invention is combined with phage display, both test systems are repeated in an alternating manner, that is a shuffling between intracellular expression in the substantial identical cells and panning of a phage library.

In order to obtain an controlled distribution of amino acids in the randomized peptides, when the modulator is a peptide, it is practical that the random nucleotides are prepared by random codon synthesis where defined DNA codons are synthesized in a random order; a thorough description of this principle is given in WO 96/38553, cf. Example 1 therein. The preferred embodiment in this context is one wherein the relative amount of synthesized codons ensure that all encoded amino acids will be present with substantially the same frequency in the total of encoded polypeptide fragments, *i.e.* that the chance of encountering one specific amino acid in a library peptide is substantially the same as for any other encoded amino acid.

In order to introduce the randomized fragments properly into the vectors, it is according to the invention preferred that the random nucleotides are introduced into the expression vector by the principle of site directed PCR-mediated mutagenesis. However, other options are known to the skilled person, and it is e.g. possible to insert synthetic random sequence libraries into the vectors as well.

Apart from having the randomized fragment of the expression product introduced into a scaffold in accordance with the present invention, it is often necessary to couple the random sequence to a fusion partner by having the randomized nucleotide sequence fused to a nucleotide sequence encoding at least one fusion partner. Such a fusion partner can e.g. facilitate expression and/or purification/isolation and/or further stabilization of the expression product.

For the purposes of purification, the fusion partner can include a purification tag such as $His_6$ tag, myc tag, BSP biotinylation target sequence, of BirA, flu tag, lacZ, and GST. Furthermore, the fusion partner may include a sorting signal or a targeting sequence, cf. the discussions below.

In embodiments where the modulator is itself a modulator of enzyme activity, it is in theory possible to effect both the $K_M$ and/or the $V_{max}$ of the relevant enzyme. A reduction in $K_M$ results in less effectivity of the relevant enzyme insofar that an increased substrate concentration is required to obtain 50% of maximum activity of the enzyme. An increase of $K_M$ has the opposite effect. Of course, interference with an enzyme which effects $V_{max}$ has as a consequence that the maximum possible rate of activity of the enzyme is increased (when $_{vmax}$ is increased) or decreased (when $_{vmax}$ is increased). At any rate, the modulator of the enzyme activity will give the phenotypic impression that the enzyme activity has either been inhibited or stimulated. It is preferred in this embodiment that the modulator is an inhibitor.

When the method of the invention has finally lead to the identification of a modulator or of a target therefor, it is preferred that the 3-dimensional structure of the identified modulator is resolved, since this allows for the implementation of rational drug screening and computer drug modelling methods.

Use of the inventive methods in prokaryotic systems

The originally envisaged technology disclosed in WO 96/38553 focussed on screening for interactions in *eukaryotic* cells. However, the technology is also applicable in prokaryotic systems.

For instance, it is expected that the present invention will allow for identification of hitherto unknown interactions in pathogenic bacteria, interactions which will be useful in the course of developing new antibiotics. Since the inventive methods allows for the identification of both novel ligand peptides and ribonucleic acids as well as of the target molecules for these ligands, the investigator is provided with the necessary tools for instigating computer drug modelling and for performing traditional drug screening, once such ligands and/or targets have been identified/isolated.

However, apart from the approach of identifying antibacterial effects and substances, the method also opens up for improvements in industrial fermentation processes. In such cases it will e.g. be possible to identify biomolecules which are important in the biochemical pathways in lactic acid bacteria and thereby provide tools for the production of new dairy products such as cheese, yoghourt, and other products of lactic acid bacterial fermentation.

Somewhat related to this approach is the use of the methods of the present invention in screening performed on bacterial cultures used in purification processes. It is well-known in the art of e.g. waste water purification that the microbiological cultures (activated sludge) which conduct the degradation of organic material, are relatively vulnerable *vis à vis* changes in the environment and therefore the provision of more robust strains of bacteria would be one way to improve such systems. Alternatively, the method of the present invention would also allow for the identification/ isolation of ligands and targets in such bacteria which, when interacted with, can lead to e.g. increased efficacy in degradation of specific organic or inorganic substrates.

As will be appreciated from the above, the present invention therefore is highly useful in prokaryotic systems.

For the purposes of using the method of the invention in prokaryotic cells, it is preferred that the prokaryotic cells are bacteria selected from the group consisting of *Bacillus* spp. (e.g. *B. anthracis, B. subtilis* and *B. cereus), Clostridium* spp. (e.g. *C. botulinuin, C. difficile, C. perfringens,* and C. *tetani*), *Corynebacterium* spp. (e.g. *C. diphtheriae,* and *C. pyogenes*), *Staphylococcus* spp. (e.g. *S. aureus* and *S. albicans*), *Streptococcus* spp. (e.g. *S. pneumoniae*, *S. pyogenes,* and *S. agalactiae*), *Escherichia coli, Serratia marcescens, Klebsiella* spp. (e.g. *K. pneumoniae*), *Proteus* spp. (e.g. *P. mirabilis*), *Citrobacter* spp. (e.g. *Citrobacter freundii*), *Salmonella* spp. (e.g. *S. typhi*, *S. typhimurium*, *S. shottmülleri* and S. *paratyphi*), *Shigella* spp. (e.g. *S. dystenteriae, S. flexneri, S. boydii,* and *S. sonnei*), *Pseudomonas* spp. (e.g. *P. aeruginosa*, *P. pseudomallei,* and *P. mallei), Acinetobacter* spp., *Aeromonas* spp., *Plesiomonas* spp., *Yersinia* spp. (e.g. *Y. pestis*, *Y. enterocolitica,* and *Y. pseudotuberculosis), Francisella tularensis, Vibrio* spp. (e.g.

*V. cholerae* and *V. parahaemolyticus*), *Campylobacter* spp. (e.g. *C. jejuni* and *C. coli*), *Helicobacter pylori, Haemophilus* spp. (e.g. *H. influenzea, H. parainfluenzae,* and *H. aegyptius), Bordetella* spp. (e.g. *B. pertussis, B. parapertussis,* and *B. bronchiseptica*), *Brucella* spp., *Neisseria* spp. (e.g. *N. gonnorhoeae* and *N. meningitidis), Treponema pallidum, Leptospira interrogans; Borrelia* spp. (e.g. *B. burgdorferi sensu stricto, B. garinii, B. afzelii,* and *B. recurrentis*), *Legionella pneumophila, Listeria monocytogenes, Mycobacterium* spp. (e.g. *M. tuberculosis, M. bovis, M. africanum, M. kansasii,* and *M. leprae*), *Treponema pallidum, Chlamydia trachomatis, Actinomyces* spp., *Rickettsia* spp., and *Mycoplasma* spp. (e.g. *M. pneumoniae*).

This list of bacteria thus entails bacterial families and species which are involved in pathology of a large number of diseases in humans. Of these, *E. coli* and *B. subtilis* are also used for industrial fermentation; this is also the case for lactic acid bacteria, notably *Lactococcus* spp. and *Lactobacillus* spp. and therefore it is also preferred that the inventive methods, when employed in bacterial systems, are performed on such non-pathogenic species.

When using the inventive methods in a prokaryotic system, it is very often interesting to identify those cells which are impaired in growth or lethally damaged due to the presence of the heterologous expression product introduced according to the invention. This, however, is not completely unproblematic since the main phenotypic trait associated with e.g. bacterial death is *absence* of the bacterium.

It is therefore necessary to device an experimental setup which will allow identification of cells transformed so as to be less good survivors than other otherwise corresponding cells. One advantage is that expression of the heterologous genetic material is under the control of an inducible promoter. In this way it is possible to expand colonies of cells which have been transformed with genetic material which, when expressed, is lethal or growth-impairing to the cells. After that, the expression can be switched on and careful examination of expanded colonies should reveal those clonal colonies which do not follow the same growth pattern as e.g. an untransformed control.

One method of doing this is to spread transformed cells on plates with growth medium and allow the cells to grow up to a pre-determined average size. The spreading of cells should be such that the visible colonies forming will generally be comprised on one single clone of cells. When the pre-determined size of colonies have been reached, all plates are blotted to a carrier medium so as to prepare a "negative" of each agar plate. After this, the expression of the inserted random sequences is induced and the colonies are allowed to grow again. The plates are examined continuously or at suitable intervals (e.g. by means of digital image processing systems well known to the skilled person). Those colonies which reveal an impaired or arrested growth compared to the remainder of the colonies or compared to controls are thereafter identified, since the growth pattern of each colony in an automated manner can be followed. These colonies can then be identified and isolated from the "negative" blot and it is thereafter a relatively simple procedure to extract the transforming genetic material and determine the sequence thereof.

In this context, one interesting option is to render antibiotic-resistant bacteria non-resistant. The growth medium either contains, or is during culturing enriched with, the antibiotic in question and the colonies which upon induction of expression can be demonstrated to be less drug resistant than controls are examined further. Also pure bactericidal/ bacteriostatic effects can be examined. In such an embodiment, the bacteria are e.g. cultured on a suitable growth medium. Those colonies which after induction of expression shows evidence of reduced or arrested growth are examined further: It is expected that some of the bacterial cells will be demonstrated to carry genetic material encoding an expression product which interacts with novel (or known) targets for antibacterial agents.

Another phenotypic trait of interest is of course superior *survival* of cells. It is, when dealing with utilisable bacteria, of interest to identify targets which will increase the survival rate of the bacteria. For example, bacteria used in industrial fermentations normally can be lethally damaged as a consequence of their own uncontrolled production of heterologous expression products. If genes or target molecules can be identified which have a positive effect on the survival of such bacteria, the economic potential is enormous, since a fermentation process will be rendered more economic (less need to startup of new fermentations). Similarly, bacteria used in e.g. waste water purification can be made more resistant against toxic agents in their environment.

The experimental setup in this context is relatively simple: The transformed bacteria are simply subjected to the potentially lethal condition, and only colonies which exhibit a superior survival are isolated and examined (and that will typically be the colonies which are detectable). A setup like the above-described for identifying cell death should thus not be necessary.

Finally, a large group of phenotypic traits to be examined are those which can be detected by e.g. biochemical or immunological means. It is expected that the method of the invention will allow for identification of systems in bacterial cells which, when properly modulated, can render the bacteria more effective as producers in industrial fermentation. Such phenotypic traits could e.g. be changes in enzyme activity, changes in receptor density, changes in expression rate etc.

Special considerations apply when the randomized expression product is fused to a fusion partner which decides the final location of the expression product. Signal sequences in prokaryotes are well-known in the art, but it should briefly be mentioned that membrane-anchoring signals are known, and it is also possible to export the expression product to the periplasmic space of bacteria. Finally, it is also possible to include secretion signals so as to allow the isolation of the expression product from culture supernatant. However, in many cases it is of course most relevant to keep the expression product inside the prokaryotic cytoplasm.

Use of the method in eukaryotic systems

It is especially preferred that the inventive method utilises eukaryotic cells as the substantially identical cells in order to allow screening for active biomolecules. Hence, these eukaryotic cells can be fungal cells, protozoan cells, animal cells, and plant cells.

As is the case for bacteria, a number of fungi are pathogens in mammals, and therefore the present technology will, in parallel with what has been described above concerning antibacterial agents, be useful for identifying antifungal agents by using pathogenic fungi as the substantial identical cells in the method. Furthermore, fungi (especially yeast strains), like bacteria, are also utilised in fermentation processes (e.g. in the wine and brewing industries), and the method can therefore also be utilised using such non-pathogenic fungi as the substantially identical cells which are transformed with the vectors, whereby improvements in these strains can be obtained.

Preferred examples of fungi serving as the eukaryotic cell in the inventive methods are *Epidermophyton* spp., *Trichophyton* spp., *Microsporum* spp., *Candida albicans, Philophora* spp., *Coccidioides immitis, Histoplasma capsulatum, Blastomyces dermatitidis, Paracoccidioides brasiliensis, Cryptococcus neoformans, Aspergillus* spp., *Saccharomyces cerevisiae, Klyveromyces lactis,* and *Piccia pastoris.*

Unlike fungal cells, protozoan cells are only relevant as pathogens for humans and other mammals, although some protozoans form part of biocultures conducting biological waste water purification. The method of the invention is therefore contemplated to be useful in identifying new targets for antiprotozoan agents. In this context, the preferred protozoan cells used as the substantially identical cell in the methods of the invention are selected from the group consisting of *Giardia lamblia, Trichomonas vaginalis, Dientamoeba fragilis, Trypanosoma* spp., *Leishmania* spp., *Entamoeba histolytica, Naegleria fowleri, Acanthamoeba castellani, Harmanella* spp., *Isospora belli, Cryptosporidium* spp., *Sarcocystis* spp., *Toxoplasma gondii, Plasmodium* spp. (e.g. *P. falciparum, P. vivax, P. malariae, P. knowlesi,* and *P. ovale*), *Babesia* spp., and *Balantidium coli.*

Also plant cells are according to the invention interesting as target eukaryotic cells. The plant cells can be any plant cell which can be subjected to genetic engineering techniques allowing for single cell expression and growth. Thus, cells derived from e.g. *Nicotiana tabacum* (tobacco plant), *Arabidopsis thaliana, Brassica napus, Brassica juncea, Musa sp.* (banana plants), rice, and corn are examples of plant cells useful in the invention. The skilled person in the art of plant genetic engineering will know to choose suitable plant cells in the appropriate stage of their life cycle, suitable vector systems as well as suitable transformation methods. A short summary is given here:

> As other organisms, plant cells can be transformed with foreign DNA. One strategy for plant transformation employs *Agrobacterium tumefaciens,* a naturally occurring plant pathogenic bacterium which contains a plasmid (the Ti plasmid) with the ability to enter plant cells and insert a portion of its genome into plant chromosomes. The Ti plasmid has been engineered to make it a vector for plant transformation by including sequences for replication in *E. coli* and *Agrobacterium,* unique restriction sites for inserting foreign genes, and selectable markers.

Unfortunately, *Agrobacterium* is not very effective at transforming monocots, a large group of plants that includes all of the agriculturally important cereals. Legumes, another important group of food crops, are also difficult to transform and regenerate with *Agrobacterium.* Therefore, a second strategy for transforming plants has been developed involving the Gene Gun, where a gene is inserted into an expression vector and coated onto beads. The DNA-coated beads are then introduced by means of the gene gun into plant cells, where a small fraction are taken up and incorporated into the DNA. Individual plant cells, callus, regenerating shoots, and embryos are all suitable targets in this technique.

To determine whether cells have actually incorporated foreign DNA and become transgenic, reporter genes such as GUS and Luciferase genes are used. In any case, foreign genes must be flanked by a plant promoter in order to be expressed.

It is preferred to use cells derived from animals. These cells can be mammalian cells, arthropod cells such as insect cells, avian cells, and piscine cells. A number of reasons can be listed for using such cell types which each require a relevant choice of transformation and expression systems, growth conditions, etc, all easily determined and chosen by the skilled person. It suffices to note that e.g. certain insects cause enormous problems in human society (due to their direct damaging activities or due to their functions as vectors carrying infectious agents), and therefore the method of the invention would supplement in the attempts of controlling such insects. As for the mammals, birds and fish, a number of these are important in agri- and aquaculture, where disease control is of interest.

According to the invention mammalian cells such as human cells/cell clones or human cell lines are most preferred. This is due to the fact that a large number of diseases in humans and other mammals etiologically depend on molecular interactions in the living cell - the provision of drugs or lead compounds which interact *in vivo* with biomolecules which play a role in diseases is therefore of great interest. Preferred mammalian cells are Chinese hamster ovary (CHO) cells, VERO cells, HeLa cells, W138 cells, BHK cells, COS-7 293 cells, and MDCK cells, which are all well-known in the art.

The candidate nucleic acids are hence introduced into eukaryotic cells as part of a vector to screen for modulators of target enzyme activity. By the term "introduced into" is herein meant that the nucleic acids enter the cells in a manner suitable for subsequent expression of the nucleic acid. The method of introduction is largely dictated by the targeted cell type, cf. below. Exemplary, but non-limiting methods include $CaPO_4$ precipitation, liposome fusion, lipofectin®, electroporation, viral infection, etc.

The randomly modified nucleic acids are preferably integrated into the host cell genome (e.g. by means of retroviral infection of the host cell), or may exist either transiently or stably in the cytoplasm (*i.e.* through the use of traditional plasmids utilizing standard regulatory sequences, selection markers, etc.).

Currently, the most efficient gene transfer methodologies for mammalian cells harness the capacity of engineered viruses, such as retroviruses, to bypass natural cellular barriers to exogenous nucleic acid uptake.

The vector is preferably selected from the group consisting of a retroviral vector, a vaccinia virus vector, an adenoviral vector, an adeno associated virus (AAV) vector, a herpes simplex virus (HSV) vector, an alpha virus vector, and a semliki forest virus vector.

Retroviral transduction

As many pharmaceutically important screens require human or model mammalian cell targets, retroviral vectors capable of transfecting such targets are preferred.

Therefore, the candidate nucleic acids are preferably part of a retroviral virion which infects the cells. Generally, infection of the cells is straightforward with the application of the infection-enhancing reagent polybene. Infection can be optimized such that the cells predominantly express a single construct each, e.g. by using the ratio of virus particles to the number of cells. Alternatively, it is possible to "screen out" cells which have been infected with more than one single virion, e.g. by quantitatively assessing a selection marker and only. The rate of infection is well-known to follow a Poisson distribution.

A preferred embodiment of the invention where the substantially identical cells are eukaryotic thus comprises that step (a) is carried out by

1) transfecting suitable packaging cells with vectors comprising the randomly modified nucleotide sequences and which are integratable in virions produced by said packaging cells,
2) culturing said transfected packaging cells in a culture medium under conditions which facilitate production by the packaging cells of virions containing the randomly modified nucleotide sequences,
3) recovering and optionally concentrating said virions, and
4) transducing said substantially identical cells with the virions.

Thus, preferably the candidate nucleic acids are introduced into the substantially identical cells using retroviral vectors. The use is well-known in the art of helper-defective packaging cell-lines which are capable of producing all necessary proteins (*gag, pol,* and *env*) required for packaging, processing, reverse transcription, and integration of recombinant genomes, cf. the below discussion of such cell lines. Those RNA molecules which have in cis a ψ packaging signal are packaged into maturing virions. In eukaryotes, retroviruses are preferred for a number of reasons. First, their derivation is fairly easy. Second, unlike Adenovirus-mediated gene delivery, expression from retroviruses is long-term (adenoviruses do not integrate). Adeno-associated viruses have limited space for genes and regulatory units and there is some controversy as to their ability to integrate. Retroviruses therefore currently provide the best compromise in terms of long-term expression, genomic flexibility and stable integration, among other features. The main advantage of retroviruses is that their integration into the host genome allows for their stable transmission through cell division. This ensures that in cell types which undergo multiple independent maturation steps, such as hematopoietic cell progression, the retrovirus construct will remain resident and continue to express.

Preferred retroviral vectors include vectors derived from retrovirus selected from the group consisting of Avian Leukosis-Sarcoma Virus (ALSV), Mammalian type C, Mammalian type B, and Lentivirus as well as vectors derived from MSCV (murine stem cell virus), modified MFG virus and pBABE, and optionally modified with heterologous cis-acting elements.

In general, retroviral vectors should contain as few viral sequences as possible in order to minimize potential recombination events. Only the sequences necessary for packaging, reverse transcription and integration should be retained, as well as the viral promoter, enhancer, and polyadenylation sequences.

The library of random nucleotides can be generated in a retrovirus DNA construct backbone, as is generally described in e.g. WO 97/27212. Standard oligonucleotide synthesis generates the random portion of the candidate modulator, using techniques well known in the art (cf. Eckstein, Oligonucleotides and Analogues, A Practical Approach, IRL Press At Oxford University Press, 1991); oligonucleotide libraries may be commercially purchased. Libraries with up to $10^9$ unique sequences can be readily generated in such DNA backbones. After generation of the DNA library, the library is cloned into a first primer. The first primer serves as a "cassette" which is inserted into the retroviral construct. The first primer generally contains a number of elements, including for example, the required regulatory sequences (e.g. translation, transcription, promoters, etc), fusion partners and scaffold molecule(s), restriction endonuclease (cloning and subcloning) sites, stop codons (preferably in all three reading frames), regions of complementarity for second strand priming (preferably at the end of the stop codon region as minor deletions or insertions may occur in the random region), etc.

A second primer is then added, which generally consists of some or all of the complementarity region to prime the first primer and optional necessary sequences for a second unique restriction site for subcloning. DNA polymerase is added to make double-stranded oligonucleotides. The double-stranded oligonucleotides are cleaved with the appropriate subcloning restriction endonucleases and subcloned into the target retroviral vectors, described below.

In this manner the primers create a library of fragments, each containing a different random nucleotide sequence within a scaffold sequence derived from genetic material encoding a enzyme modulator. The ligation products are then transformed into bacteria, such as *E. coli* and DNA is prepared from the resulting library, as is generally outlined in Kitamura, PNAS U.S.A. 92: 9146-50 (1995), which is incorporated by reference herein.

Any number of suitable retroviral vectors may be used. Generally, the retroviral vectors may include: selectable marker genes under the control of internal ribosome entry sites (IRES), which allows for bicistronic operons and thus greatly facilitates the selection of cells expressing peptides at uniformly high levels; and promoters driving expression of a second gene, placed in sense or anti-sense relative to the 5'-LTR (long terminal repeat). Suitable selection genes include, but are not limited to, neomycin, blastocidin, bleomycin, puromycin, and hygromycin resistance genes, as well as self-fluorescent markers such as green fluorescent protein, enzymatic markers such as *lacZ,* and surface proteins such as CD8, etc.

The retroviruses may include inducible or constitutive promoters. For example, there are situations wherein it is necessary to induce peptide expression only during certain phases of the selection process. For instance, a scheme to provide pro-inflammatory cytokines in certain instances must include induced expression of the peptides. This is because there is some expectation that over-expressed pro-inflammatory drugs might in the long-term be detrimental to cell growth. Accordingly, in this situation constitutive expression is undesirable, and the peptide in only turned on during that phase of the selection process when the phenotype is required, and then the peptide is shut down by turning off the retroviral expression to confirm the effect or ensure long-term survival of the producer cells. A large number of both inducible and constitutive promoters are known to the skilled person.

In addition, it is possible to configure a retroviral vector to allow inducible expression of retroviral inserts after integration of a single vector in target cells; importantly, the entire system is contained within the single retrovirus. Tet-inducible retroviruses have been designed incorporating the Self-Inactivating (SIN) feature of 3'LTR enhancer/promoter retroviral deletion mutant (Hoffmann *et al*., PNAS U.S.A. 93:5185 (1996)). Expression of this vector in cells is virtually undetectable in the presence of tetracycline or other active analogues. However, in the absence of Tet, expression is turned on to maximum within 48 hours after induction, with uniform increased expression of the whole population of cells that harbour the inducible retrovirus, indicating that expression is regulated uniformly within the infected cell population. A similar, related system uses a mutated Tet DNA-binding domain such that it bound DNA in the presence of Tet, and was removed in the absence of Tet. Either of these systems is suitable.

According to the present invention, the most preferred vectors (described in Example 1) are based on the murine Akv retrovirus, a mammalian type C retrovirus (NCBI taxonomy Id #11791). The Akv virus has high homology with the Moloney retrovirus, commonly used in the field. A brief description of the design of these preferred vectors is as follows:

- The vectors contain a chimeric 5' LTR, allowing expression from the strong Cytomegalovirus (CMV) promoter when transcription is driven from the plasmid (as in transfections). Following integration of the vector into the host genome, transcription is driven from the retroviral LTR (as in transductions).

- A versatile polylinker is present downstream of the packaging signal. This enables the insertion of peptide libraries being part of a scaffold molecule in this position.

- Immediately downstream of the polylinker is an internal ribosomal entry site (IRES), derived from the encephalomyocarditis (EMC) virus or an internal promoter, originating from the SV-40 virus. This allows efficient translation from the downstream expression cassette either in a CAP independent (IRES), or in a CAP dependent (internal promoter) manner.

- Several different marker genes have been cloned into the downstream expression cassette. For example antibiotics resistance genes such as Neo and Hygro, fluorescent proteins such as EGFP, or surface proteins such as ΔNGFR. The availability of vectors containing different markers allows for the selection of transduced cells using either drug treatment, flow cytometry or magnetic bead separation.

- Any scaffold protein or marker can be combined, either in the bicistronic vector or in a vector containing the SV-40 internal promoter.

In view of the above, it is hence preferred that the retroviral vector has non-identical ends so as to facilitate PCR-based generation of random DNA sequences. It is furthermore preferred that these non-identical ends contain non-identical promoters. An especially preferred retroviral vector contains a heterologous promoter replacing the viral promoter in the 5'-LTR, such as a CMV promoter, an RSV promoter, an SV-40 promoter, a TK promoter, an MT promoter, or an inducible system such as Tet or Ecdysone.

Particularly well suited retroviral transfection systems (packaging cells) are PE501 (US 4,861,719), Bosc23 (WO 94/19478), Ψ2 (R. Mulligan/D. Baltimore), GP+E86 (US 5,278,056), PhoenixEco (WO 97/27212), PA317 (US 4,861,719), GP+AM12 (US 5,278,056), DA(ampho) (WO 95/10601, WO 92/05266), Bing (WO 94/19478), FLYA13 (WO 97/08330), ProPak (available from SyStemix), CRIP (R. Mulligan), ΨAM (R. Mulligan/D. Baltimore), Phoenix-Ampho (WO 97/27212), PG13 (Targeted Genetics), H9 (293GPG) (D. Ory, M Sadelain, R. Mulligan, J. Schaffer), and EcoPack (Clonetech).

Retroviral transduction is dependent upon the interaction between the virus envelope glycoproteins and host cell surface receptors. By far the two most commonly exploited receptors for retroviral gene delivery are the ecotropic receptor (restricted to murine and rat cells) and the amphotropic receptor (widely distributed on both immortalized cell lines and on primary mammalian cells). A number of packaging cell lines, for generating either ecotropic or amphotropic viruses, exists. In addition, packaging cell lines, which pseudotype retroviral particles with either GALV (Gibbon Ape Leukemia Virus glycoprotein) or VSV G (Vesicular Stomatitis Virus G glycoprotein) have also recently been developed.

Until recently most packaging cell lines were based on NIH-3T3 cells, such as the Ψ2 and GP+86 (ecotropic) lines and the PA317, GP+AM12 and CRIP (amphotropic) lines. These have been used extensively and work well, particularly when stable producer lines are made. However, NIH-3T3 based packaging cell lines give relatively low titers when virus is generated from transient transfection. Over the past few years several packaging cell lines based on the highly transfectable 293 cell line (human embryonic kidney cells) have been developed. These include the Bosc23 cell line (Pear *et al.,* 1993) an ecotropic cell line which has been demonstrated to work very well within the boundaries of the present invention, as well as the EcoPack cell line from Clonetech. The advantage of 293 based lines is that very high titers (up to $10^7$ infectious units/ml, IU/ml) of viral supernatant can be produced from transient transfections in as little as 48 hours. In a library situation transient transfections are preferred, as this gives all library members a chance of being equally well expressed, without any bias introduced by expression from different integration sites.

Having access to well characterised stable packaging cell lines is critical for gene therapy associated projects.

For the purposes of the present invention, however, it is not necessary to employ such well defined lines, as the libraries will always be produced from transient transfections and no stable "producer line" will or need be established. An alternative strategy for gaining entry into non-murine cells that has been explored by the present inventors is therefore to use a heterologous viral envelope glycoprotein to pseudotype viruses produced in ecotropic packaging cell lines, e. g. that from Vesicular Stomatitis Virus (the VSV G protein). VSV G pseudotyping of retroviruses is interesting for two main reasons. First, the cell surface receptors for VSV G are ubiquitous membrane components, such as phosphatidylserine and gangliosides. Pseudotyping with VSV G therefore confers broad tropism to the virions. Second, the VSV G protein is extremely stable once incorporated into the virions. This is important as it allows concentration of the viral supernatant by ultra-centrifugation, a step which can increase the viral titers per volume unit by 10 to 100 fold.

The VSV G protein is highly fusogenic and, as a consequence, exhibits cytotoxicity in tissue culture. It has therefore not been possible to establish packaging cells that express VSV G constitutively. To circumvent this problem, inducible systems have been developed (Ory *et al.,* 1996). However, because the CellScreen™ retroviral libraries are produced from transient transfections, a very simple alternative is to transiently transfect VSV G, together with the library, into an ecotropic packaging cell line, such as Bosc23. This approach allows viral supernatants of broad tropism and high titers to be produced, before severe toxicity is observed in the culture. Using this method, a panel of non-murine target cell lines have been tested for transducability and titers of up to $10^7$ IU/ ml of viral supernatants have routinely been achieved by the inventors.

A recently reported alternative to pseudotyping with VSV G is to pseudotype with the envelope glycoprotein of Lymphocytic Choriomeningitis Virus (LCMV) (cf. Miletic *et al.,* 1999, J. Virol. 73; 6114-6116). This alternative is also included as an embodiment of the present invention.

After production in packaging cells, concentration of virus may be performed as follows: Generally, retroviruses are titred by applying retrovirus-containing supernatant onto indicator cells, such as NIH-3T3 cells, and then measuring the percentage of cells expressing phenotypic consequences of infection. The concentration of the virus is determined by multiplying the percentage of cells infected by the dilution factor involved, and taking into account the number of target cells available to obtain a relative titre. If the retrovirus contains a reporter gene, such as *lacZ,* then infection, integration, and expression of the recombinant virus is measured by histological staining for *lacZ* expression or by flow cytometry (FACS). In general, retroviral titres generated from even the best of the producer cells do not exceed $10^7$ per ml, unless concentration is performed on relatively expensive or exotic apparatus. However, it is believed that particles as large as retrovirus will not move very far by means of brownian motion in liquid, fluid dynamics predictions show that much of the virus never comes in contact with the cells in order to initiate infection. However, if cells are grown or placed on a porous filter surface and retrovirus are allowed to pass the cells by gradual gravitometric flow, a high concentration of virus around cells can be effectively maintained at all times. Thus, up to a ten-fold higher infectivity by infecting cells on a porous membrane and allowing retrovirus supernatant to flow past them has been seen. This should allow titres of $10^9$ after concentration.

Upon isolation/concentration of virus, the substantially identical target cells are transduced by methods well-known in the art.

In applications when effector molecules with oncogenic potential are present in the library it is important to use retroviral supernatants which are non-infectious to humans. In these cases the ecotropic receptor can be stably introduced into the target cell of interest and viruses can be produced using ecotropic systems. The ecotropic receptor is a cationic amino acid transporter protein (mCAT), shown to be sufficient to confer susceptibility to ecotropic virus infection (Albritton *et al*. 1989). Expression of this receptor in a variety of human cells, including lymphocytes, have been documented in the literature (Hitoshi *et al.,* 1998). The present inventors have demonstrated that introduction of mCAT, both by stable transfection and by transduction (using a retroviral vector encoding mCAT), yield target cells that are highly susceptible to infection by Bosc23 generated virions.

Hence, the cell lines discussed above, and the other methods for producing retrovirus, are useful for production of virus by transient transfection. The virus can be either used directly or used to infect another retroviral producer cell so as to expand the library.

Fusion partners

As mentioned above, fusion of the expression product to at least one fusion partner which facilitates expression and/or purification/isolation and/or further stabilization of the expression product is often desired.

In eukaryotes, the fusion partner is often a sorting signal or a targeting sequence. Such a sorting signal will be in the form of a signal patch or a signal peptide. The well-known function of a sorting signal is to effect export of an expressed peptide into endoplasmic reticulum, into Golgi apparatus, into lysosomes, into secretory vesicles, into mitochondria, into peroxisomes, or into the nucleus. Of course, also export to the membrane or out of the cell are possibilities. Preferably, the sorting signal or targeting sequence is selected from the group consisting of

- a nuclear localization signal (NLS) such as Pro-Lys-Lys-Lys-Arg-Lys-Val (SV40 large T antigen NLS), Ala-Arg-Arg-Arg-Arg-Pro (human retinoic acid receptor-β NLS), Glu-Glu-Val-Gln-Arg-Lys-Arg-Gln-Lys-Leu (NFκB p50), Glu-Glu-Lys-Arg-Lys-Arg-Thr-Tyr-Glu (NFκB p65), and Ala-Val-Lys-Arg-Pro-Ala-Ala-Thr-Lys-Lys-Ala-Gly-Gln-Ala-Lys-Lys-Lys-Lys-Leu-Asp (Xenopus nucleoplasmin NLS);
- a membrane anchoring sequence such as those derived from CD8, ICAM-2, IL-8, CD4, and LFA-1, and a lipidation sequence such as a myristylation or a palmitoylation sequence;
- a lysosomal sorting signal such as a lysosomal degradation sequence, and a lysosomal membrane sequence
- a mitochondrial localization sequence such as a mitochondrial matrix sequence, a mitochondrial inner membrane sequence, a mitochondrial intermembrane space sequence, and a mitochondrial outer membrane sequence; an endoplasmic reticulum localization sequence such as the sequence from calreticulin (KDEL) and the sequence from adenovirus E3/19K protein (LULSRRSFIDEKKMP) ;
- a peroxisome sequence such as the peroxisome matrix sequence from Luciferase;
- a farnesylation sequence such as LNPPDESGPGCMSCKCVLS;
- a geranylgeranylation sequence such as LTEPTQPTRNQCCSN;
- a destruction sequence such as RTALGDIGN; and
- a secretory signal sequence such as the secretory signals from IL-2, growth hormone, preproinsulin, and influenza HA protein.

Enzyme modulators useful in the invention

The art has demonstrated the existence of numerous effective peptide enzyme activity modulators. Especially the enzyme inhibitors are well-characterized. Non-limiting examples which are all incorporated by reference herein are listed in the following:

***BPTI/Kunitz family of protease inhibitors:***

Pancreatic trypsin inhibitor (BPTI) from *Bos taurus;*
Spleen trypsin inhibitor from *Bos taurus;*
Inter-alpha-trypsin inhibitor light chain (bikunin) from *Bos taurus, Homo sapiens, Meriones unguiculatus, Mesocricetus auratus, Mus musculus, Sus scrofa, Pleuronectes platessa,* and *Rattus norvegicus, respectively;*
Inter-alpha-trypsin inhibitor from *Equus caballus, Ovis aries,* and *Capra hircus,* respectively;
Hemolymph trypsin inhibitor A from *Manduca sexta;*
Hemolymph trypsin inhibitor B from *Manduca sexta;*
Colostrum trypsin inhibitor from *Bos taurus;*
Trypstatin from *Rattus norvegicus;*
Proteinase inhibitor from *Tachypleus tridentatus;*
Serum basic protease inhibitor from *Bos taurus;*
Chymotrypsin inhibitor SCI-III from *Bombyx mori;*
Male accessory gland serine-protease inhibitor from *Drosophila funebris;*
Protease inhibitor 5 II from *Anemonia sulcata;*
Chymotrypsin inhibitors SCI-I and SCI-II from *Bombyx mori;* Proteinase inhibitors SHPI and SHPI-2 from *Stoichactis helianthus;*
Isoinhibitor K from *Helix pomatia;*
Trypsin inhibitor IV from *Radianthus macrodactylus;*
Venom basic protease inhibitors IX and VIIIB from *Bungarus fasciatus;*
Venom basic protease inhibitors I and III from *Vipera ammodytes ammodytes;*
Venom basic protease inhibitor II from *Daboia russelli siamensis, Hemachatus haemachatus,* and *Naja nivea,* respectively;
Venom basic protease inhibitors B and E from *Dendroaspis polylepis polylepis;*
Venom chymotrypsin inhibitor from *Naja naja;*
Venom basic protease inhibitors I and K from *Dendroaspis polylepis polylepis;*
Venom basic protease inhibitor K from *Dendroaspis angusticeps* Venom trypsin inhibitor from *Eristocophis macmahoni* and *Naja naja,* respectively;
Protease inhibitor from *Sarcophaga bullata;*
Tissue factor pathway inhibitor from *Homo sapiens, Oryctolagus cuniculus,* and *Rattus norvegicus,* respectively;
Tissue factor pathway inhibitor 2 from *Homo sapiens;*

Uterine plasmin/trypsin inhibitor from *Sus scrofa;*
Protease nexin II (fragment of Alzheimer's disease amyloid A4 protein) from *Homo sapiens, Mus musculus, rattus norvegicus, Macaca fascicularis* and *Macaca mulatta,* respectively;
Amyloid protein 2 from *Homo sapiens* and *Rattus norvegicus,* respectively; and
Ornithodorin from *Ornithodoros moubata,*

as well as inhibitors homologous therewith isolated from other sources than those explicitly mentioned.

***Serpin family of protease inhibitors:***

Alpha-1-proteinase inhibitors (alpha-1-antitrypsins)from *Equus caballus, Mus musculus, Cavia porcellus, Oryctolagus cuniculus, Bos taurus, Chinchilla villidera, Didelphis marsurpiales virginiana, Homo sapiens, Macropus eugenii, Mus caroli, Papio anubis, Sus scrofa, Rattus norvegicus,* and *Ovis aries, respectively;*
Alpha-1-antichymotrypsin from *Homo sapiens;*
Antithrombin III from *Homo sapiens, Bos taurus, Mus musculus, Ovis aries, Mesocricetus auratus,* and *Gallus gallus,* respectively;
Alpha-2-plasmin inhibitor (alpha-2-antiplasmin) from *Bos taurus, Homo sapiens,* and *Mus musculus,* respectively;
Bomapin (Protease Inhibitor 10) from *Homo sapiens;*
Contrapsin from *Mus musculus* and *Cavia porcellus;*
Contrapsin-like protease inhibitors from *Rattus norvegicus;*
Factor XIIa inhibitor from *Bos taurus;*
Glia derived nexin (protease nexin I) from *Homo sapiens, Mus musculus,* and *Rattus norvegicus,* respectively;
Heparin co-factor II from *Homo sapiens, Mus musculus, Oryctolagus cuniculus,* and *Rattus norvegicus,* respectively;
47 kDa heat shock protein (serine protease inhibitor J6) from *Mus musculus* and *Gallus gallus,* respectively;
Cl-inhibitor from *Homo sapiens;*
Leukocyte elastase inhibitor from *Equus caballus, Homo sapiens,* and *Sus scrofa,* respectively;
Protein C inhibitor from *Homo sapiens;*
Kallistatin from *Homo sapiens;*
Kallikrein-binding protein from *Mus musculus* and *Rattus norvegicus,* respectively;
Maspin from *Homo sapiens, Mus musculus,* and *Rattus norvegicus,* respectively;
Plasminogen activator inhibitor-1 from *Bos taurus, Homo sapiens, Mus musculus, Mustela vison,* and *Rattus norvegicus,* respectively;
Plasminogen activator inhibitor-2 from *Homo sapiens, Mus musculus,* and *Rattus norvegicus,* respectively;
Neuroserpin from *Homo sapiens, Mus musculus,* and *Gallus gallus* Cytoplasmic antiproteinases 1, 2 and 3 from *Homo sapiens* Antitrypsin from *Bombyx mori,* respectively;
Antichymotrypsins I and II from *Bombyx mori;*
Alaserpin from *Manduca sexta;*
Serine protease inhibitor 2.1 from *Rattus norvegicus;*
Serine proteinase inhibitor 1 from Cowpox virus, Rabbit pox virus, Swine pox virus, Vaccinia virus, and Variola virus, respectively;
Serine proteinase inhibitor 2 from Rabbit pox virus, Vaccinia virus, and Variola virus, respectively;
Serine proteinase inhibitor 3 from Vaccinia virus and Variola virus, respectively;
Serpin 1 from Myxoma virus;
Serine proteinase inhibitor from *Halocynthia roretzi;*
Ice inhibitor from Cowpox virus (thiol-protease inhibitor);

as well as inhibitors homologous therewith isolated from other sources than those explicitly mentioned.

***Kazal family of protease inhibitors:***

Acrosin inhibitors from *Bos taurus, Homo sapiens, Sus scrofa,* and *Macaca fascicularis,* respectively;
Elastase inhibitor from *Anemonia sulcata;*
Ovoinhibitor from *Gallus gallus;*

Rhodniin from *Rhodnius prolixus;*
Pancreatic secretory trypsin inhibitor from *Rattus norvegicus, Anguilla anguilla, Bos taurus, Canis familiaris, Homo sapiens, Sus scrofa* and *Ovis aries,* respectively;
Pancreatic secretory trypsin inhibitor II from *Rattus norvegicus;*
Double-headed protease inhibitor (submandibular gland) from *Canis familiaris, Felis silvestris catus, Meles meles, Panthera leo,* and *Vulpes vulpes,* respectively;
Trypsin inhibitor from *Halocynthia roretzi;*
Tryptase inhibitor from *Hirudo medicinalis;*
Prostatic secretory glycoprotein from *Mus musculus ;*
Ovomucoid (third domain) from *Aburria pipile, Aepypodius arfakianus, Afropavo congensis, Alectoris chukar, Alectoris rufa, Anas platyrhynchos, Chloephaga picta, Cyanochen cyanop tera, Neochen jubata, Tadorna radjah, Lophonetta specularioides, Anas capensis*, *Aix galericulata, Aix sponsa, Sarkidiornis melanotos, Alopochen aegyptiaca, Mergus cucullatus, Anhinga novaehollandiae, Anser anser anser, Anser indicus, Anseranas semipalmata, Arborophila torqueola, Argusianus argus, Aythya americana, Netta rufina, Balearica pavonina, Bambusicola thoracica, Bonasa umbellus, Branta canadensis, Anser canagicus, Callipepla squamata castanogastric, Callipepla squamata pallida, Carpodacus mexicanus, Carpococcyx renauldi, Casuarius casuarius, Casuarius bennetti, Cereopsis novaehollandiae, Chauna chavaria, Chauna torquata, Gallus gallus, Chrysolophus amherstiae, Chrysolophus pictus, Circus aeruginosus, Colinus virginianus, Corvus albus, Corvus monedula, Coscoroba coscoroba, Coturnix delegorguei, Coturnix coturnix japonica, Crossoptilon crossoptilon, Cygnus atratus, Cygnus olor, Oxyura jamaicensis, Oxyura vittata, Cyrtonyx montezumae, Dacelo novaeguineae, Dendrocygna arborea, Dendrocygna arcuata, Dendrocygna autumnalis, Dendrocygna bicolor, Dendrocygna eytoni, Dendrocygna viduata, Dromaius novae-hollandiae, Eudromia elegans, Francolinus afer coqui, Francolinus erckelii, Francolinus francolinus, Francolinus pondicerianus, Fulica atra, Gallinula chloropus, Gallirallus australis, Gallus varius, Geococcyx californianus, Grus carunculatus, Grus japonensis, Grus vipio, Anthropoides virgo, Guira guira, Guttera pucherani, Gyps coprotheres, Polyboroides radiatus, Aquila audax, Necrosyrtes monachus, Haliaeetus albicilla, Haliastur indus, Larus ridibundus, Larus marinus, Vanellus spinosus, Leipoa ocellata, Lophura bulweri, Lophortyx californica, Lophortyx gambelii, Lophura ignita, Lophura diardi, Lophura leucomelana, Megapodius freycinet, Meleagris gallopavo, Agriocharis ocellata, Nothoprocta cinerascens, Nothoprocta perdicaria, Numida meleagris, Acryllium vulturinum, Nycticorax nycticorax, Opisthocomus hoazin, Oreortyx pictus, Ortalis vetula, Pavo cristatus, Pavo muticus, Penelope jacquacu, Penelope superciliaris, Perdrix perdrix, Phasianus colchicus colchicus, Phasianus versicolor, Phalacrocorax sulcirostris, Podargus strigoides, Polyplectron bicalcaratum, Polyplectron emphanum, Polyborus plancus, Pygoscelis adelie, Phalacrocorax albiventer, Rhea americana, Pterocnemia pennata, Rhynchotus rufescens, Rollulus roulroul, Scythrops novaehollandiae, Spheniscus humboldti, Struthio camelus, Syrmaticus mikado, Syrmaticus reevesii, Tinamus major, Turdus merula, Turnix sylvatica, Tympanuchus cupido, Centrocercus urophasianus, Tragopan blythii, Tragopan caboti, Tragopan satyra, Tragopan temminckii, Lophophorus impejanus, Crossoptilon auritum, Crossoptilon mantchuricum, Lophura edwardsi, Lophura nycthemera, Lophura swinhoei, Pucrasia macrolopha, Catreus wallichii, Syrmaticus ellioti, Syrmaticus humiae, Syrmaticus soemmerringii, Lagopus leucurus*, and *Vultur gryphus,* respectively,

as well as inhibitors homologous therewith isolated from other sources than those explicitly mentioned.

***Soybean trypsin inhibitor (Kunitz) family of protease inhibitors:***

Aspartic proteinase inhibitor from *Solanum tuberosum;*
Cathepsin D inhibitors from *Solanum tuberosum;*
Wound-induced aspartate proteinase inhibitor from *Solanum tuberosum;*
Chymotrypsin inhibitor 3 precursor from *Psophocarpus tetragonolobus;*
Trypsin inhibitor from *Adenanthera pavonina;*
Trypsin inhibitor from *Prosopsis juliflora;*
Trypsin inhibitor from *Erythrina caffra;*
Trypsin inhibitor from *Erythrina latissima;*
Chymotrypsin inhibitor from *Erythrina variegata;*
Trypsin inhibitors 1A, 1B, and 2 from *Psophocarpus tetragonolobus;*
Trypsin/chymotrypsin inhibitor from *Alocasia macrorrhiza;*
Trypsin inhibitor from *Albizzia julibrissin;*
Trypsin inhibitor from *Acacia confusa;*

Trypsin inhibitors A, B and C from *Glycine max;*
Trypsin inhibitors KTIland KTI2 from *Glycine max;*
Latex serine proteinase inhibitor from *Carica papaya;*
Cysteine proteinase inhibitor PCPI 8.3 from *Solanum tuberosum;* and
Kunitz-type inhibitors 1 and 2 (PKI-1) from *Solanum tuberosum,*

as well as inhibitors homologous therewith isolated from other sources than those explicitly mentioned

***Potato inhibitor I family (protease inhibitors):***

Trypsin/subtilisin inhibitor from *Amaranthus caudatus;* Subtilisin inhibitor from *Momordica charantia;*
Wound-induced proteinase inhibitor I from *Lycopersicon esculentum, Lycopersicon peruvianum,* and *Solanum tuberosum,* respectively;
Subtilisin inhibitors I and II from *Phaseolus angularis;*
Proteinase inhibitor I from *Solanum tuberosum;*
Chymotrypsin inhibitor 2A (CI-2A) from *Hordeum vulgare;*
Chymotrypsin inhibitor 2B (CI-2B) from *Hordeum vulgare;*
Chymotrypsin inhibitor 1A (CI-1A) from *Hordeum vulgare;*
Chymotrypsin inhibitor 1B (CI-1B) from *Hordeum vulgare;*
Chymotrypsin inhibitor 1C (CI-1C) from *Hordeum vulgare;*
Chymotrypsin inhibitor I, a, b and c subunits from *Solanum tuberosum;*
Subtilisin inhibitor from *Vicia faba;*
Ethylene-responsive proteinase inhibitor from *Lycopersicon esculentum;*
Proteinase inhibitors I-A and I-B from *Nicotiana tabacum;*
Eglin C from *Hirudo medicinalis;*
Inhibitor of trypsin and Hageman factor from *Cucurbita maxima;* Trypsin inhibitor MCI-3 from *Momordica charantia;* and
Trypsin inhibitor I from *Nicotiana sylvestris,*

as well as inhibitors homologous therewith isolated from other sources than those explicitly mentioned.

***Bowman-Birk family of protease inhibitors:***

Bowman-birk type protease inhibitors from *Arachis hypogaea, Coix lachryma-jobi, Phaseolus angularis, Glycine max, Triticum aestivum, Arachis hypogaea, Phaseolus vulgaris, Setaria italica, Dolichos axillaris, Lonchocarpus capassa, Oryza sativa, Hordeum vulgare, Medicago scutellata, Phaseolus aureus, Phaseolus lunatus, Vicia angustifolia, Vicia faba,* and *Vigna unguiculata,* respectively; and
Wound induced trypsin inhibitors from *Medicago sativa* and *Zea mays*, respectively,

as well as inhibitors homologous therewith isolated from other sources than those explicitly mentioned.

***Squash inhibitor family:***

Elastase inhibitor from *Momordica charantia;*
Trypsin inhibitors I, II, and III from *Lagenaria leucantha;*
Trypsin inhibitor I from *Citrullus vulgaris;*
Trypsin inhibitors I, III, and IV from *Cucurbita maxima;*
Trypsin inhibitors I and II from *Luffa cylindrica;*
Trypsin inhibitors I and II from *Momordica charantia;*
Trypsin inhibitor I from *Momordica repens;*
Trypsin inhibitor II from *Bryonia dioica;*
Trypsin inhibitors IIB and IV from *Cucumis sativus;*

Trypsin inhibitor II from *Ecballium elaterium;*
Trypsin inhibitors I, II and III from *Cucumis melo* var. *Conomon;*
Trypsin inhibitors II and III from *Cucurbita pepo;* and
Trypsin inhibitor A from *Momordica charantia,*

as well as inhibitors homologous therewith isolated from other sources than those explicitly mentioned.

***Streptomyces subtilisin inhibitor (SSI) family:***

Subtilisin inhibitor from *Streptomyces albogriseolus;*
Subtilisin inhibitor-like protein-1 from *Streptomyces cacaoi;*
Trypsin inhibitor STI2 from *Streptomyces longisporus;*
Subtilisin inhibitor-like protein-2 from *Streptomyces rochei;*
Subtilisin inhibitor-like protein-3 from *Streptomyces coelicolor;*
Subtilisin inhibitor-like protein-4 from *Streptomyces lavendulae;*
Subtilisin inhibitor-like protein-8 from *Streptomyces virginiae;*
Protease inhibitor SIL-V3 from *Streptoverticillium eurocidicus;*
Protease inhibitor SIL-V1/SIL-V4 from *Streptoverticillium flavopersicus;*
Protease inhibitor SIL-V5 from *Streptoverticillium luteoverticillatus;*
Protease inhibitor SIL-V2 from *Streptoverticillium orinoci;* Alkaline protease inhibitor 2C' from *Streptomyces griseoincarnatus;*
Protease inhibitor from *Streptomyces lividans;* and
Plasminostreptin from *Streptomyces antifibrinolyticus,*

as well as inhibitors homologous therewith isolated from other sources than those explicitly mentioned.

***Bombyx family of protease inhibitors***

Fungal protease inhibitor F (FPI-F) from *Bombyx mori*, as well as inhibitors homologous therewith isolated from other sources than *Bombyx mori.*

***Wap-type 'Four-disulfide Core' Proteinase inhibitors***

Antileukoproteinase 1 from *Homo sapiens* and *Mus musculus,* respectively;
Elafin from *Homo sapiens* and *Sus scrofa,* respectively; and
Chelonianin (basic protease inhibitor) from red sea turtle,

as well as inhibitors homologous therewith isolated from other sources than those explicitly mentioned.

***Hirudin family of protease inhibitors***

Hirudins from *Hirudo medicinalis* and *Hirudinaria manillensis,* respectively, as well as inhibitors homologous therewith isolated from other sources than those explicitly mentioned.

***Factor Xa inhibitors***

Antistasin from *Haementeria officinalis, Haementeria ghilianii, Hirudo medicinalis, Hirudo nipponia,* and *Hydra magnipapillata,* respectively, as well as inhibitors homologous therewith isolated from other sources than those explicitly mentioned.

***Ascaris trypsin inhibitor family***

Chymotrypsin/elastase inhibitors from *Ascaris suum* and Trypsin inhibitor from *Ascaris suum* as well as inhibitors homologous therewith isolated from other sources than *Ascaris suum.*

***Cystatin family of protease inhibitors***

Leukocyte cysteine proteinase inhibitors 1 and 2 from *Sus scrofa;*
Stefins 1, 2 and 3 from *Mus musculus;*
Cystatins A1, A5, A8 and B from *Sus scrofa;*
Cystatins A and B from *Bos taurus;*
Stefin C from *Bos taurus;*
Cystatin B from *Ovis aries;*
Cystatins A, C, D, M, SN, S and SA from *Homo sapiens;*
Cystatins B and C from *Mus musculus;*
Cystatins C and S from *Rattus norvegicus;*
Cystatins C from *Macaca mulatta* and *Saimiri sciureus,* respectively;
Cystatin alpha (epidermal thiol proteinase inhibitor) from *Rattus norvegicus;*
Cystatin B (liver thiol proteinase inhibitor) from *Homo sapiens* and *Rattus norvegicus,* respectively;
Cystatin (colostrum thiol proteinase inhibitor) from *Bos taurus;*
Cystatins from *Gallus gallus* and *Coturnix coturnix japonica,* respectively;
Cystatin from *Cyprinus carpio;*
Cystatin from *Bitis arientas;*
Cystatin I from *Zea mays;*
Oryzacystatin-I from *Oryza sativa;*
Oryzacystatin-II from *Oryza sativa;*
Cystatin A from *Helianthus annuus;*
Cystatin B from *Helianthus annuus;*
Cystatin from *Vigna unguiculata;*
Onchocystatin from *Onchocerca volvulus;*
Cysteine proteinase inhibitor from *Solanum tuberosum;*
Cystatin WCPI-3 from *Wisteria floribunda;*
Cystatin from *Glycine max;* and
Kininogens from *Bos taurus, Homo sapiens* and *Rattus norvegicus,* respectively,

as well as inhibitors homologous therewith isolated from other sources than those explicitly mentioned.

***Calpain family of cysteine protease inhibitors***

Calpastatin from *Bos taurus, Cercopithecus aethiops, Homo sapiens, Mus musculus, Sus scrofa, Oryctolagus cuniculus, Rattus norvegicus,* and *Ovis aries,* respectively,
as well as inhibitors homologous therewith isolated from other sources than those explicitly mentioned.

***Tissue inhibitor of metalloproteinases family***

Metalloproteinase inhibitor 1 from *Bos taurus, Homo sapiens, Mus musculus, Papio cynocephalus, Sus scrofa, Oryctolagus cuniculus, Rattus norvegicus,* and *Ovis aries,* respectively;
Metalloproteinase inhibitor 2 from *Bos taurus, Homo sapiens, Mus musculus, Rattus norvegicus,* and *Gallus gallus,* respectively; and
Metalloproteinase inhibitor 3 from *Bos taurus, Homo sapiens, Mus musculus, Rattus norvegicus,* and *Gallus gallus,* respectively,

as well as inhibitors homologous therewith isolated from other sources than those explicitly mentioned.

***Carboxypeptidase A inhibitors***

Carboxypeptidase A inhibitor from *Ascaris suum*, as well as inhibitors homologous therewith isolated from other sources than *Ascaris suum.*

***Metallocarboxypeptidase inhibitors***

Metallocarboxypeptidase inhibitors from *Lycopersicon esculentum* and *Solanum tuberosum,* respectively, as well as inhibitors homologous therewith isolated from other sources than those explicitly mentioned.

***Angiotensin-converting enzyme inhibitors***

Angiotensin-converting enzyme inhibitor from *Thunnus albacares;*
Angiotensin-converting enzyme inhibitors from *Bothrops insularis;*
Angiotensin-converting enzyme inhibitors from *Bothrops jararaca;*
Angiotensin-converting enzyme inhibitor from *Agkistrodon halys blomhoffi;*
Angiotensin-converting enzyme inhibitor from *Agkistrodon halys pallas;* and
Angiotensin-converting enzyme inhibitor from *Vipera aspis,*

as well as inhibitors homologous therewith isolated from other sources than those explicitly mentioned.

***Protease inhibitors not belonging to a specific family***

Ecotin from *Escherichia coli;*
Metalloproteinase inhibitor from *Streptomyces nigrescens;*
Proteinase inhibitor from *Erwinia chrysanthemi;*
Proteinase inhibitor from *Pseudomonas aeruginosa;*
Protease A inhibitor 3 from *Saccharomyces cerevisiae;*
Protease B inhibitors 1 and 2 from *Saccharomyces cerevisiae;*
Major pepsin inhibitor PI-3 from *Ascaris suum;*
Intracellular proteinase inhibitor from *Bacillus subtilis;*
Proteinase inhibitor PTI from *Solanum tuberosum;*
Proteinase inhibitor PCI-I from *Solanum tuberosum;*
Proteinase inhibitor IIA from *Solanum tuberosum;*
Proteinase inhibitor IIB from *Solanum tuberosum;*
Proteinase inhibitors A and B from *Sagittaria sagittifolia;*
Proteinase inhibitor from *Solanum melongena;*
Trypsin inhibitor from *Brassica napus;*
Trypsin inhibitor 2 from *Sinapis alba;*
Trypsin inhibitor from *Zea mays;*
Trypsin inhibitor from *Sinapis arvensis;*
Trypsin inhibitor from *Trichosanthes kirilowii;*
Wound-induced proteinase inhibitor II from *Lycopersicon esculentum;*
Protease inhibitors LCMI I and PMP-D2 from *Locusta migratoria;*
Protease inhibitor from *Bacillus brevis;*
Marinostatins C-1, C-2 and D from *Alteromonas* sp.; and
Host protease inhibitor from bacteriophage T4,

as well as inhibitors homologous therewith isolated from other sources than those explicitly mentioned.

***Cereal alpha-amylase/trypsin inhibitor family***

Alpha amylase/trypsin inhibitor CM1 from *Triticum aestivum;*
Alpha amylase/trypsin inhibitor CM2 from *Triticum aestivum;*
Alpha amylase/trypsin inhibitor CM3 from *Triticum aestivum;*
Alpha amylase/trypsin inhibitor CM16 from *Triticum aestivum;*
Alpha amylase/trypsin inhibitor CM17 from *Triticum aestivum;*

Alpha amylase inhibitor 0.19 from *Triticum aestivum;*
Alpha amylase inhibitor 0.28 from *Triticum aestivum;*
Alpha amylase inhibitor 0.53 from *Triticum aestivum;*
Alpha amylase inhibitor WDAI-3 from *Triticum aestivum;*
Alpha amylase/trypsin inhibitor CMA from *Hordeum vulgare;*
Alpha amylase/trypsin inhibitor CMB from *Hordeum vulgare;*
Alpha amylase/trypsin inhibitor CMC from *Hordeum vulgare;*
Alpha amylase/trypsin inhibitor CMD from *Hordeum vulgare;*
Alpha amylase inhibitor CME from *Hordeum vulgare;*
Alpha amylase inhibitor BMAI-1 from *Hordeum vulgare;*
Alpha amylase inhibitor BDAI-1 from *Hordeum vulgare;*
Alpha amylase/trypsin inhibitor from *Eleusine coracana;* and
Trypsin/factor XIIa inhibitor from *Zea mays,*

as well as inhibitors homologous therewith isolated from other sources than those explicitly mentioned.

***Alpha-amylase/trypsin inhibitor homologous to thaumatin***

Alpha-amylase/trypsin inhibitor from Zea *mays* as well as inhibitors homologous therewith isolated from other sources than *Zea mays.*

***Alpha-amylase/subtilisin inhibitor family***

Alpha-amylase/subtilisin inhibitor from *Hordeum vulgare;*
Alpha-amylase/subtilisin inhibitor from *Triticum aestivum;* and
Alpha-amylase/subtilisin inhibitor from *Oryzae sative,*

as well as inhibitors homologous therewith isolated from other sources than those explicitly mentioned.

***Inhibitors of insect alpha-amylases***

Small protein inhibitor of insect alpha-amylases 1, 2 and 3 from *Sorghum bicolor milo* as well as inhibitors homologous therewith isolated from other sources than *Sorghum bicolor milo.*

***Inhibitors of mammalian alpha-amylases derived from Streptomyces species***

Alpha-amylase inhibitor HAIM I from *Streptomyces griseosporeus;*
Alpha-amylase inhibitor PAIM I from *Streptomyces olivaceoviridis;*
Alpha-amylase inhibitor HAIM II from *Streptomyces griseosporeus;*
Alpha-amylase inhibitor PAIM II from *Streptomyces olivaceoviridis;*
Alpha-amylase inhibitor AI-3688 from *Streptomyces aureofaciens;*
Alpha-amylase inhibitor Z-2685 from *Streptomyces rochei;* and
Alpha-amylase inhibitor HOE-467A from *Streptomyces tendae,*

as well as inhibitors homologous therewith isolated from other sources than those explicitly mentioned.

***Trehalase inhibitors***

Trehalase inhibitor from *Periplaneta americana* as well as inhibitors homologous therewith isolated from other sources.

***Polygalacturonase inhibitors***

Polygalacturonase inhibitors from *Phaseolus vulgaris* and *Pyrus communis* as well as inhibitors homologous therewith isolated from other sources than those explicitly mentioned.

***Fucosyltransferase inhibitors***

Fuctinins from *Rattus norvegicus* as well as inhibitors homologous therewith isolated from other sources.

***Protein kinase C inhibitors***

14-3-3 Protein beta/alpha from *Bos taurus*, *Ovis aries, Homo sapiens,* and *Rattus norvegicus,* respectively;
14-3-3 Protein epsilon from *Mus musculus, Ovis aries, Homo sapiens,* and *Rattus norvegicus,* respectively;
14-3-3 Protein eta from *Bos taurus, Mus musculus,* and *Rattus norvegicus,* respectively;
14-3-3 Protein gamma from *Bos taurus, Ovis aries,* and *Rattus norvegicus,* respectively;
14-3-3 Protein zeta/delta from *Bos taurus, Mus musculus, Ovis aries, Homo sapiens,* and *Rattus norvegicus,* respectively;
Hint protein from *Bos taurus*, *Rattus norvegicus, Homo sapiens, Mus musculus,* and *Oryctolagus cuniculus,* respectively; and
14 kDa zinc-binding protein from *Brassica juncea* and *Zea mays,* respectively,

as well as inhibitors homologous therewith isolated from other sources than those explicitly mentioned.

***cAMP-dependent protein kinase inhibitors***

cAMP-dependent protein kinase inhibitor (muscle/brain form) from *Homo sapiens, Oryctolagus cuniculus, Mus musculus,* and *Rattus norvegicus,* respectively, and
cAMP-dependent protein kinase inhibitor (testis form) from *Mus musculus,* and *Rattus norvegicus,* respectively,

as well as inhibitors homologous therewith isolated from other sources than those explicitly mentioned.

***Cyclic nucleotide phosphodiesterase inhibitor***

Cyclic nucleotide phosphodiesterase inhibitor from *Dictyostelium discoideum* as well as inhibitors homologous therewith isolated from other sources.

***Protein phosphatase inhibitors***

Protein phosphatase inhibitor 1 from *Homo sapiens, Oryctolagus cuniculus,* and *Rattus norvegicus,* respectively;
Protein phosphatase inhibitor 2 from *Homo sapiens, Oryctolagus cuniculus,* and *Rattus norvegicus,* respectively;
Heat-stable protein phosphatase 2a inhibitor I1PP2A from *Bos taurus* and *Homo sapiens,* respectively; and
Phosphatase RAPA inhibitor from *Bacillus subtilis,*

as well as inhibitors homologous therewith isolated from other sources than those explicitly mentioned.

***TCD/MRS6 family of GDP dissociation inhibitors***

Secretory pathway GDP dissociation inhibitor from *Saccharomyces cerevisiae;*
Rab GDP dissociation inhibitor alpha from *Bos taurus, Homo sapiens, Mus musculus,* and *Rattus norvegicus,* respectively;
Rab GDP dissociation inhibitor beta from *Homo sapiens, Mus musculus,* and *Rattus norvegicus,* respectively;
Rho GDP-dissociation inhibitor 1 from *Bos taurus, Homo sapiens, Caenorhabditis elegans* and *Cavia porcellus,* respectively;

Rho GDP-dissociation inhibitor from *Saccharomyces cerevisiae* ;
Rho GDP-dissociation inhibitor 2 from *Homo sapiens* and *Mus musculus,* respectively; and
Rho GDP-dissociation inhibitor from *Mus musculus,*

as well as inhibitors homologous therewith isolated from other sources than those explicitly mentioned.

***ATPase inhibitors***

Mitochondrial ATPase inhibitors from *Bos taurus, Caenorhabditis elegans, Pichia jadinii, Sus scrofa, Rattus norvegicus,* and *Saccharomyces cerevisiae,* respectively, and
Sodium/potassium ATPase inhibitor from *Sus scrofa,*

as well as inhibitors homologous therewith isolated from other sources than those explicitly mentioned.

***Phospholipase A2 inhibitory proteins***

Annexin I from *Bos taurus, Homo sapiens, Mus musculus, Sus scrofa, Rattus norvegicus, Oryctolagus cuniculus, Cavia cutleri, Gallus gallus,* and *Columba livia,* respectively;
Annexin III from *Homo sapiens* and *Rattus norvegicus,* respectively;
Annexin V from *Homo sapiens, Mus musculus, Rattus norvegicus,* and *Gallus gallus,* respectively;
Uteroglobulin from *Oryctolagus cuniculus* and *Lepus capensis,* respectively; and
Phospholipase A2 inhibitor from *Trimeresurus flavoviridis,*

as well as inhibitors homologous therewith isolated from other sources than those explicitly mentioned.

***Ribonuclease inhibitors***

Barstar from *Bacillus amyloliquefaciens;*
Ribonuclease inhibitor from *Homo sapiens, Sus scrofa,* and *Rattus norvegicus,* respectively, as well as inhibitors homologous therewith isolated from other sources than those explicitly mentioned.

***RNA polymerase inhibitors***

Bacterial RNA polymerase inhibitors from Bacteriophages T3 and T7 as well as inhibitors homologous therewith isolated from other sources.

***DNA-entry nuclease inhibitors***

Competence protein J from *Bacillus subtilis* as well as inhibitors homologous therewith isolated from other sources.

***Beta-lactamase inhibitors***

Beta-lactamase inhibitor from *Streptomyces clavuligerus* as well as inhibitors homologous therewith isolated from other sources.
To this list of enzyme inhibitors can be added functionally related inhibitors such as the inhibitor of calcium transport seminalplasmin from *Bos taurus* and *Mus musculus,* respectively, as well as inhibitors homologous therewith isolated from other sources.
To the above list of suitable scaffold molecules to be used in the invention should be added other agents having an effect on the activity of enzymes. One interesting candidate is thioredoxin.
All of the above-listed scaffold molecules can be substituted with an effective part of the complete molecule.

Properties of the scaffold molecule

Since the peptide library presumably can reach every compartment of a cell, it is beneficial if the scaffold enzyme inhibitor protein is not too large, and that it is stable towards e.g. proteolytic attack and insensitive to the reducing environment inside eukaryotic cells. Hence, its function should preferably not be dependent on the formation of disulfide bridges, since these are not formed in the cytosol or nucleus of such cells. In addition the scaffold protein should contain one or more exposed loops in which peptides can be inserted without markedly changing the structure or stability of the protein. On this basis the chymotrypsin inhibitor 2A (cf. the discussion below) is a suitable scaffold but other scaffolds such as the examples mentioned below could also be used.

The members of the peptide library introduced into target cells can effect change of the target cells' phenotype either by direct or indirect (transdominant) interaction. However, the method of the invention renders possible the identification of not only the peptide or ribonucleic acid sequence which is responsible for the direct or indirect effect on phenotype, but also allows for the purification, isolation and identification of the target molecule with which the peptide interacts.

In the present specification the barley-derived chymotrypsin inhibitor 2A has been used by way of example, mainly because this protein is very well-characterized and extremely stable. It will be understood, however, that the use of this enzyme inhibitor in the Examples in no way should limit the scope of the present invention.

Barley chymotrypsin inhibitor 2A belongs to a large family of homologous protease inhibitors mainly found in plants. This family includes barley chymotrypsin inhibitors 1A, 1B, 2A and 2B, potato inhibitor I , wound-induced tomato inhibitor I, ethylene-responsive tomato inhibitor, wild tomato fruit inhibitor I, a subtilisin inhibitor from broad bean, adzuki bean subtilisin inhibitor, pumpkin trypsin/Hageman factor inhibitor, bitter gourd inhibitor, protoplast-specific trypsin inhibitor from Nicotiana sylvestris, tobacco subtilisin inhibitor, amaranth trypsin/subtilisin inhibitor, and beach canavalia subtilisin inhibitor. The only member of this inhibitor family that is of non-plant origin is the leech elastase/cathepsin G inhibitor eglin C.

CI-2A was originally purified from the endosperm of the high-lysine barley Hiproly and shown to be a tight binding inhibitor of the microbial subtilisins Carlsberg and Novo as well as of chymotrypsin. The N-terminal amino acid residue in CI-2A has a blocked amino group as direct amino acid sequencing was unsuccessful. However, most of the amino acid sequence of CI-2A has been determined at the protein level. In addition, it was shown that CI-2A purified from barley is N-terminally processed either during synthesis and storage in the endosperm or in the process of purification. The absence of the 17 N-terminal amino acid residues does not influence the complex formation of CI-2A with subtilisins. Combining the results of amino acid sequencing and cDNA sequencing it has been deduced that CI-2A consists of 83 amino acid residues in a single polypeptide chain containing no disulfide bonds. The blocked N-terminal amino acid residue is serine. The reactive site in CI-2A has been determined to be the Met59-Glu60 peptide bond and the residues in the region Ile56-Arg62 have been demonstrated to be involved in the intermolecular contacts between inhibitor and protease.

The three-dimensional structures of uncomplexed CI-2A as well as of CI-2A complexed with subtilisin Novo are known from X-ray crystallography. The three-dimensional structure of CI-2A has also been determined using NMR spectroscopy, revealing that the reactive loop of CI-2A is dynamic. CI-2A consists of a single $\alpha$-helix docking against four $\beta$-strands. The surface loop stretches across the free side of the sheet and is composed of eight residues: Gly54-Tyr61. In contrast to most enzyme inhibitors, CI-2A lacks disulfide bonds as well as glycosylation sites. In the structures determined, only the 64 C-terminal amino acid residues are defined (L20-G83); this truncated version retains the functionality of the native protein. Comparing the complexed form with the two uncomplexed forms of CI-2A reveals few differences. The most notable difference is that the reactive site loop seems to have a less ordered structure in the uncomplexed forms than in the complexed form. The three-dimensional structure of CI-2A in complex with subtilisin Novo has also been compared to the three-dimensional structure of eglin C in complex with subtilisin Carlsberg. The two homologous inhibitors have highly similar secondary and tertiary structures.

Recombinant variants of N-terminally truncated CI-2A (CI-2A(L20-G83)) and CI-2A with an N-terminal Asp-Pro extension have been widely used to study the folding and stability of CI-2A. The structure of CI-2A in complex with subtilisin Novo has revealed that the number of intermolecular contacts between inhibitor and protease in the P4-P1 region (Ile56-Met59) of the inhibitor are much larger than in the P1'-P3' region (Glu60-Arg62).

Further aspects of the invention

Having identified a modulator according to the above-detailed methods of the invention it is normally of interest to provide large quantities thereof for the purposes of further research and development, including possible identification of the target molecule with which the modulator physically interacts.

Therefore, the invention also pertains to a method for the preparation of a replicable expression vector, the method comprising the steps of identifying a modulator by the methods of the invention, and subsequently

- isolating or synthesizing a nucleic acid sequence which encodes the modulator, and
- engineering a replicable expression vector comprising an operon which comprises, in the 5'-3' direction and in operable linkage, 1) a promoter for driving expression of the nucleic acid sequence, 2) optionally a nucleotide sequence encoding a leader peptide, 3) the nucleic acid sequence, and 4) a termination signal.

Such methods are widely known in the field of genetic engineering and molecular biology. The skilled person will find suitable guidance in e.g. Sambrook J, Fritsch EF, Maniatis T. 1989. "Molecular cloning: A laboratory manual", 2nd ed. Cold Spring Harbor Laboratory, Cold Spring Harbor, N.Y.

In this part of the invention it is preferred that the promoter is inducible; constitutive promoters are not excluded though. Depending on the choice of host cell to carry and express the vector, the promoter is selected from the group consisting of a bacterial promoter, a fungal promoter such as a yeast promoter, and a mammalian promoter. The vector can be in the form of a plasmid, a phage, a cosmid, a minichromosome, or a virus, again depending on choice of host cell and other considerations applying for the specific case. However, it is most preferred that the expression vector is capable of being integrated into the genome of a suitable host cell, since the expression therein will then be more stable over time than is the case with non-chromosomal transformation of the host cell.

Well-known vector systems are based on bacterial plasmid pBR322, λ-phage, and yeast plasmid YRp7, but other suitable and feasible choices are known to the skilled person.

After having provided a suitable expression vector as outlined above, it is preferred that the modulator is produced by transforming a suitable host cell with an expression vector prepared as described above. Such a host cell can be bacterial (e.g. *E. coli*, *Bacillus subtilis,* or any other suitable bacterial host cell), a fungus (e.g. a yeast cell such as *Saccharomyces cerevisiae* or Piccia pastoris) or a plant, insect or mammalian cell (which can be any of the above-discussed cells or cell-types suitable for use as the "substantially identical cells" in the method of the invention).

After having provided a producer cell line as described above it is now possible to produce the modulator of the invention by growing the transformed cell prepared as described above in a culture medium under conditions which facilitates expression by the cell of the randomly modified nucleotide sequence, and subsequently harvesting the expression product from the cell and/or the culture medium. Alternatively, the modulator can be produced by synthesizing the modulator by means of chemical synthesis on the basis of the sequence determined in step (e). In the case that the modulator is a peptide, the well-known techniques of solid- or liquid-phase peptide synthesis can be employed and also if the modulator is a ribonucleic acid, methods for synthetic production thereof are readily available.

An important part of the invention pertains to the isolation/identification of the target biomolecules which are engaging the modulator, the identification, isolation and production of which is described above. Hence an important part of the invention pertains to a method for isolating and/or identifying a target biomolecule, the method comprising providing a modulator according to the methods described herein and subsequently using the modulator as an affinity ligand in an affinity purification step so as to isolate the target biomolecule from a suitable sample. The affinity purification step can e.g. be an affinity chromatographic step, an affinity mass spectrometry step, or a co-immunoprecipitation step. However, any suitable method for affinity-based isolation/purification can be employed.

Alternatively, the modulator can be used as a probe against a cDNA library derived from the substantially identical cells or as bait in a two- or three-hybrid system (bacterial, fungal or mammalian).

It is preferred that the target biomolecule is a peptide or a nucleic acid, since this also allows for sequence determination thereof.

The potential use of such a target biomolecule is to employ it in a drug-development program. For this purpose it is often useful to resolve or get information about the 3-dimensional structure of the target biomolecule (by means of methods available, e.g. X-ray diffraction studies, NMR analysis, circular dichroism, etc).

Having isolated a target biomolecule as described above, the invention allows for the rational selection of a chemical compound to be used as a putative drug candidate in drug development, the method comprising the steps of

- assaying a library of chemical compounds for interaction with a target biomolecule which has been *de novo* isolated according to the methods of the invention, and
- selecting compounds which interact significantly with the target biomolecule.

Such an identified drug candidate can be a lead compound or a drug candidate as such. A "lead compound" is in the present application understood as being a compound which is not in itself suitable as a drug but which exhibits a number of characteristics which are interesting when viewed from the point of view of medical therapy. The reason such a lead compound is unsuitable could be toxicity, unsuitable pharmacokinetic or pharmacodynamic properties, difficulties relating to preparation and purification etc. In such cases, the lead compound is used as a model for *de novo* synthesis of other chemical compounds which are designed so as to be related to the active part of the lead compound in 3D structure and distribution of charged, polar and non-polar groups.

This approach can be refined by initially identifying the members of the library by methods of structure-based or non-structure based computer drug-modelling. Suitable non-structure based methods are disclosed in US 5,307,287 and US 5,025,388; a method known as CoMFA). An alternative is HASL (Hypothetical Active Site Lattice; Hypothesis Software). Both these methods are based on 3D-QSAR. A feasible structure-based approach is e.g. disclosed in WO 95/06293.

Finally, a very important part of the present invention pertains to a method for the preparation of a medicinal product, the method comprising the steps of

a) selecting a chemical compound by the methods of the invention described above,
b) performing pre-clinical tests with the chemical compound in order to assess the suitability thereof as a medicinal product,
c) entering, if the chemical compound is deemed suitable in step (b), clinical trials using the chemical compound in order to obtain market authorization for a medicinal product including the chemical compound as a pharmaceutically active substance, and
d) upon grant of a market authorization, admixing the chemical compound with a pharmaceutically acceptable carrier, excipient or diluent and marketing the thus obtained medicinal product.

In other words, also encompassed by the present invention is a method for developing a medicinal product, the method comprising that a modulator identified according to the method of the invention serves as a lead compound in the development phase or wherein a target biomolecule isolated/identified according the invention serves as an interaction probe against putative drug candidates in the drug discovery phase.

The above-outlined methods should of course take into consideration all necessary requirements in order to meet GCP and GMP standards. At any rate, this method is completely depending on the initial provision of the modulator identified according to the invention.

LEGEND TO THE FIGURES

Fig. 1: A schematic representation of pCMVbipep/CI-2A with the functional *cis*-elements found in pCMVbipep indicated.
At the top, a schematic representation of pCMVbipep is presented and the CI-2A cDNA region is expanded in the illustrations below showing the position of the various signal peptides (SEQ ID NOs: 35-37) present in pCMVbipepER/CI-2A, pCMVbipepNLS/CI-2A, and pCMVbipepSL/CI-2A, respectively. The extra amino acids added to CI-2A are written in one letter code with the essential amino acid sequences required for function underlined. Abbreviations: nuclear localization signal, NLS; endoplasmic reticulum, ER; secretoric leader, SL; retention signal, RS.

Fig. 2: Total extracts from CMVbipep/CI-2A transduced cells are capable of inhibiting the protease activity of subtilisin. Subtilisin was incubated with increasing amounts of extracts from the indicated cell lines either transduced with CMVbipep (NIH-3T3, U2OSmCAT, and 293mCAT) or CMVbipep/CI-2A (NIH-3T3/CI-2A, U2OSmCat/CI-2A, and 293mCat/CI-2A) and subsequently assayed for residual proteolytic activity. Each reaction with a given extract concentration was determined in triplicate and the shown velocities are based on the mean values.

Fig. 3: Nuclear extract from CMVbipepNLS/CI-2A, but not from CMVbipep/CI-2A transduced NIH-3T3 cells exerts CI-2A activity.

A) Subtilisin was incubated with either 2 or 10 μl of nuclear extract from NIH-3T3 cells transduced with either CMVbipep (-), CMVbipep/CI-2A (CI-2A), or CMVbipepNLS/CI-2A (NLS/CI-2A) and subsequently assayed for proteolytic activity.
B) As for A except that 0.4 or 2 μl total cell extract was used. Each reaction with a given extract concentration was determined in triplicate and the shown velocities are based on the mean values.

Fig. 4: Schematic presentation of pFAB60 constructs.
Upper panel: pFAB60/CI-20 contains the truncated version of CI-2A is inserted in frame with a pelB leader sequence at the 5' end and the deleted gene encoding a fragment of the M13 phage surface protein III (ΔgIII) at the 3'end. The pelB leader directs the expressed fusion protein to the bacterial membrane thereby facilitating incorporation of the CI-2A/ΔpIII fusion protein into phage particles.
Middle panel: pFAB60/muCI-2A contains the CI-2A cDNA including the recognition sites for *Mun*II and *Sal*I restriction enzymes. Lower panel: pFAB60/muCI-2A_rc has amino acid 58-61 substituted for the underlined 19 randomly composed amino acids (SEQ ID NO: 38, residues 2-20). The amino acids are shown in one letter codes and the numbers refers to the unmodified CI-2A amino acid sequence (cf. The numbering in SEQ ID NO: 2).

Fig. 5: CI-2A and CI-2A_rc can be displayed on the surface of phage particles.
Anti CI-2A antibodies were immobilized and incubated with 0 or 5 x $10^{11}$ phage particles, produced and purified as in Johansen et al., 1995, Protein Eng. 10, pp. 1063-1067 and quantified by OD269 measurement. CI-2A negative (-) CI-2A or CI-2A_rc carrying phage particles were used. The retained phage particles were finally detected by a horse radish peroxidase conjugated anti phage antibody. The results shown are mean values from 4 independent measurements with indicated standard deviation.

Fig. 6: Construction of a CI-2A presented library using the *Mun*I and *Sal*I cloning sites.

A) A degenerated oligo (SEQ ID NO: 33, residues 21-46) covering the recognition sites for *Mun*I and *Sal*I is converted to a double stranded form in a single extension reaction.
B) Schematic presentation of the modified cDNA of a CI-2A peptide library.
C) Three-dimensional structure of the 64 C-terminal amino acids. The substituted amino acids are shown in white with the sequence of the randomized CI-2A gene above.

Fig. 7: Construction of a CI-2A presented library using *Bam*HI and *Sal*I cloning site.

A) The 5' part of the CI-2A cDNA can be made by using overlapping oligos that after annealing are extended by the use of a DNA polymerase. The resultant fragment can then be cleaved with BamHI and *Sal*I before ligation into pCMVbipep/muCI-2A.
B) Another strategy is to use the randomized oligo in a PCR together with an upstream forward primer. Again a *Bam*HI/*Sal*I fragment can be purified an ligated into pCMVbipep/muCI-2A.

PREAMBLE TO EXAMPLES

In the following, the present invention is illustrated by way of example wherein CI-2A is used as starting point for the CellScreen™ technique adapted according to the invention so as to allow intracellular expression of a scaffold protein inhibitor which is randomly modified in the active site. This example is non-limiting, in the sense that other suitable protein inhibitors of enzymes could be used instead of CI-2A. The skilled person can readily perform the necessary substitutions and modifications necessary in order to apply the principles exemplified below using other protein inhibitors of enzymes as starting point.

EXAMPLE 1

*Materials and methods*

<u>1-a: Construction of pCMVbipep/CI-2A</u>

The 5788 bp hybrid plasmid pCMVbipep (the sequence of which is set forth in SEQ ID NO: 39 and which is shown schematically in Fig. 1 with CI-2A inserted downstream the packaging signal) consists of an AKV derived retroviral insert which has been cloned into the pUC-19 cloning vector. The retroviral insert contains a chimeric 5' LTR, allowing expression from the strong cytomegalovirus (CMV) promoter when transcription is driven from the plasmid. Following integration of the vector into a host genome, transcription is driven from the retroviral LTR. A versatile polylinker is present downstream of the packaging signal. This enables the insertion of peptide libraries in this position. Immediately downstream of the polylinker is an internal ribosomal entry site (IRES), derived from the encephalomyocarditis (EMC) virus (Koo et al. 1992, figure 2A-G). This allows efficient translation from the downstream expression cassette in a CAP independent (IRES). A Neo marker gene is found in the downstream expression cassette.

pCMVbipep includes a chimeric CMV and Akv promotor/enhancer. It was constructed from the vector plasmid constructs PUT 649 (CAYLA, toulouse FRANCE) and AkvBiPep (Duch *et al.,* unpublished, described below). AkvBiPep was digested with *Eco*RI and *Asc*I and the 2779 bp fragment from position 3293 through 5670 to 401 was isolated and ligated to a 543 bp fragment containing the enhancer/promotor of CMV position 44 to 548. This fragment was obtained by PCR amplifying an *Eco*RI and *As*cI digest of PUT 649 using the following two primers GGTCAGGAATTCTCCGGAATTGGCTAGCCTAGAGTCCGTTACATAACT (SEQ ID NO: 40) and GAGGACTGGCGCGCCGAGTGTGGGGTTCTTACCCTTTTTATAGACCTCCCACCGTACACGCC T (SEQ ID NO: 41). The resulting fragment was digested with AscI and ligated to an AscI fragment of AkvBiPep (fragment from position 828 to 3293).

AkvBiPep was constructed by digesting pBiZeo-Neo (Duch *et al.,* Biotechniques, 1999, **26**(6): 1032-4, 1036) with *Bam*HI and *Stu*I. The fragment from 1708 through 6054 to 1240 was ligated to a DNA fragment made by extending with two olignucleotides. The oligonucleotides were AGATCTCCGAGGCCTGGGACCCTTCGAATTCGTTAACTGATCAACGCGTTCTAGACTACATG GCGGCCGCGTGTTT (SEQ ID NO: 42) and GGGGGATCCAGAGCTCGAGCTTTGAAAAACACGCGGCCGCCATG (SEQ ID NO: 43). After extension and prior to ligation, the DNA fragment was digested with *Bam*HI and *Stu*I. This operation removes the Zeo gene in pBiZeo-neo and replaces them with a polylinker.

To construct pCMVbipep/CI-2A, 2 µg pCMVbipep was digested with *Bam*HI/*Xho*I as described in A-5. The 5778 base pair fragment was subsequently purified from a 0.8% agarose gel according to A-7. A CI-2A cDNA fragment encoding amino acids 21-83 (cf. the amino acid numbering in SEQ ID NO: 2) of wild type CI-2A protein fused to an in frame methionine start codon was amplified by PCR from a pUC 19 derived plasmid containing a fragment encoding the wild type CI-2A protein (cf. the nucleotide numbering in SEQ ID NO: 1) (Generous gift from Dr. Ib G. Clausen, Novo Nordic A/S, Denmark) - the cDNA fragment includes the nucleotide sequence of SEQ ID NO: 1, where nucleotides 249, 252, and 279 are T, C, and T, respectively. The PCR conditions in the 4 performed reactions were as described in A-3 using the following primers CGGGATCCATGAAGACAGTGGCCAGAG (SEQ ID NO: 3) and CGCTCGAGTCAGCCGACCCTGGGGACCT (SEQ ID NO: 4) which flank the specified region of CI-2A with recognition sites for *Bam*HI and *Xho*I. The reaction mixture was applied to 15 three-step cycles of 94°C for 1 minute, 60°C for 30 seconds, 72°C for 30 seconds followed by purification of the amplified DNA fragment as described in A-3 with a 100 µl elution volume. The purified DNA fragment was then digested (see section A-5) and purified (see section A-7) using 40 units of *Bam*HI and *Xho*I with 120 µl as final reaction volume. This fragment was ligated into the *Bam*HI/*Xho*I cleaved pCMVbipep (see section A-1) and confirmed by DNA sequencing (section A-8) using the pCMVbipep specific primer CTGTATCTGGCGGCTCCGTGG (SEQ ID NO: 5).

1-b: Construction of pmCATIREShyg

To enable transduction of non-murine cells with retroviral vectors harvested from the Bosc packaging cell line a vector (pmCATIREShyg) encoding the ecotropic receptor was constructed. The mCAT cDNA was obtained from pJET (Albritton et al. (1989) Cell **57,** pp 659-666) and inserted into the pIRESHhyg (CloneTech) to give rise to pmCATIREShyg. This was done by digesting pJET in 1 x *Eco*RI restriction enzyme buffer together with 2 units/µl *Eco*RI (see section A-5). The 5' overhangs of the *Eco*RI digestion were filled out using Klenow polymerase according to the manufacturer's protocol (New England Biolabs). After incubation for 1 hour the sample was purified by phenol/$CHCl_3$ extraction (see section A-4) followed by digestion with 20 units *Bam*HI in a reaction volume of 20 µl (see section A-6) and purified on a 1.0% agarose gel (see section A-7). This fragment was ligated into the 5699 base pair *Bam*HI/*Bst*XI fragment of pIREShyg prepared as the mCAT fragment except that the blunt ended termini were derived from the *Bst*XI site. After ligation and transformation, positive clones events were isolated and confirmed by DNA sequencing (section A-9) using the following primers ACAGCTGGCCCTCGCAGAC (SEQ ID NO: 6), CCCACTGCTTACTGGCTTAT (SEQ ID NO: 7), TGGGGCTGCACGTCATTTG (SEQ ID NO: 8), TGTGCTACGGCGAGTTTGGT (SEQ ID NO: 9), GGTTCGTGAAAGGCTCCATT (SEQ ID NO: 10), and GAAATGTTCACAATTAGCCCTG (SEQ ID NO: 11).

1-c: Construction of pCMVbipepNLS/CI-2A

An oligonucleotide encoding the SV-40 large T antigen nuclear localization signal was added in frame to the N-terminus of the truncated CI-2A version situated in pCMVbipep/CI-2A (Fig.

1). The construction was performed by PCR (see section A-2) using 25 pmol of GAAGATCTATGGCGGCCGCACCAAAAAAGAAGAGAAAGGTAGGATCCATGAAGACAGAGT (SEQ ID NO: 12) and CGCTCGAGTCAGCCGACCCTGGGGACCT (SEQ ID NO: 13) as primers and pCMVbipep/CI-2A as template. This reaction was performed in duplicate and applied to 25 cycles consisting of 94°C for 1 min, 45°C for 1 minute, 72°C 1 minute. The solution was ethanol precipitated before purification of the 250 base pair DNA fragment using a 2% agarose gel (see section A-7). The termini of the fragment were trimmed by addition of 20 units of *Bgl*II and *Xho*I and 6 µl NEBuffer 3 to a 60 µl reaction volume (see section A-6) including 1 mg/ml bovine serum albumine (New England Biolabs). The cleavage products were separated on a 2% agarose gel and the 240 base pair fragment was subsequently purified (see section A-7) with a 50 µl elution volume. This fragment was finally ligated into *Bam*HI/*Xho*I cleaved pCMVbipep (see construction of pCMVbipep/CI-2A) and confirmed by DNA sequencing (see A-8).

1-d: Construction of pCMVbipepSL/CI-2A and pCMVbipepER/CI-2A

The secretoric leader (SL) was amplified by PCR using pBapePuro containing the human immunoglobulin heavy chain signal peptide (Beerli et al. (1994) J. Biol. Chem. 269, pp23931-23936) as template and 25 pmol of GAAGATCTATGGACTGGATCTGGCGCATCC (SEQ ID NO: 14) and GAGGATCCAGAATGAGCGCCGGTAGCAG (SEQ ID NO: 15) as primers. The reaction conditions were similar to those described in construction of pCMVbipepNLS/CI-2A. The amplified 74 base pair product was after phenol/$CHCl_3$ extraction (see section A-4) digested with *Bam*HI and *Bgl*II as described in section A-5 except that the reaction included 1 µl/ml bovine serum albumin (New England Biolabs).

After digestion, the 68 base pair fragment was purified using a 3% low melting agarose gel (see section A-6) ending up with a 30 µl volume. A *Bam*HI digested pCMVbipep/CI-2A vector was prepared essentially as described for construction of pCMVbipep/CI-2A except that 10 units of calf intestinal phosphatase (Boehringer Mannheim) were present during the last hour of the incubation period. The purified secretory leader containing fragment was then ligated into the *Bam*HI cleaved pCMVbipep/CI-2A (as described in section A-1) to give rise to pCMVbipepSL/CI-2A. Positive clones were confirmed by DNA sequencing (section A-8) using CTGTATCTGGCGGCTCCGTGG (SEQ ID NO: 16) as primer.

Construction of pCMVbipepER/CI-2A (Fig. 1) was performed by addition of a retention signal to the C-terminus of CI-2A in the context of pCMVbipepSL/CI-2A. The retention signal was added in frame with the CI-2A sequence by PCR using CTAATCTAGACTACAGCTCGTCCTTGTAGTCCTCGAGGCCGACCCTGGGGACCTG (SEQ ID NO: 17) and CGGGATCCATGAAGACAGAGTGGCCAGAG (SEQ ID NO: 18) and pCMVbipep/CI-2A as template. The 237 base pair fragment was digested with *Xba*I instead of *Xho*I and ligated into a *Bam*HI/*Xba*I digested pCMVbipepSL/CI-2A prepared as *Bam*HI/*Xho*I digested pCMVbipep. All reaction parameters were as described for construction of pCMVbipepNLS/CI-2A.

1-e: Construction of pCMVbipep/muCI-2A

Two silent mutations were introduced into the CI-2A sequence by a 5 step PCR procedure. The first PCR exchanged $C^{192} \rightarrow A$ (cf. The numbering in SEQ ID NO: 1) and the product was after purification used as reverse primer for amplification of the CI-2A encoding region in pCMVbipep/CI-2A. This $C^{192} \rightarrow A$ mutated fragment was used as template for introducing the second mutation substitution of $T^{300} \rightarrow C$ (cf. The numbering in SEQ ID NO: 1) and thereby leading to a PCR-product including the previously introduced mutation. The double mutated fragment was used as forward primer to amplify the pCMVbipep/CI-2A defined reading frame. All the PCRs were done in duplicate and in essence performed as described in section A-2 with 25 cycles of 94°C for 30 seconds, 55°C for 30 seconds, and 72°C for 30 seconds. The products were pooled before purification. In the first PCR, 50 ng of pCMVbipep/CI-2A was used as template with 50 pmol of the following primers CCGGCCTTATTCCAAGCGGC (SEQ ID NO: 19) and CTGCCGGTGGGTACAATTGTGACCATGG (SEQ ID NO: 20). The 248 base pair product was purified using the QIAquick PCR purification kit (see section A-3) with 50 µl as elution volume (PCR-product 1). The second PCR consisted of 12.5 µl PCR-product 1 and 50 pmol CTGTATCTGGCGGCTCCGTGG (SEQ ID NO: 21) as primers with 50 ng pCMVbipep/CI-2A as template. The complete reaction was loaded on a 2% agarose gel and 448 base pair product was purified as described in section A-6 (PCR-product 2). This product was then used as template in the third PCR that besides the 12.5 µl PCR-product 2 consisted of 50 pmol CTGTATCTGGCGGCTCCGTGG (SEQ ID NO: 22) and CGAGTTTGTCGACAAAGAGGCGGACGCGATCGATGCGATATTCC (SEQ ID NO: 23) leading to amplification of a 248 base pair fragment (PCR-product-3). This fragment was purified as PCR-product 2 before using 12.5 µl as forward primer together with 50 pmol of CCGGCCTTATTCCAAGCGGC (SEQ ID NO: 24) and 50 ng pCMVbipep/CI-2A as template. The double mutated 448 DNA fragment was after gel purification PCR amplified using 50 pmol of CCGGCCTTATTCCAAGCGGC (SEQ ID NO: 25) and CTGTATCTGGCGGCTCCGTGG (SEQ ID NO: 26). The amplified double mutated fragment was inserted into pCMVbipep described for construction of pCMVbipep/CI-2A.

1-f: Construction of pFab60/CI-2A and pFAB60/muCI-2A

The fragment of the CI-2A cDNA that encodes amino acids 21-83 of the wild type protein was inserted into the phagemid vector pFAB60 (Johansen et al., 1995, Protein Eng. 10, pp 1063-7). The CI-2A cDNA fragment was amplified by PCR using pCMVbipep/CI-2A as template with 10 pmol of the primers ATTTGCTAGCTGCACAACCAGCAATGGCACTGAAGACAGAGTGGCCAGAGTTGG (SEQ ID NO: 27) and ATAAGAATGCGGCCGCGCCGACCCTGGGGACCTGGGC (SEQ ID NO: 28) in 4 reactions performed under conditions similar to those described under construction of pCMVbipep/CI-2A. The purified 237 base pair fragment and 2 µg pFAB60 were digested in parallel using 10 units *Nhe*I and *Not*I in 50 ml 1 x NEBuffer 4 including 1 µg/ml bovine serum albumin (see sections A-5 and A-6). The digested 225 and 4655 base pair fragments were subsequently purified using 1% agarose gel and ligated to each other (see section A-7) and A-1, respectively. Correct insertion was finally confirmed by DNA sequencing using CACACAGGAAACTATGA (SEQ ID NO: 29) as primer (See section A-8). An identical approach was utilized for constructing pFAB60/muCI-2A except that pCMVbipep/muCI-2A was used as CI-2A cDNA source.

1-g: Construction of pFab60/muCI-2A_rc

Substitution of amino acid 59 to 62 in the full length CI-2A sequence with a 19-mer randomly composed amino acid sequence was performed in the context of pCMVbipep/muCI-2A and the modified CI-2A was subsequently moved to pFAB60 following the same procedure as for constructing pFAB60/CI-2A. The coding region for the amino acid sequence was obtained from a synthetic oligo that was amplified by PCR. Four parallel reactions were performed using 12.5 pmol of CTGCCGGTGGGTACAATTGTGCTGCGCTACATGGACCGCGCAATAGTGATGAACGTGAACAT TAGCGCACGCAAACTACGGATTGATCGCGTCCGCCTCTTTGTCGACAAACTCG (SEQ ID NO: 30) as template with 50 pmol of the primers CGAGTTTGTCGACAAAGAGGCGGAC (SEQ ID NO: 31) and TCTGCCGGTGGGTACAATTG (SEQ ID NO: 32). These reactions were applied to 25 cycles of 94°C for 2.5 minutes, 45°C for 2 minutes, and 72°C for 2 minutes with all other conditions as described in section A-2. The product was purified by phenol/$CHCl_3$ extraction and dissolved in 30 µl sterile $H_2O$. To facilitate insertion into muCI-2A, 5 µl of the fragment was supplied with 20 units *Mun*I (Boehringer Mannheim), 5 µl 10 x SureCut buffer M (Boehringer Mannheim) and sterile $H_2O$ to adjust the volume to 50 µl, and incubated at 37°C for 4 hours. After addition of 6 µl 10 x *Sal*I restriction enzyme buffer and 3 µl $H_2O$ together with 20 units of *Sal*I the incubation continued for 4 hours. Purification of the 89 base pair fragment was done using a 2.5% low temperature agarose gel (see section A-6). Preparation of the *Mun*I/*Sal*I digested pCMVbipep/muCI-2A was in principle similar to the procedure described above. Four µg pCMVbipep were digested with 30 units of *Mun*I in a 150 µl reaction followed by addition of 30 units *Sal*I in 50 µl 4 x *Sal*I restriction enzyme buffer and purified using a 1% gel as described in section A-7. The ligation and confirmation procedures were performed as for the construction of pCMVbipep/CI-2A.

1-h: Library construction

The degenerated oligo TCTGCCGGTGGGTAGAATTCNNNNKNNKNNKNNKNNKNNKNNKNNKCGGATTGATCGCGTCC GCCTCTTTGTCGACAAACTCG (SEQ ID NO: 33) was converted into a double stranded form by an extension reaction. The oligo was mixed with a 3-fold excess of CGAGTTTGTCGACAAAGAGGCGGAC (SEQ ID NO: 34) in 1 x SuperTaq buffer (Enzyme Technologies Ltd.) including 8 units SuperTaq polymerase (Enzyme Technologies Ltd.) and 0.2 mM dNTP. Heating of the mixture to 94°C for 1 minute followed by 45°C for 2 minutes was done to ensure a sufficient annealing between the oligos before the temperature was changed to 55°C for 45 minutes to increase polymerase activity. After the reaction was completed, the sample was phenol/$CHCl_3$ extracted before cleavage of 1/3 of the product with 100 units *Eco*RI and *Sal*I in 100 µl 1 x *Eco*RI buffer including 1 µg/ml bovine serum albumin (See section A-6). The complete digested 56 base pair DNA fragment was purified on a 2% low temperature agarose gel (see section A-6) and ligated (see section A-1) into the *Mun*I/*Sal*I cleaved pCMVbipep/muCI-2A described in Example 1-g. Randomly chosen colonies were sequenced as described for construction of pCMVbipep/CI-2A.

1-i: Subtilisin assay

The cell extracts were diluted to the indicated amounts in 10 µl PBS and added to 25 µl 0.1 M Tris/HCl pH 8.6 containing $5 \times 10^{-8}$ M Subtilisin Carlsberg (Sigma). After incubation at 25°C for 30 minutes, 25 µl 0.1 M Tris/HCl pH 8.6 containing 5 mM N-succinyl-Ala-Ala-Pro-Phe-p-Nitroanilide (Sigma) was added and the substrate conversion was followed by measuring the absorbency at 405 nm every two minutes until the reaction had reached exhaustion.

1-j: Cell lines and culturing conditions

All the cells were cultured in Dulbeco's modified Eagle's medium (DMEM) containing 10% fetal calf serum or 10% new born calf serum, 1% L-glutamine, and 50 µg/ml penicillin/streptomycin and incubated under standard cell culture conditions.

The 293mCAT cells and U20SmCAT cells are derived from 293 (ATCC# CRL-1573) and U2OS (ATCC# HTB-96) by stable transfection with pmCAT/IRES-hyg. The 293 cells were transfected by the Calcium co-precipitation method outlined for transfection of the Bosc packaging cells and the U2OS cells were tranduced using the Fugene transfection kit according to the manufacturer's protocol (Boehringer Mannheim). Stable transfectants were selected by culturing in the presence of 150-200 µg active hygromycin B/ml (Sigma). After transducing 293mCAT or U2OSmCAT with retroviral vectors the selection was shifted to 0.6 mg/ml or 0.4 mg/ml geniticin (GibcoBRL), respectively.

The murine NIH-3T3 cell line (ATCC# CRL-165) was upon transduction with the retroviral vectors cultured in the presence of 0.6 mg/ml geniticin.

1-k: Transduction of NIH-3T3, 293mCAT, and U20SmCAT cells

For production of retroviral vector particles, pCMVbipep derived constructs were transfected into the BOSC packaging cell line using a $CaPO_4$ co-precipitation method. BOSC packaging cells (also known as BOSC23 cells, cf. WO 94/19478) were diluted to $5 \times 10^5$ cells/cm$^2$ the day before the transfection and washed once in complete DMEM 2 hours before the transfection. The $CaPO_4$ co-precipitated mixtures were prepared by diluting 10 mg of pCMVbipep or the pCMVbipep derived construct with 10 mg salmon sperm DNA in 450 ml $ddH_2O$ and adding 50 ml 2.5 M $CaCl_2$. These solutions were slowly added to 500 ml 2 × HEPES-buffered saline pH 7.05 (280 mM NaCl, 1.5 mM $Na_2HPO_4$, 50 mM HEPES/NaOH pH 7.05) under gentle shaking followed by two to five minutes of incubation at 25°C before adding the precipitate to the prepared BOSC cells. After 24 hours of incubation, the cells were washed twice in PBS (137 mM NaCl, 2.7 mM KCl, 8.3 mM $Na_2HPO_4$ 1.4 mM $KH_2PO_4$) and further cultivated in 10 ml DMEM including supplements for another 24 hours. The media from the transfected BOSC cells were collected and diluted from 10 to $10^5$-fold in complete DMEM including 6 mg/ml Polybrene (Sigma). The recipient cells, which had been plated at $10^4$ cells/$cm^2$ and incubated at standard cell culture conditions for 24 hours in complete DMEM, were exposed to virus-containing media for 24 hours at standard conditions. After this incubation period, the transduced cell were washed twice in PBS and incubated in complete DMEM including geneticin.

1-1: Preparation of total cell extracts

The CMVbipep/CI-2A or CMVbipep transduced NIH-3T3 cells were harvested from two confluent T175 cell flasks by incubation with 5 ml 0.5 × Trypsin-EDTA solution (GibcoBRL)/plate. The recovered cells were diluted 1:1 in complete DMEM and subsequently collected by centrifugation, washed twice in 10 ml complete DMEM and twice in 1 ml PBS. Finally, the cells were suspended in 100 µl PBS. The cells were rendered permeable by three cycles of freezing in liquid nitrogen and thawing by incubation at 37°C and subsequently centrifuged at 20000 × g for 15 minutes to remove cell debris. To inactivate endogenous protease activity, the extracts were incubated at 65°C for 15 minutes and re-centrifuged.

1-m: Preparation of nuclear extracts

Two approximately 80% confluent T-175 cell flasks of each cell line were used. The cells were harvested by addition of 3 ml Trypsin/EDTA solution (GibcoBRL) to each flask followed by centrifugation of the 6 ml cell suspensions. All the following reactions were performed at 4°C using chilled solutions. The harvested cells were washed in 10 ml complete DMEM, 10 ml PBS, and 2 times in 1 ml PBS. After the last wash the cells were suspended in 1 ml NP40 lysis buffer (10 mM Tris/HCl pH 7.4, 10 mM NaCl, 3 mM $MgCl_2$, and 0.5% NP-40), mildly mixed and incubated on ice for 5 minutes. The cells were then collected by centrifugation at 500 x g at 4°C for 5 minutes and suspended in 1 ml NP-40 buffer and the nuclei were immediately harvested by repeating the centrifugation. Proteins were extracted from the nuclei by suspension in 50 µl low salt buffer (20 mM HEPES pH 7.9, 25% glycerol, 0.02 M KCl, 1.5 mM $MgCl_2$, 0.2 mM EDTA) followed by addition of 50 µl High salt buffer (Low salt buffer supplied with 1 M KCl). After 30 minutes incubation on ice, the nuclear extract was cleared by centrifugation at 20000 x g for 30 minutes. The supernatants were stored at -20°C.

1-n: SDS-PAGE and Western blots

Samples were supplied with ¼ volume of SDS-load buffer (NOVEX) including 1/10 volume 1 M dithiotreitol and heated to 95°C for 2 minutes before loading on a NuPage (NOVEX) 4-12% gradient SDS-gel in 1 x MES buffer (NOVEX) and run at a constant current of 40 mA. The gels were equilibrated in blotting buffer (10mM CHAPS/NaOH pH 11.0, 10% Methanol, 0.5% SDS) for 5 minutes before transfer of the proteins to a 0.2 µm Obititran BA-S 83 membrane (Schleicher & Schoell) by semi dry blotting for 70 minutes. The membrane was allowed to air dry before blocking 1 hour at room temperature in ECL-buffer (50 mM Tris/HCl pH 7.4, 150 mM NaCl, 5 mM EDTA, 0.5% Gelatin, 0.1% NP-40). After blocking, the ECL-buffer was displaced with anti CI-2A rabbit serum diluted 1:1000 in ECL-buffer and the incubation was continued for 1 hour. The membrane was subsequently washed 3 times, each by incubation for 20 minutes in ECL-buffer before adding 1:5000 fold diluted Horse Radish Peroxidase conjugated goat anti rabbit serum (Dako) in ECL-buffer and incubated and washed as described above. The development of the signal was done using an enhanced chemoluminescent kit according to the manufacturer's protocol (Amersham-Pharmacia).

1-o: Production of anti CI-2A polyclonal antibodies, immunoprecipitation.

Rabbits were immunized with 100 µg full length recombinant CI-2A (generous gift from Dr. Ib G. Clausen, Novo Nordic A/S, Denmark) in complete Freunds adjuvant (Sigma) and boosted 4 times with 3 weeks intervals by injecting 50 µg CI-2A formulated in incomplete Freunds adjuvant. The anti CI-2A response was detected by ELISA with recombinant CI-2A immobilized on a MaxiSorb plate (Nunc). Several dilutions of the CI-2A serum were allowed to bind and subsequently detected with a horse radish peroxidase conjugated goat anti rabbit antibody (Dako). All reactions were performed as described in example 1-p.

Protein A containing sepharose CL-4B beads (Pharmacia Biotech) were pre-washed in dilution buffer (PBS including 0.1% Triton X-100, 0.1% bovine serum albumin) before addition of 40 µl bead volume to 100 µl of the harvested media together with 10 µl of the anti CI-2A antibody. The mixtures were rotating at 4°C for 1 hour followed by 3 washes in dilution buffer. The beads were suspended in 2 x SDS loading buffer and run on a SDS-PAGE before detection of CI-2A by western blot (Example 1-n).

1-p: ELISA

Purified pig anti rabbit polyclonal antibody (Dako) was diluted to 120 ng/ml in 0.1 M $NaHCO_3$ pH 8.5 before 50 µl aliquots were added to each well in a MaxiSorb plate (Nunc) and incubated at 4°C over night. The plate was washed 3 times in ELISA wash buffer (0.01% pyrophosfate, 500 mM NaCl, 0.1% Triton X-100) and 50 µl rabbit anti CI-2A serum diluted 1:200 in binding buffer (PBS supplied with 0.5% bovine serum albumin, 0.5% Tween-20, 10% glycerol) was added to each well. After 1 hour of incubation at room temperature, the washing procedure was repeated and the indicated number of phage particles was added in binding buffer. The phage particles were allowed to react for 1 hour at room temperature before washing the plate 10 times in ELISA wash buffer. Bound phage particles were detected by using a horse radish peroxidase conjugated anti phage antibody (Pharmacia biotech) by binding and washing procedures as described above. The horse radish peroxidase was finally assayed using OPD-tablets according to the Manufacturer's protocol (Sigma).

Standard reactions:

A-1: Ligation and transformation of *Escherichia coli* by electroporation

- 30 ng vector
- 2 µl fragment (containing a fragment concentration resulting in a 10:1 relation between fragment:vector)
- 2 µl 10 x T4 DNA ligase reaction buffer (New England Biolabs)
- 300 units T4 DNA ligase (New England Biolabs)
- Sterile $H_2O$ to adjust the volume to 20 µl
- 198 cycles consisting of 30°C for 30 seconds and 10°C for 30 minutes followed by 16°C over night

The ligation products were transformed into *E. Coli* by electroporation:

- 10 µl of the ligation reaction was dialyzed against 2 ml sterile $H_2O$
- 2 µl of the dialyzed ligation reaction were mixed with 25 µl competent *E. Coli* in a 0.1 cm electroporation cuvette (Bio-Rad) and pulsed with 2 kV
- Addition of 1 ml of SOC-media (20 g Bacto-Tryptone, 5 g Yeast extract, 0.5 g NaCl, 0.19 KCl, dissolved in 950 ml $H_2O$ and adjusted to pH 7.0 using NaOH and supplied with 50 ml 20% glucose and $MgCl_2$ to a final concentration of 10 mM before use)
- Incubation at 37°C for 0.5-1 hours before streaking 50 µl
- 200 µl on LB plates (100 ml LB (10 g Bacto-Tryptone, 5 g Yeast extract, 5 g NaCl, $H_2O$ to 1 l) including 1.5 g agar and 50 mg/ml carbenicillin)

The electroporation competent *E. Coli* cells were prepared as described below:

- An over night culture was used to inoculate 37°C LB to achieve an $OD_{600}$ below 0.020
- The culture was incubated at 37°C until $OD_{600}$ was between 0.8-1.0 and subsequently chilled on ice
- The chilled culture was harvested by centrifugation
- The harvested bacteria were suspended in 300 ml 10% ice cold glycerol solution
- Repetition of the centrifugation
- The suspension and centrifugation steps were repeated twice
- The bacteria were suspended in 50 ml 10% ice cold glycerol solution and recollected by centrifugation
- The bacteria were suspended in 10% ice cold glycerol buffer to a final density at 6-12 x $10^{12}$ cells/ml and stored at -80°C in aliquots

A-2: Polymerase chain reactions using TaqGold™

- 100 ng template
- 10 pmol of specified primers, unless otherwise indicated
- 5 µl 10 x TaqGold™ reaction buffer (Perkin Elmer)
- 3 µl $MgCl_2$ solution for TaqGold™ (Perkin Elmer)
- 0.4 µl 25 mM dNTP (dGTP, dCTP, dATP, and dTTP)
- 0.4 µl 5 units/µl TaqGold™ (Perkin Elmer)
- Sterile $H_2O$ to 50 µl
- All reactions were initiated by 10 minutes at 95°C and after the specified cycles followed by 72°C for 4 minutes.
- For purification of PCR products see section A-3

A-3: PCR using Vent™ DNA polymerase

- 100 ng template
- 10 pmol of specified primers, unless other is indicated
- 5 µl 10 x Vent™ DNA Polymerase reaction buffer (New England Biolabs)
- 0.4 µl 25 mM dNTP
- 0.4 µl 5 units/µl Vent™ DNA Polymerase (New England Biolabs)
- Sterile $H_2O$ to 50 µl
- All reactions were initiated by 1 minute at 94°C and after the specified cycle program followed by 72°C for 4 minutes

The amplified DNA fragments were purified by phenol/$CHCl_3$ extraction followed by ethanol precipitation if the size was less than 100 base pairs. Otherwise, the QIAquick™ PCR-purification kit was used (Qiagen). The PCR-purification kit was used according to the manufacturer's protocol with 50 µl as the elution volume.

A-4: Phenol/$CHCl_3$ extraction

- The sample volume adjusted to at least 200 µl with sterile $H_2O$
- Addition of 1 volume Phenol pH 6.7 (supplier)
- Mixed extensively and centrifuged.
- Addition of 1 volume phenol/$CHCl_3$ 1:1 solution to the water phase
- Repeat the mix-spin procedure
- Addition of 1 volume $CHCl_3$ to the water phase
- Repeat mix-spin procedure
- Water phase supplied with 1/10 volume 3M NaAc pH 6.0 and 2.5 volume -20°C 96% ethanol
- Centrifuged at 20.000 x g for 20 minutes, displacing the supernatant with 70% ethanol and repeated the centrifugation for 2 minutes
- After the DNA was air dried it was dissolved in sterile $H_2O$

A-5: Vector preparations

The plasmid DNA used for vector preparations was purified by Qiagen maxi prep columns according to the manufacturer's protocol (Qiagen). All the used restriction enzymes and their reaction buffers were purchased from New England Biolabs unless otherwise indicated.

- 5 µg - 10 µg vector DNA
- Approximately 5 to 10 units of the indicated restriction enzymes pr. µg of plasmid DNA
- 1/10 of the final volume 10 x *Bam*HI restriction enzyme buffer unless other is indicated
- Sterile $H_2O$ to the indicated final volume
- Incubation at 37°C until complete digestion was achieved
- 65°C for 20 minutes

- Purification of the digested fragment as described in A-7

A-6: Fragment preparation

As for vector preparation (see A-6) except for the amount of DNA and that the purification of cleaved DNA fragments smaller that 100 base pairs were done using low melting agarose. The agarose slice was supplied with 300 μl 0.3 M NaAc, pH 6.0 and incubated at 65°C until the agarose was completely melted followed by phenol/$CHCl_3$ extraction and ethanol precipitation (see A-4) to purify the DNA.

A-7: gel extraction using Qiagen gel purification kit

- Samples were added 1/6 volume DNA load buffer (60% glycerol, 0.025% Bromphenol blue, 0.025% Xylene Cyanol)
- Loaded on an agarose gel in 1 x TBE (89 mM Tris-borate, 89 mM Boratic acid, 2 mM EDTA)
- After a satisfactory separation was achieved the DNA was extracted using the Qiagen gel extraction kit according to the manufacturer's protocol with an elution volume of 50 μl onless otherwise indicated

A-8: DNA sequencing

All DNA sequencing was performed with the DNA sequencing Kit BigDye™ terminator cycle sequencing (Perkin Elmer) using a 25 cycle program consisting of 96°C for 10 seconds, 50°C for 5 seconds, and 60°C for 4 minutes. The amount of DNA was between 0.2 and 0.5 μg with 3.2 pmol of the indicated primers. The BigDye™ was diluted twice according to the manufacturer's advice. After the cycle program, the DNA was ethanol precipitated and extensively washed with 70% ethanol before analyses using an ABI prism 310 sequencing machine (Perkin Elmer).

EXAMPLE 2

*Expression of CI-2A in mammalian cells*

A potential scaffold for intracellular presentation of peptide libraries expressed from the retroviral vectors utilized in the CellScreen™ technology should not have any significant effects on neither the retroviral replication cycle nor the viability of the transduced mammalian cells. Furthermore, the scaffold must retain a stable core structure to ensure a constrained peptide presentation. In order to test whether CI-2A fulfills these requirements we constructed pCMVbipep/CI-2A (See Example 1-a and Fig. 1). The CI-2A expression construct was in parallel with pCMVbipep transiently transfected into BOSC packaging cells to produce viral particles for transduction of NIH-3T3, 293mCAT, and U20SmCAT cells (Example 1-k). The titers obtained by transduction with the two viral vectors were similar, indicating that the CI-2A expression unit does not interfere with viral packaging and infection.

Total cell extracts were prepared from the transduced NIH-3T3, 293mCAT, and U20SmCAT cells and analysed for the presence of CI-2A by western blot to obtain a direct proof of the CI-2A expression (Examples 1-1 and 1-n). A rabbit anti CI-2A serum raised against wild type CI-2A recognized a protein with migration properties that corresponded to that expected for the CI-2A protein expressed from CMVbipep/CI-2A. Even an overexposure of the western blot did not reveal any bands with similar mobility properties in the extracts derived from pCMVbipep transduced cells thereby showing that soluble CI-2A is expressed by CMVbipep/CI-2A and tolerated by the tested cell lines.

Interestingly, a CMVbipep/CI-2A specific band with a slightly lower mobility was reproducibly detected by western blots. The nature of this band is uncertain at present. The decreased mobility is consistent with a slight secondary modification such as a phosphorylation, although no experimential proof exists. Other more trivial and less likely explanations could be a non-consensus start codon use or that the CI-2A stop codon is leaky thereby giving rise to an extended CI-2A protein. The inventors are currently in the process of characterizing the expression products more detailed by N-terminal amino acid sequencing and Mass spectrometry.

A consistent constrained presentation of a peptide library in a scaffold context demands that the structure of the scaffold is stable in the given environment. Since CI-2A is naturally found in the seeds of barley, the shift to the environment inside mammalian cells could influence the folding kinetics and thereby the stability of CI-2A. The protease inhibitory activity of CI-2A provides a simple method for assaying functionality (Example 1-i), which reflects the amount of native folded CI-2A. Increasing amounts of the total cell extracts used for the western blots were pre-incubated with subtilisin before measuring the residual protease active by addition of a chromogenic substrate (Fig. 2). The presence of 0.1-2 μl extract from CMVbipep/CI-2A transduced cells completely blocked subtilisin activity. In contrast, when increasing the amount of the extracts from CMVbipep transduced cells to as much as 10 μl no effects on the subtilisin activity was observed. This result proves that the decrease in subtilisin activity observed using extracts from CMVbipep/CI-2A transduced cells is due to expression of CI-2A. Furthermore, the observation that CI-2A extracted from mammalian cells is functional suggests a native structure inside the cells, which support that at random peptide library will be presented by CI-2A in a constrained manner. By comparing the amount of CI-2A containing extract required to inhibit subtilisin to a standard curve based on purified CI-2A, a rough estimation of the active CI-2A concentration can be obtained. This subsequently allows a calculation of the cellular concentration. By doing so, we estimated the CI-2A content in the tested cell lines to be in the μM range.

The combined evidence from the experiments described above suggests that CI-2A is tolerated as an intracellularly located protein in mammalian cells at a sufficient concentration to exert a biological activity. Furthermore, the pronounced CI-2A activity found in cell extracts indicates a native conformation enabling a constrained peptide presentation from the loop region.

EXAMPLE 3

*Expression of CI-2A fusion proteins with a defined subcellular localization*

A number of biological reactions are restricted to defined cellular compartments. To increase the probability of selecting peptides that interfere with such types of reactions we fused amino acid sequences to CI-2A that in other contexts have been shown to direct the fusion protein to a defined subcellular localization. Signals mediating localization to either the endoplasmic reticulum or the nucleus were added to test whether or not a restricted localization of CI-2A could be achieved.

The nuclear localization signal from the SV-40 large T-antigen was fused to the N-terminal of CI-2A thereby giving rise to the pCMVbipepNLS/CI-2A construct (Fig. 1 and Example 1-c). NIH-3T3 cells were transduced with CMVbipepNLS/CI-2A, CMVbipep/CI-2A, and CMVbipep derived retroviral particles. Western blotting and the subtilisin activity assay (see example 1-i and 1-n) were subsequently used to analyze the CI-2A content in total extracts and nuclear extracts prepared from all the transduced cell lines. Neither the total extracts nor the nuclear extracts derived from the CMVbipep transduced cells were able to interfere with the protease activity of subtilisin, consistent with the results described in example 2. In contrast, both the nuclear extracts and the total extracts derived from CMVbipepNLS/CI-2A blocked inhibiting the protease activity of subtilisin whereas only the total extract of CMVbipep/CI-2A exerted CI-2A activity (Fig. 3). Western blotting revealed an equal amount of CI-2A in the total extracts from CMVbipep/CI-2A and CMVbipepNLS/CI-2A transduced cells, thereby indicating a similar expression level. Consistent with the activity test, the amount of CI-2A detected in the nuclear extracts by western blotting was at least 10-fold higher for NLS/CI-2A than for CI-2A. Thus, two independent types of analysis support that the presence of the NLS results in an increased concentration of NLS/CI-2A in the nucleus.

Endoplasmic reticulum localization requires targeting to this compartment and subsequently an ongoing retention. To achieve this, the pCMVbipepER/CI-2A construct contains a secretoric leader (SL) peptide and a retention peptide fused to the N-terminal and C-terminal of CI-2A, respectively (Fig. 1 and Example 1-d). To be able to evaluate the functionality of the retention signal, the pCMVbipepSL/CI-2A construct that only contained the secretoric leader peptide was made (Fig. 1 and see example 1-d). NIH-3T3 cells were transduced with these retroviral vectors in conjunction with the CMVbipep and CMVbipep/CI-2A. These constructs allow investigation of the activity of both the leader peptide and the retention signal by determination of the amounts of CI-2A protein secreted to the cell media. After media exchange, the secreted CI-2A protein was detected at different time points by a combined immunoprecipitation western blot procedure (cf. 1-o). SL/CI-2A was detected after 3 hours incubation and significantly increased during the incubation periode. In contrast, ER/CI-2A was not detectable before 5.5 hours of incubation and thereafter only at a level comparable to that produced by the CMVbipep/CI-2A transduced cells. The CI-2A found in the media from the CMVbipep/CI-2A and CMVbipepER/CI-2A two cell lines is therefore likely to be due to cell death instead of active secretion. In summary, the presence of the leader peptide increased the secretion of CI-2A, but this secretion was significantly delayed by addition of the KDEL retention signal. The combined data propose that the CI-2A expressed from CMVbipepER/CI-2A becomes translocated to the endoplasmic reticulum.

By defining the subcellular localization of a peptide library, the likelihood of isolating active peptides interfering with reactions known to be restricted to occur at such locations can be significantly increased. In addition to the examples described above, one could also target CI-2A to other locations such as the cell membrane, mitrocondria, lysosomes etc.

EXAMPLE 4

*Displaying CI-2A on phage particles*

The phage display technology has since its discovery been used extensively for screening of peptide libraries. Phage display can be used in combination with CellScreen™ to enrich the peptide library for binders to e.g. crude cell extracts or whole cells and thereby reduce the number of peptides needed to be handled in the biological screening systems. We therefore tested the applicability of displaying CI-2A at the surface of phage particles by insertion of CI-2A into the pFAB60 phagemid (Fig. 4 and Johansen et al. (1995), Protein Eng. 10, pp 1063-1067). The presence of CI-2A on the surface of pFAB60/CI-2A derived phage particles was verified using both an ELISA assay and the subtilisin assay (Example 1-o & 1-i). In the ELISA assay, immobilized rabbit anti CI-2A polyclonal antibodies retained phage particles derived from pFAB60/CI-2A at levels several orders of magnitude higher than CI-2A negative phage particles (Fig. 5). Consistent with this result, subtilisin activity could only be inhibited by the pFAB60/CI-2A derived phage particles. These two experiments clearly demonstrate that CI-2A can be displayed on the phage surface and therefore fulfill the features necessary for a scaffold protein in both the phage display and the CellScreen™ technologies.

The loop region of CI-2A will be extensively modified in the situation where a peptide library is presented by CI-2A. To mimic this situation and test whether phage particles presenting such modified CI-2A proteins could be produced we exchanged 4 amino acids situated in the loop region with a 19-mer randomly composed peptide, thereby generating pFAB60/CI-2A_rc (Fig. 4 and Example 1-g). When analyzed by ELISA, a significant signal was obtained although it was slightly decreased compared to the phage particles carrying the unmodified CI-2A (Fig. 5). This variation could be due the presence of an important antibody recognition site in the loop region. In conclusion, the fact that phage particles displaying the modified CI-2A generated a signal comparable to that obtained for the pFAB60/CI-2A containing phage particles suggests that CI-2A can be displayed independently of the amino acid composition in the loop region.

EXAMPLE 5

*Constructions of CI-2A presented peptide libraries*

To facilitate the exchange of amino acids situated in the loop of CI-2A, recognition sites for *Mun*I and *Sal*I were introduced into the CI-2A cDNA in pCMVbipep/CI-2A by silent mutagenesis to generate pCMVbipep/muCI-2A (Example 1-e). The presence of the cleavage sites enables a non-PCR based library construction procedure. In this procedure, a synthetic oligo that includes the randomized region is converted into a double stranded form before cloning into the *Mun*I/*Sal*I sites (Fig. 6). The feasibility of producing a peptide library using this procedure was tested by a small scale ligation followed by sequencing of a limited number of randomly chosen clones. Out of 8 sequenced clones, all contained insertion of the random oligo and none of the insertions encoded identical peptides. This suggests that that transfer of diversity from the synthetic oligo into a biologically active form is possible using this strategy.

The use of the *Mun*I site to generate peptide libraries limits the portion of the loop region in CI-2A that can become substituted. To utilize the complete loop region for peptide presentation it is contemplated to create a complementary peptide library by using the more 5'-located *Bam*HI cleavage site situated in pCMVbipep. In this case, the DNA fragment that contains the randomized region can be generated by either a non-PCR based or a PCR based method as outlined in Fig. 7. This allows the 5'-border of the randomized region to be defined based on theoretical considerations.

The two combined cloning strategies allow construction of CI-2A presented libraries that diverge in the manner whereby the peptides are presented. Such different libraries are likely to complement each other regarding interactions with possible target molecules. Screening different types of libraries will therefore increase the number of possible target molecules identified.

EXAMPLE 6

*Discussion of CI-2A as scaffold in the CellScreen™ technology*

As demonstrated in Example 2, CI-2A can be expressed in a functional form in mammalian cells. Establishment of a functional system for displaying randomized peptide sequences using CI-2A as a scaffold is thus relatively uncomplicated to envisage. In order to direct the CI-2A scaffold to different compartments of the cell, retroviral vectors harboring different leader sequences have been constructed. The data presented in Example 3 illustrates that a defined localization for CI-2A can be obtained. Especially the nucleus and the endoplasmic reticulum are compartments where several specific reactions occur, such as transcriptional regulation and receptor folding. Such processes are obvious targets for peptide antagonists. The intracellular tolerance to CI-2A in mammalian cells and the capability of CI-2A to translocate to the nucleus and the endoplasmic reticulum makes it reasonable to assume that CI-2A can be targeted to other compartments and intracellular organelles if desired.

CI-2A is an extremely stable protein that has the advantage of being small, having no disulfide bridges, no glycosylation sites and a loop of eight amino acids that protrudes from the core structure (Macphalen C.A. et al. (1983) J. Mol. Biol. 168, pp 445-447). It has been shown that insertion of 7, 9, 11 and 13 residues between the Met40 and Glu41 (corresponding to the Met59 and Glu60 in the native molecule) in CI-2A have a minimal effect on the stability and folding rates of the protein. Moreover, CI-2A has been found to fold through interactions of key residues in the C-terminal domain of the protein, irrespective of the amino acids situated in the loop region (Osmark P. et al. (1993) Biochem. 32, 11007-11014, Ladurner A.G. and Fersht A.R. (1997) J. Mol. Biol. 273, pp 330-337). The loop therefore seems to be suitable for the insertion of random residues which is the purpose of the present invention. As described in example 4, substitution of 4 amino acids in the loop region with a 19-mer randomly composed peptide did no significantly affect the capability of this modified CI-2A to be displayed on the phage surface. This result correlates with previous data showing that folding of the CI-2A core structure is independent of both the size and amino acid sequence of the loop region.

One important feature of the present invention is that the peptides are selected based on a biological activity exerted inside mammalian cells. This implicates that the stability of the applied scaffold most be independent of disulfide bridge formation. Since no disulfide bridges are present in the CI-2A structure the stability and the folding rate of CI-2A must be independent of the redox potential of the solvent. The extraction of active CI-2A from mammalian cells suggests that CI-2A is capable of adopting a native structure in the intracellular environment, which is the major demand to a CellScreen™ scaffold.

The N-terminal 19 amino acid residues do not have any known function for the folding of CI-2A (De Prat Gay G. et al. (1994), Proc. Natl. Acad. Sci. 91, pp 10943-10946 and references herein). As they might be able to perform unspecific interactions during screening and target isolation we decided to use the shorter 64 residue version of CI-2A. In addition, the limited size increases the accessibility of the peptide/CI-2A protein to binding pockets which might increase the number of possible targets and thereby the likelihood for isolation of peptide antagonists.

The ability of CI-2A to be exposed at the surface of phage particles was clearly demonstrated by the data presented in Example 4. The phage display technology allows selection of peptides that interacts with any immobilized component(s). This could be crude cell extracts, receptor containing membranes or partly purified material containing the activity against which a peptide antagonist is desired. At present, a higher diversity can be handled by phage display due to the difference in the physical size of a phage particle and a mammalian cell. By reducing the peptide library to a pool which only contains peptides that are capable of interacting with the potential target molecules the actual diversity that needs to be handled inside the cells, can be significantly reduced

To be able to isolate the CI-2A scaffold - and thereby the target molecule to which it binds - from selected cells, a peptide tag will be fused to the N-terminus of the truncated CI-2A. We are currently considering the following tags: Histag, Strep-tag or FLAG-tag. However, co-immunoprecipitation using an anti CI-2A antibody can also be used. Alternatively to the biochemical methods, genetic approaches such as yeast or mammalian two- or three-hybrid systems will also be applied to identify the targets that interacts with the selected peptides.

## Claims

1. A method for identifying a modulator in the form of a biologically active polypeptide fragment which is capable of detectably modulating, *in vivo*, a phenotypic trait in a cell, the method comprising the steps of

   (a) preparing a pool of expression vectors, each vector of said pool containing at least one member from a library of randomly modified nucleotide sequences derived from a parent nucleotide sequence encoding a parent peptide which *in vivo* directly modulates activity of a known protease, wherein the randomly modified nucleotide sequences comprise

   - an invariable part encoding a scaffold portion of the parent peptide, said scaffold portion serving to stabilize said polypeptide fragment and being stable towards proteolytic attack and/or being insensitive to a reducing environment, and
   - random nucleotides,

   (b) transforming a population of substantially identical cells with said vectors of said pool so as to obtain transformed cells,
   (c) culturing said transformed cells under conditions facilitating expression of said randomly modified nucleotide sequences,
   (d) examining said transformed cells and isolating transformed cell(s) wherein the preselected phenotypic trait is modulated thereby indicating that the expression product of said randomly modified nucleotide sequence is biologically active, and
   (e) identifying the modulator by determining said randomly modified nucleotide sequence of said vector present in cell(s) isolated in step (d) and/or determining the amino acid sequence of the expression product encoded by said randomly modified nucleotide sequence.

2. The method according to claim 1, wherein the substantially identical cells are prokaryotic cells.

3. The method according to claim 1, wherein the substantially identical cells are eukaryotic cells.

4. The method according to claim 3, wherein the eukaryotic cells are selected from the group consisting of fungal cells, protozoan cells, animal cells, and plant cells.

5. The method according to claim 4, wherein the animal cells are selected from the group consisting of mammalian cells, arthropod cells such as insect cells, avian cells, and piscine cells.

6. The method according to any of the preceding claims, wherein the transformed cells examined in step (d) predominantly carries one single copy of the vector.

7. The method according to claim 6, Wherein transformation step (b) is performed under such conditions that the cells transformed are predominantly or at most transformed with one single vector from said pool, or wherein, prior to carrying out step (d), cells being transformed with more than one vector from said pool are substantially excluded from the further steps.

8. The method according to any one of the preceding claims, wherein the random nucleotides are introduced in part (s) of the parent nucleotide sequence which encode(s) the active site(s) of the parent peptide, or the part(s) which encode(s) structure(s) interfering with the active site(s).

**9.** The method according to any one of the preceding claims, wherein the invariable part of the nucleotide sequence encodes truncated parts of the scaffold portion of the parent peptide sufficient to maintain stability.

**10.** The method according to any of the preceding claims, wherein the invariable part of the parent nucleotide sequence encodes a peptide which is free from disulfide bridges.

**11.** The method according to any one of claims 1-9, wherein the invariable part of the parent nucleotide sequence encodes a peptide having disulfide bridges.

**12.** The method according to any one of the preceding claims, wherein the random nucleotides are introduced in the form of an insertion or a substitution into the parent nucleotide sequence, optionally in combination with deletion (s) in the parent nucleotide sequence.

**13.** The method according to claim 12, wherein the number of random nucleotides which are introduced is in the range from 3 to about 100.

**14.** The method according to any one of the preceding claims, wherein the random nucleotides are nucleotide sequences and/or are single random nucleotides introduced at specific sites in the parent nucleotide sequence.

**15.** The method according to any one of the preceding claims, wherein the random nucleotides are selected from the group consisting of

- synthetic, completely random deoxyribonucleotides;
- synthetic random DNA sequences, wherein limitation on randomization of some nucleotides is introduced so as to limit the number of available sequences and/or to avoid undesired stop codons and/or to facilitate introduction of post-translational modifications of expressed peptide (s) ;
- synthetic random DNA sequences as in (1) or (2) coupled to a sequence encoding a purification tag; and
- CDR encoding nucleotide sequences isolated from a library of immune-competent cells raised against an antigen.

**16.** The method according to claim 15, wherein the CDR encoding nucleotide sequences encode CDR-3 peptide sequences.

**17.** The method according to any one of claims 14-16, wherein the random nucleotides are prepared by random codon synthesis where defined DNA codons are synthesized in a random order.

**18.** The method according to claim 17, wherein the relative amount of synthesized codons ensure that all encoded amino acids will be present with substantially the same frequency in the total of encoded polypeptide fragments.

**19.** The method according to any one of the preceding claims, wherein the random nucleotides are introduced into the expression vector by the principle of site directed PCR-mediated mutagenesis.

**20.** The method according to any one of the preceding claims, wherein the parent peptide is an inhibitor of activity of the known protease.

**21.** A method according to claim 20, wherein the inhibitor is selected from the group consisting of
a BPTI/Kunitz family protease inhibitor, a serpin family protease inhibitor, a Kazal family protease inhibitor, a soybean trypsin inhibitor (Kunitz) family protease inhibitor, a potato inhibitor I family member, a Bowman-Birk family protease inhibitor, a squash inhibitor family member, a wap-type 'Four-disulfide Core' proteinase inhibitor, a hirudin family protease inhibitor, a factor Xa inhibitor, an Ascaris trypsin inhibitor family member, a cystatin family protease inhibitor, a calpain family cysteine protease inhibitor, a tissue inhibitor of metalloproteinases family member, a carboxypeptidase A inhibitor, a metallocarboxypeptidase inhibitor, and an angiotensin-converting enzyme inhibitor.

**22.** The method according to claim 21, wherein the parent peptide is a potato inhibitor family I member.

**23.** The method according to claim 22, wherein the parent peptide is chymotrypsin inhibitor 2A (CI-2A).

**24.** The method according to any of the preceding claims wherein the substantially identical cells are mammalian cells and the vector is selected from the group consisting of a retroviral vector, a vaccinia virus vector, an adenoviral vector, an adeno associated virus (AAV) vector, a herpes simplex virus (HSV) vector, an alpha virus vector, and a semliki forest virus vector.

**25.** The method according to claim 24, wherein the vector is retroviral.

**26.** The method according to claim 25, wherein the retroviral vector is derived from retrovirus selected from the group consisting of Avian Leukosis-Sarcoma Virus (ALSV), Mammalian type C, Mammalian type B, and Lentivirus, and optionally modified with heterologous cis-acting elements.

**27.** The method according to claim 25 or 26, wherein the retroviral vector has non-identical ends.

**28.** The method according to claim 27, wherein the non-identical ends contain non-identical promoters.

**29.** The method according to any one of claims 25-28, wherein the retroviral vector contains a heterologous promoter replacing the viral promoter in the 5'-LTR, such as a CMV promoter, an RSV promoter, an SV-40 promoter, a TK promoter, an MT promoter, or an inducible system such as Tet or Ecdysone.

**30.** The method according to any one of claims 25-29, wherein step (a) is carried out by

1) transfecting suitable packaging-cells with vectors which comprise the randomly modified nucleotide sequences and which are integratable in virions produced by said packaging cells,
2) culturing said transfected packaging cells in a culture medium under conditions which facilitate production by the packaging cells of virions containing the randomly modified nucleotide sequences,
3) recovering and optionally concentrating said virions, and
4) transducing said substantially identical cells with the virions.

**31.** The method according to claim 30, wherein the packaging cells are selected from the group consisting of PE501, Bosc23, Ψ2, GP+E86, PhoenixEco, PA317, GP+AM12, DA(ampho), Bing, FLYA13, ProPak, CRIP, ΨAM, Phoenix-Ampho, PG13, H9 (293GPG), and EcoPack.

**32.** The method according to any one of claims 25-31, wherein the virions are pseudotyped retrovirus produced by an ecotropic packaging cell line so as to confer broad tropism to the virions produced thereby, or wherein an ecotropic receptor has been introduced into the substantially identical cells so as to allow transduction with ecotropic virions.

**33.** The method according to claim 32, wherein the ecotropic receptor has been introduced in the substantially identical cells by means of transduction.

**34.** The method according to any of the preceding claims wherein the randomly modified nucleotide sequences are coupled to a nucleotide sequence encoding at least one fusion partner.

**35.** The method according to claim 34, wherein the fusion partner serves to facilitate expression and/or purification/isolation and/or further stabilization of the expression product.

**36.** The method according to claim 35, wherein the fusion partner includes a purification tag such as $His_6$ tag, myc tag, BSP biotinylation target sequence, of BirA, flu tag, lacZ, and GST.

**37.** The method according to claim 34 or 35, wherein the fusion partner is a sorting signal or a targeting sequence.

**38.** The method according to claim 37, wherein the sorting signal is a signal patch or a signal peptide.

**39.** The method according to claim 37 or 38, wherein the sorting signal effects export of the expressed peptide out of the cell or into the cell membrane, or, when the substantially identical cells are eukaryotic, into endoplasmic reticulum, into Golgi apparatus, into lysosomes, into secretory vesicles, into mitochondria, into peroxisomes, or into the nucleus.

**40.** The method according to any one of the preceding claims, which further comprises the step of resolving the 3-dimensional structure of the identified modulator.

**41.** A method for the preparation of a replicable expression vector, the method comprising the steps of identifying a modulator by the method according to any one of the preceding claims, and subsequently

i) isolating or synthesizing a nucleic acid sequence which encodes the modulator, and
ii) engineering a replicable expression vector comprising an operon which comprises, in the 5'-3' direction and in operable linkage, 1) a promoter for driving expression of the nucleic acid sequence, 2) optionally a nucleotide sequence encoding a leader peptide, 3) the nucleic acid sequence, and 4) optionally a termination signal.

**42.** A method for the preparation of a transformed cell carrying a nucleic acid sequence encoding a modulator as defined in any one of claims 1-40, the method comprising transforming a suitable host cell with an expression vector prepared according to claim 41.

**43.** A method for providing a modulator as defined in any one of claims 1-40, the method comprising

I) growing a transformed cell prepared according to the method of claim 42 in a culture medium under conditions which facilitates expression by the cell of the randomly modified nucleotide sequence, and
II) subsequently harvesting the expression product from the cell and/or the culture medium, or
Ia) identifying the modulator according to the method of any one of claims 1-40, and
Ib) subsequently synthesizing the modulator by means of chemical synthesis on the basis of the sequence determined in step (e).

**44.** A method for isolating and/or identifying a target biomolecule, the method comprising providing a modulator according to the method of claim 43 and subsequently using the modulator as an affinity ligand in an affinity purification step so as to isolate the target biomolecule from a suitable sample.

**45.** The method according to claim 44, wherein the affinity purification step is an affinity chromatographic step, an affinity mass spectrometry step, or a co-immunoprecipitation step.

**46.** A method for isolating and/or identifying a target biomolecule, the method comprising providing a peptide modulator according to the method of claim 42 and subsequently using the modulator as a probe against a cDNA library derived from the substantially identical cells or using the modulator as bait in a two- or three-hybrid system.

**47.** The method according to any of claims44-46, wherein the target biomolecule is a peptide or a nucleic acid.

**48.** The method according to claim 47 further comprising the step of determining the amino acid sequence of the peptide or determining the nucleotide sequence of the nucleic acid.

**49.** The method according to any of claims 44-48, further comprising the step of resolving the 3-dimensional structure of the target biomolecule.

**50.** A method for selecting a chemical compound as a putative drug candidate in drug development, the method comprising the steps of

- assaying a library of chemical compounds for interaction with a target biomolecule which has been de novo isolated according to the method of any one of claims 44-49, and
- selecting compounds which interact significantly with the target biomolecule.

**51.** The method according to claim 50, wherein the library of chemical compounds has been provided by chemical synthesis upon initial identification of the members of the library by structure-based or non-structure based computer drug-modelling.

**52.** A method for the preparation of a medicinal product, the method comprising the steps of

A) selecting a chemical compound by the method according to claim 50 or 51,
B) performing pre-clinical tests with the chemical compound in order to assess the suitability thereof as a medicinal product,

C) entering, if the chemical compound is deemed suitable in step (B), clinical trials using the chemical compound in order to obtain market authorization for a medicinal product including the lead compound as a pharmaceutically active substance, and
D) upon grant of a market authorization, admixing the chemical compound with a pharmaceutically acceptable carrier, excipient or diluent and marketing the thus obtained medicinal product.

**53.** A method for developing a medicinal product, the method comprising that a modulator identified according to the method of any one of claims 1-40 serves as a lead compound in the drag development phase or wherein a target biomolecule isolated/identified according to any one of claims 44-49 serves as an interaction probe for the identification of putative drug candidates in the drug discovery phase.

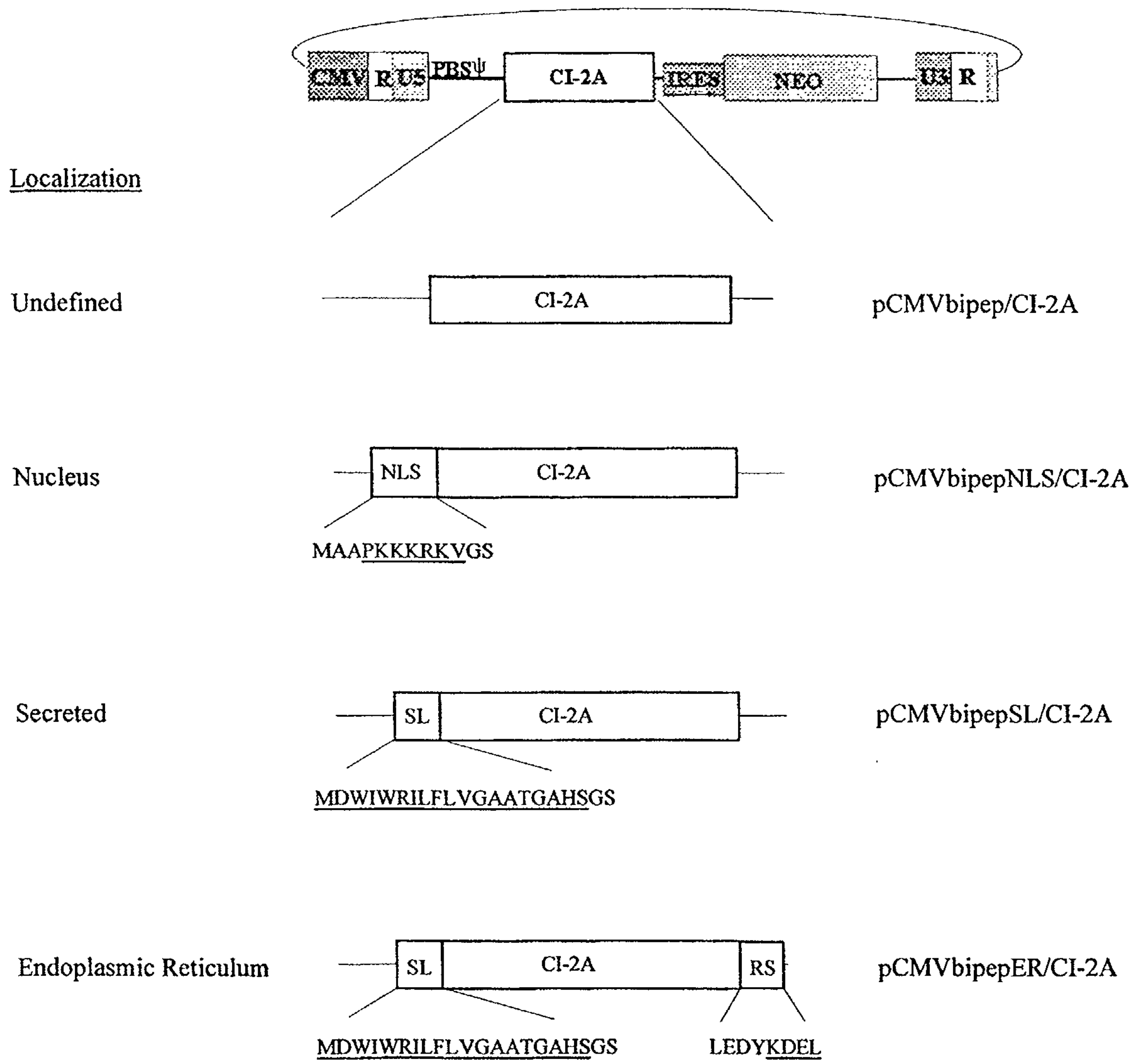
CMV
R
U5
PBSψ
CI-2A
IRES
NEO
U3
R
Localization
Undefined
CI-2A
pCMVbipep/CI-2A
Nucleus
NLS
CI-2A
pCMVbipepNLS/CI-2A
MAAPKKKRKVGS
Secreted
SL
CI-2A
pCMVbipepSL/CI-2A
MDWIWRILFLVGAATGAHSGS
Endoplasmic Reticulum
SL
CI-2A
RS
pCMVbipepER/CI-2A
MDWIWRILFLVGAATGAHSGS
LEDYKDEL

Fig. 1

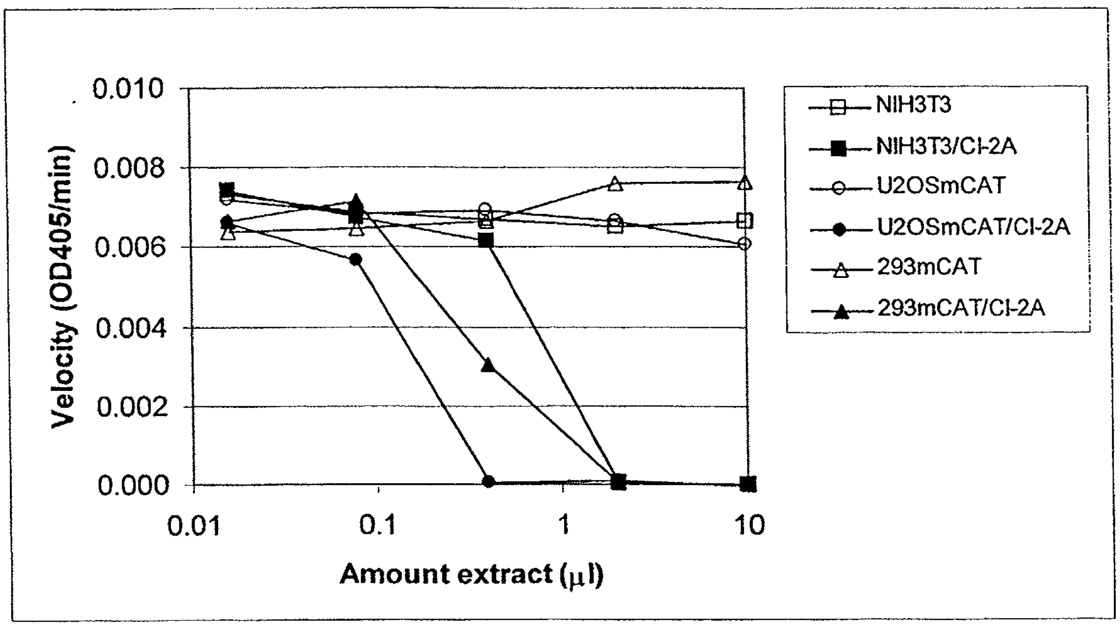
0.010
0.008
0.006
0.004
0.002
0.000
Velocity (OD405/min)
0.01
0.1
1
10
Amount extract (μl)
NIH3T3
NIH3T3/CI-2A
U2OSmCAT
U2OSmCAT/CI-2A
293mCAT
293mCAT/CI-2A

# Fig. 2

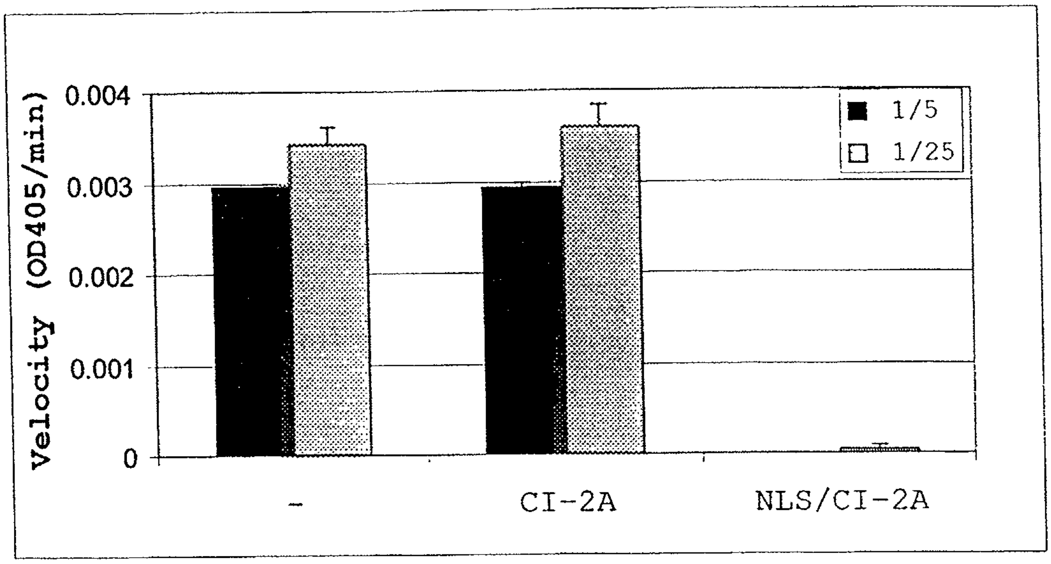
Velocity (OD405/min)
0.004
0.003
0.002
0.001
0
1/5
1/25
-
CI-2A
NLS/CI-2A

Fig. 3A

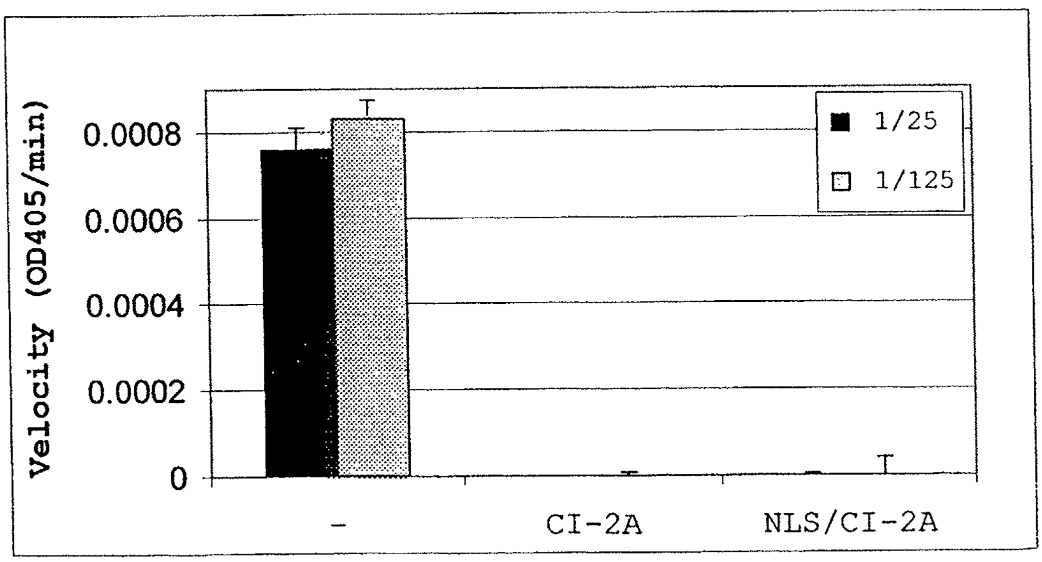
Velocity (OD405/min)
0.0008
0.0006
0.0004
0.0002
0
1/25
1/125
-
CI-2A
NLS/CI-2A

Fig. 3B

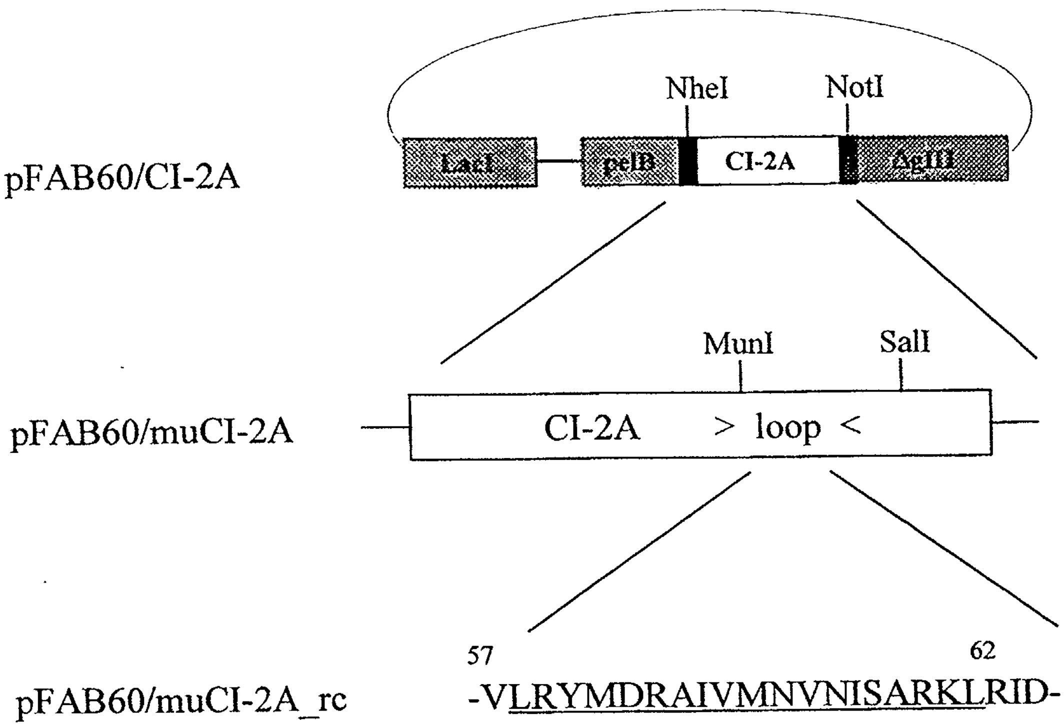
pFAB60/CI-2A
NheI
NotI
LacI
pelB
CI-2A
ΔgIII
pFAB60/muCI-2A
MunI
SalI
CI-2A > loop <
pFAB60/muCI-2A_rc
57
62
-VLRYMDRAIVMNVNISARKLRID-

Fig. 4

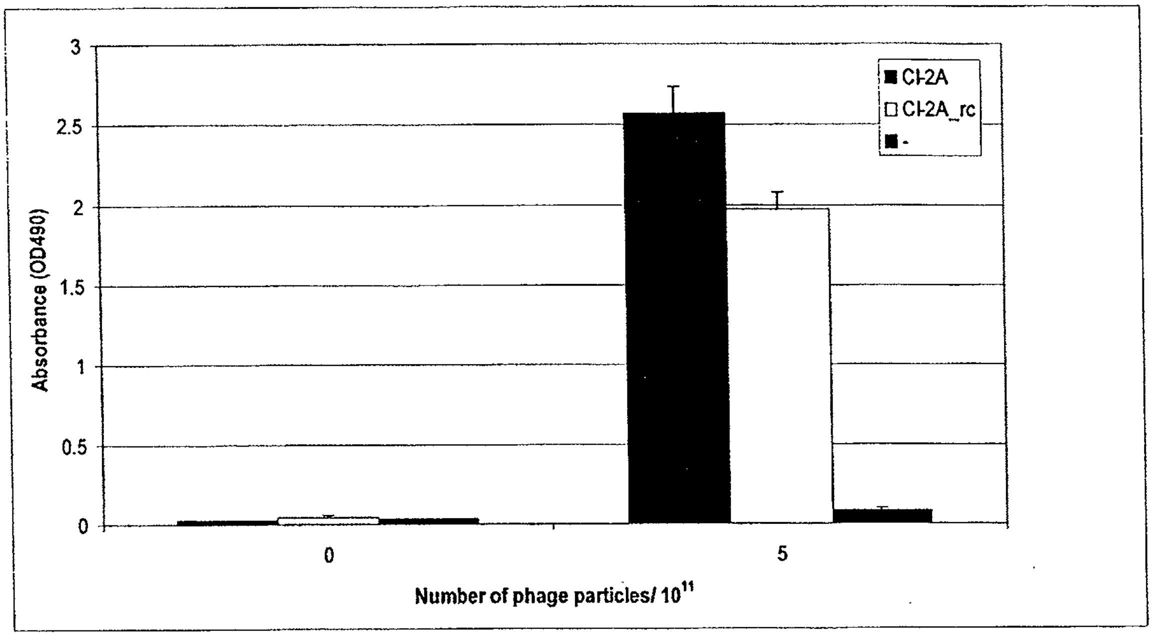
3
2.5
2
1.5
1
0.5
0
Absorbance (OD490)
CI-2A
CI-2A_rc
-
0
5
Number of phage particles/ 10^11

Fig. 5

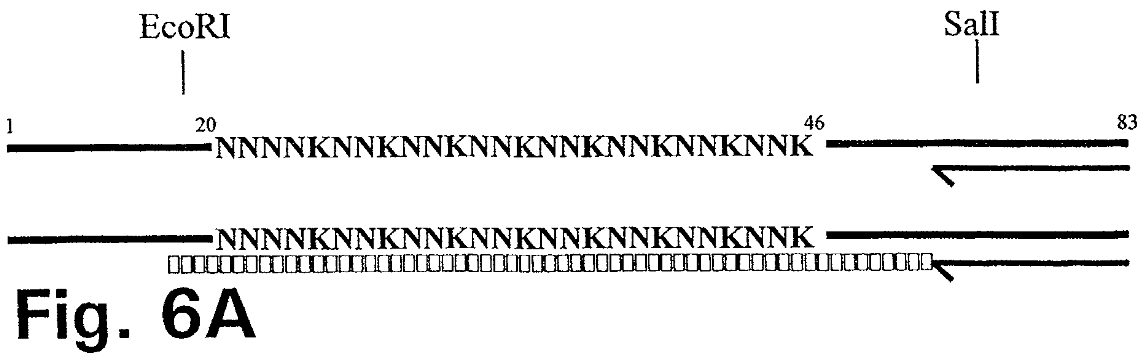


Fig. 6A

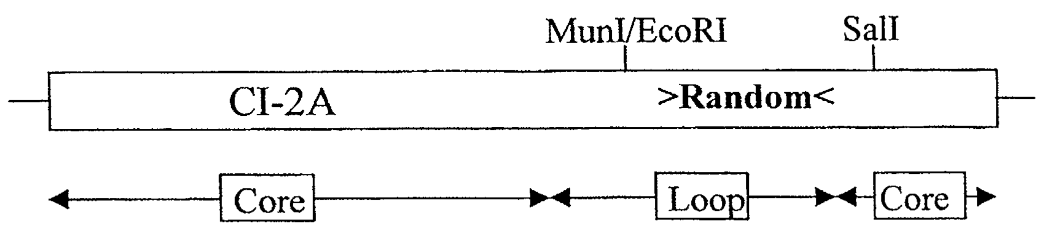


Fig. 6B

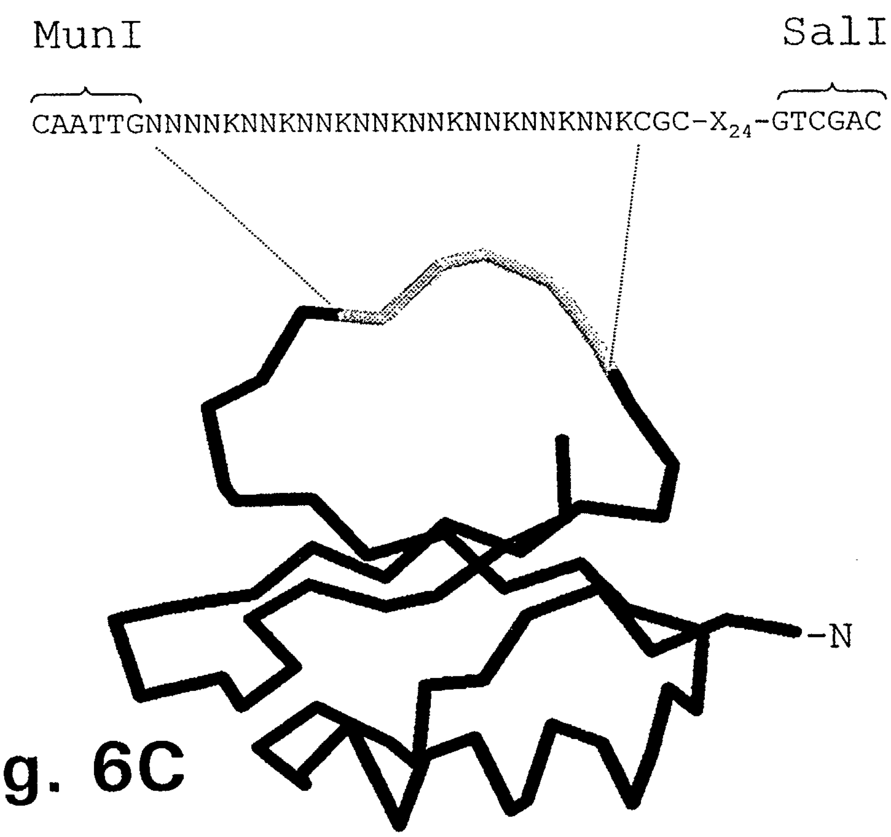


Fig. 6C

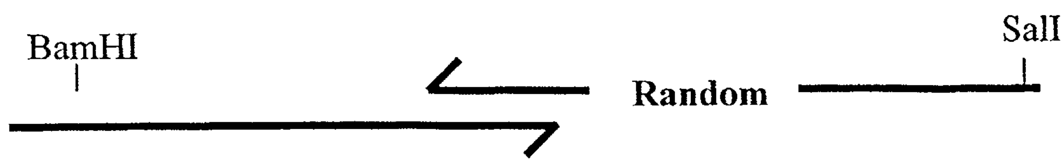


Fig. 7A

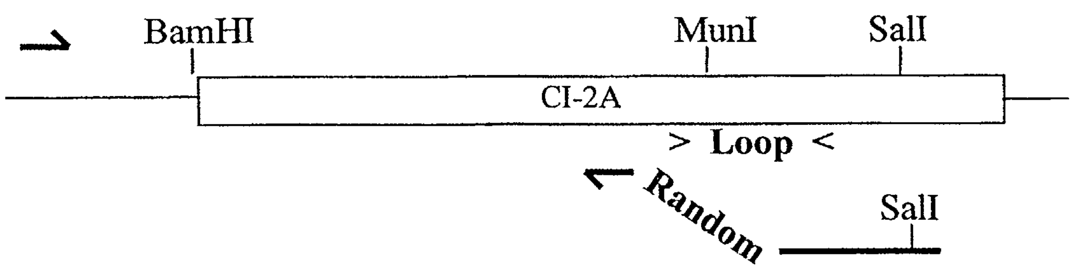


Fig. 7B

European Patent Office

## PARTIAL EUROPEAN SEARCH REPORT

which under Rule 45 of the European Patent Convention shall be considered, for the purposes of subsequent proceedings, as the European search report

Application Number EP 02 07 6171

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WO 96 38553 A (MOURITSEN & ELSNER A S ;JENSEN MARTIN ROLAND (DK); PEDERSEN FINN S) 5 December 1996 (1996-12-05)<br>* the whole document *<br>--- | 1-40 | C12Q1/68<br>C12N15/10 |
| X,D | WO 97 27212 A (RIGEL PHARMACEUTICALS INC) 31 July 1997 (1997-07-31)<br>see esp. claims<br>--- | 1-40 | |
| X,P | WO 98 32880 A (BARBER JACK ;IMMUSOL INC (US); TRITZ RICHARD (US); WELCH PETER (US)) 30 July 1998 (1998-07-30)<br>* the whole document *<br>--- | 1-40 | |
| X | BLIND M. ET AL.,: "Cytoplasmic RNA modulators of an inside-out signal transduction cascade"<br>PROC. NATL. ACAD. SCI. USA,<br>vol. 96, - March 1999 (1999-03) pages 3606-3610, XP002113353<br>* the whole document *<br>---<br>-/-- | 1-40 | |

TECHNICAL FIELDS SEARCHED (Int.Cl.7)

C12Q
C12N

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 9 October 2002 | Mueller, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 02 07 6171

Claim(s) searched completely:
1-40

Claim(s) not searched:
40-53

Reason for the limitation of the search:

Present claims 40-53 relate to compounds and products defined by reference to a desirable characteristic or property, namely modulating a known protease.
The claims cover all compounds and products having this characteristic or property, whereas the application provides no support within the meaning of Article 84 EPC and/or disclosure within the meaning of Article 83 EPC for the structural features of such compounds or products.
In the present case, the claims so lack support, and the application so lacks disclosure, that a meaningful search over the whole of the claimed scope is impossible. Independent of the above reasoning, the claims also lack clarity (Article 84 EPC). An attempt is made to define the products/compounds by reference to a result to be achieved. Again, this lack of clarity in the present case is such as to render a meaningful search over the the claimed scope impossible.
Consequently, no search has been carried out.

European Patent Office

# PARTIAL EUROPEAN SEARCH REPORT

Application Number
EP 02 07 6171

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | KLUG S.J. ET AL.,: "In vitro and in vivo characterization of novel mRNA motifs that bind special elongation factor Selb" PROC. NATL. ACAD. SCI. USA, vol. 94, - June 1997 (1997-06) pages 6676-6681, XP002113354<br>--- | | |
| A | FERBER M.J. ET AL.,: "Combinatorial selection of a small RNA that induces amplification of IncFII plasmids in escherichia coli" J. MOL. BIOL., vol. 279, - 1998 pages 565-576, XP002113817<br>* the whole document *<br>--- | | TECHNICAL FIELDS SEARCHED (Int.Cl.7) |
| A | FAMULOK AND JENNE: "OLIGONUCLEOTIDE LIBRARIES-VARIATIO DELECTAT" CURRENT OPINION IN CHEMICAL BIOLOGY, CURRENT BIOLOGY LTD, LONDON, GB, vol. 2, no. 2, 1998, pages 320-327, XP002113356<br>ISSN: 1367-5931<br>* the whole document *<br>----- | | |

EPO FORM 1503 03.82 (P04C10)

## ANNEX TO THE EUROPEAN SEARCH REPORT ON EUROPEAN PATENT APPLICATION NO. EP 02 07 6171

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2002

| Patent document cited in search report | | Publication date | Patent family member(s) | | | Publication date |
|---|---|---|---|---|---|---|
| WO 9638553 | A | 05-12-1996 | AT | 196312 | T | 15-09-2000 |
| | | | AU | 699568 | B2 | 10-12-1998 |
| | | | AU | 5893396 | A | 18-12-1996 |
| | | | CA | 2222879 | A1 | 05-12-1996 |
| | | | DE | 69610310 | D1 | 19-10-2000 |
| | | | DE | 69610310 | T2 | 15-03-2001 |
| | | | WO | 9638553 | A1 | 05-12-1996 |
| | | | DK | 832207 | T3 | 06-11-2000 |
| | | | EP | 1041143 | A2 | 04-10-2000 |
| | | | EP | 0832207 | A1 | 01-04-1998 |
| | | | ES | 2151167 | T3 | 16-12-2000 |
| | | | JP | 11505724 | T | 25-05-1999 |
| | | | PT | 832207 | T | 30-03-2001 |
| | | | SI | 832207 | T1 | 28-02-2001 |
| | | | US | 2001053523 | A1 | 20-12-2001 |
| WO 9727212 | A | 31-07-1997 | US | 6365344 | B1 | 02-04-2002 |
| | | | AU | 1707497 | A | 20-08-1997 |
| | | | AU | 725716 | B2 | 19-10-2000 |
| | | | AU | 1707897 | A | 20-08-1997 |
| | | | CA | 2244222 | A1 | 31-07-1997 |
| | | | EP | 0877752 | A1 | 18-11-1998 |
| | | | JP | 2000504220 | T | 11-04-2000 |
| | | | JP | 2001509661 | T | 24-07-2001 |
| | | | WO | 9727212 | A1 | 31-07-1997 |
| | | | WO | 9727213 | A1 | 31-07-1997 |
| | | | US | 6455247 | B1 | 24-09-2002 |
| | | | US | 6153380 | A | 28-11-2000 |
| | | | US | 2002127564 | A1 | 12-09-2002 |
| WO 9832880 | A | 30-07-1998 | AU | 5927598 | A | 18-08-1998 |
| | | | EP | 0996741 | A1 | 03-05-2000 |
| | | | JP | 2002514914 | T | 21-05-2002 |
| | | | WO | 9832880 | A1 | 30-07-1998 |

EPO FORM P0459

For more details about this annex : see Official Journal of the European Patent Office, No. 12/82

SEQUENCE LISTING

<110> M&E Biotech A/S
Halkier, Torben
Jespersen, Lene
Jensen, Allan

<120> Novel Methods for the Identification of Ligand and Target Biomolecules

<130> 21129 PC 1

<140>
<141>

<160> 43

<170> PatentIn Ver. 2.1

<210> 1
<211> 451
<212> DNA
<213> Hordeum vulgare

<220>
<221> CDS
<222> (85)..(339)

<220>
<221> mat_peptide
<222> (88)..(336)

<220>
<221> misc_feature
<222> (249)
<223> G is G or T

<220>
<221> misc_feature
<222> (252)
<223> A is A or C

<220>
<221> misc_feature
<222> (279)
<223> C is C or T

<400> 1
cattaaactg atgacatgac agttcaagat ctcacagtca catcggcgat ctaatcagtc 60

```
tcacaggaag cgagcgtaac aagg atg agt tca gtg gag aag aag ccg gag    111
                           Met Ser Ser Val Glu Lys Lys Pro Glu
                            -1   1               5

gga gtg aac acc ggt gct ggt gac cgt cac aac ctg aag aca gag tgg   159
Gly Val Asn Thr Gly Ala Gly Asp Arg His Asn Leu Lys Thr Glu Trp
     10                  15                  20

cca gag ttg gtg ggg aaa tcg gtg gag gag gcc aag aag gtg att ctg   207
Pro Glu Leu Val Gly Lys Ser Val Glu Glu Ala Lys Lys Val Ile Leu
 25                  30                  35                  40

cag gac aag cca gag gcg caa atc ata gtt ctg ccg gtg ggg aca att   255
Gln Asp Lys Pro Glu Ala Gln Ile Ile Val Leu Pro Val Gly Thr Ile
                 45                  50                  55

gtg acc atg gaa tat cgg atc gac cgc gtc cgc ctc ttt gtc gat aaa   303
Val Thr Met Glu Tyr Arg Ile Asp Arg Val Arg Leu Phe Val Asp Lys
             60                  65                  70

ctc gac aac att gcc cag gtc ccc agg gtc ggc tag caagcttgag        349
Leu Asp Asn Ile Ala Gln Val Pro Arg Val Gly
         75                  80

agctagcctg ctgctggcgt gtatgtattg cagcttcacc atctcttctt ggctatagca 409

agattgagat ttataaatca tatacaataa gagttgctgc gg                    451


<210> 2
<211> 84
<212> PRT
<213> Hordeum vulgare

<400> 2
Met Ser Ser Val Glu Lys Lys Pro Glu Gly Val Asn Thr Gly Ala Gly
  1               5                  10                  15

Asp Arg His Asn Leu Lys Thr Glu Trp Pro Glu Leu Val Gly Lys Ser
             20                  25                  30

Val Glu Glu Ala Lys Lys Val Ile Leu Gln Asp Lys Pro Glu Ala Gln
         35                  40                  45

Ile Ile Val Leu Pro Val Gly Thr Ile Val Thr Met Glu Tyr Arg Ile
     50                  55                  60

Asp Arg Val Arg Leu Phe Val Asp Lys Leu Asp Asn Ile Ala Gln Val
 65                  70                  75                  80

Pro Arg Val Gly
```

<210> 3
<211> 27
<212> DNA
<213> Artificial Sequence

<220>
<223> Description of Artificial Sequence: Synthetic DNA primer

<400> 3
cgggatccat gaagacagtg gccagag 27

<210> 4
<211> 28
<212> DNA
<213> Artificial Sequence

<220>
<223> Description of Artificial Sequence: Synthetic DNA primer

<400> 4
cgctcgagtc agccgaccct ggggacct 28

<210> 5
<211> 21
<212> DNA
<213> Artificial Sequence

<220>
<223> Description of Artificial Sequence: Synthetic DNA primer

<400> 5
ctgtatctgg cggctccgtg g 21

<210> 6
<211> 19
<212> DNA
<213> Artificial Sequence

<220>
<223> Description of Artificial Sequence: Synthetic DNA primer

<400> 6
acagctggcc ctcgcagac 19

<210> 7

<211> 20
<212> DNA
<213> Artificial Sequence

<220>
<223> Description of Artificial Sequence: Synthetic DNA primer

<400> 7
cccactgctt actggcttat 20

<210> 8
<211> 19
<212> DNA
<213> Artificial Sequence

<220>
<223> Description of Artificial Sequence: Synthetic DNA primer

<400> 8
tggggctgca cgtcatttg 19

<210> 9
<211> 20
<212> DNA
<213> Artificial Sequence

<220>
<223> Description of Artificial Sequence: Synthetic DNA primer

<400> 9
tgtgctacgg cgagtttggt 20

<210> 10
<211> 20
<212> DNA
<213> Artificial Sequence

<220>
<223> Description of Artificial Sequence: Synthetic DNA primer

<400> 10
ggttcgtgaa aggctccatt 20

<210> 11
<211> 22

<212> DNA
<213> Artificial Sequence

<220>
<223> Description of Artificial Sequence: Synthetic DNA primer

<400> 11
gaaatgttca caattagccc tg 22

<210> 12
<211> 60
<212> DNA
<213> Artificial Sequence

<220>
<223> Description of Artificial Sequence: Synthetic DNA primer

<400> 12
gaagatctat ggcggccgca ccaaaaaaga agagaaaggt aggatccatg aagacagagt 60

<210> 13
<211> 28
<212> DNA
<213> Artificial Sequence

<220>
<223> Description of Artificial Sequence: Synthetic DNA primer

<400> 13
cgctcgagtc agccgaccct ggggacct 28

<210> 14
<211> 30
<212> DNA
<213> Artificial Sequence

<220>
<223> Description of Artificial Sequence: Synthetic DNA primer

<400> 14
gaagatctat ggactggatc tggcgcatcc 30

<210> 15
<211> 28
<212> DNA

<213> Artificial Sequence

<220>
<223> Description of Artificial Sequence: Synthetic DNA primer

<400> 15
gaggatccag aatgagcgcc ggtagcag 28

<210> 16
<211> 21
<212> DNA
<213> Artificial Sequence

<220>
<223> Description of Artificial Sequence: Synthetic DNA primer

<400> 16
ctgtatctgg cggctccgtg g 21

<210> 17
<211> 55
<212> DNA
<213> Artificial Sequence

<220>
<223> Description of Artificial Sequence: Synthetic DNA primer

<400> 17
ctaatctaga ctacagctcg tccttgtagt cctcgaggcc gaccctgggg acctg 55

<210> 18
<211> 29
<212> DNA
<213> Artificial Sequence

<220>
<223> Description of Artificial Sequence: Synthetic DNA primer

<400> 18
cgggatccat gaagacagag tggccagag 29

<210> 19
<211> 20
<212> DNA
<213> Artificial Sequence

<220>
<223> Description of Artificial Sequence: Synthetic DNA primer

<400> 19
ccggccttat tccaagcggc 20

<210> 20
<211> 28
<212> DNA
<213> Artificial Sequence

<220>
<223> Description of Artificial Sequence: Synthetic DNA primer

<400> 20
ctgccggtgg gtacaattgt gaccatgg 28

<210> 21
<211> 21
<212> DNA
<213> Artificial Sequence

<220>
<223> Description of Artificial Sequence: Synthetic DNA primer

<400> 21
ctgtatctgg cggctccgtg g 21

<210> 22
<211> 21
<212> DNA
<213> Artificial Sequence

<220>
<223> Description of Artificial Sequence: Synthetic DNA primer

<400> 22
ctgtatctgg cggctccgtg g 21

<210> 23
<211> 44
<212> DNA
<213> Artificial Sequence

<220>

<223> Description of Artificial Sequence: Synthetic DNA primer

<400> 23
cgagtttgtc gacaaagagg cggacgcgat cgatgcgata ttcc 44

<210> 24
<211> 20
<212> DNA
<213> Artificial Sequence

<220>
<223> Description of Artificial Sequence: Synthetic DNA primer

<400> 24
ccggccttat tccaagcggc 20

<210> 25
<211> 20
<212> DNA
<213> Artificial Sequence

<220>
<223> Description of Artificial Sequence: Synthetic DNA primer

<400> 25
ccggccttat tccaagcggc 20

<210> 26
<211> 21
<212> DNA
<213> Artificial Sequence

<220>
<223> Description of Artificial Sequence: Synthetic DNA primer

<400> 26
ctgtatctgg cggctccgtg g 21

<210> 27
<211> 54
<212> DNA
<213> Artificial Sequence

<220>
<223> Description of Artificial Sequence: Synthetic DNA

primer

<400> 27
atttgctagc tgcacaacca gcaatggcac tgaagacaga gtggccagag ttgg 54

<210> 28
<211> 37
<212> DNA
<213> Artificial Sequence

<220>
<223> Description of Artificial Sequence: Synthetic DNA primer

<400> 28
ataagaatgc ggccgcgccg accctgggga cctgggc 37

<210> 29
<211> 17
<212> DNA
<213> Artificial Sequence

<220>
<223> Description of Artificial Sequence: Synthetic DNA primer

<400> 29
cacacaggaa actatga 17

<210> 30
<211> 115
<212> DNA
<213> Artificial Sequence

<220>
<223> Description of Artificial Sequence: Synthetic DNA primer

<400> 30
ctgccggtgg gtacaattgt gctgcgctac atggaccgcg caatagtgat gaacgtgaac 60
attagcgcac gcaaactacg gattgatcgc gtccgcctct ttgtcgacaa actcg 115

<210> 31
<211> 25
<212> DNA
<213> Artificial Sequence

<220>
<223> Description of Artificial Sequence: Synthetic DNA

primer

<400> 31
cgagtttgtc gacaaagagg cggac 25

<210> 32
<211> 20
<212> DNA
<213> Artificial Sequence

<220>
<223> Description of Artificial Sequence: Synthetic DNA
primer

<400> 32
tctgccggtg ggtacaattg 20

<210> 33
<211> 83
<212> DNA
<213> Artificial Sequence

<220>
<223> Description of Artificial Sequence:Synthetic
degenerate oligonucleotide

<400> 33
tctgccggtg ggtagaattc nnnnknnknn knnknnknnk nnknnkcgga ttgatcgcgt 60
ccgcctcttt gtcgacaaac tcg 83

<210> 34
<211> 25
<212> DNA
<213> Artificial Sequence

<220>
<223> Description of Artificial Sequence: Synthetic DNA
primer

<400> 34
cgagtttgtc gacaaagagg cggac 25

<210> 35
<211> 12
<212> PRT
<213> Artificial Sequence

<220>
<223> Description of Artificial Sequence: Fragment

constituting nuclear localization signal

<400> 35
Met Ala Ala Pro Lys Lys Lys Arg Lys Val Gly Ser
1 5 10

<210> 36
<211> 21
<212> PRT
<213> Artificial Sequence

<220>
<223> Description of Artificial Sequence: Fragment constituting secretion signal

<400> 36
Met Asp Trp Ile Trp Arg Ile Leu Phe Leu Val Gly Ala Ala Thr Gly
1 5 10 15

Ala His Ser Gly Ser
20

<210> 37
<211> 8
<212> PRT
<213> Artificial Sequence

<220>
<223> Description of Artificial Sequence: Fragment constituting retention sequence

<400> 37
Leu Glu Asp Tyr Lys Asp Glu Leu
1 5

<210> 38
<211> 23
<212> PRT
<213> Artificial Sequence

<220>
<223> Description of Artificial Sequence: Fragment constituting random insert

<400> 38
Val Leu Arg Tyr Met Asp Arg Ala Ile Val Met Asn Val Asn Ile Ser
1 5 10 15

Ala Arg Lys Leu Arg Ile Asp
20

<210> 39
<211> 5788
<212> DNA
<213> Artificial Sequence

<220>
<223> Description of Artificial Sequence: Hybrid circular plasmid

<400> 39
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt ctccggaatt ggctagccta 420
gagtccgtta cataacttac ggtaaatggc ccgcctggct gaccgcccaa cgacccccgc 480
ccattgacgt caataatgac gtatgttccc atagtaacgc caatagggac tttccattga 540
cgtcaatggg tggagtattt acggtaaact gcccacttgg cagtacatca agtgtatcat 600
atgccaagta cgccccctat tgacgtcaat gacggtaaat ggcccgcctg gcattatgcc 660
cagtacatga ccttatggga ctttcctact tggcagtaca tctacgtatt agtcatcgct 720
attaccatgg tgatgcggtt ttggcagtac atcaatgggc gtggatagcg gtttgactca 780
cggggatttc caagtctcca ccccattgac gtcaatggga gtttgttttg gcaccaaaat 840
caacgggact ttccaaaatg tcgtaacaac tccgccccat tgacgcaaat gggcggtagg 900
cgtgtacggt gggaggtcta taaaaagggt aagaacccca cactcggcgc gccagtcctc 960
cgatagactg agtcgcccgg gtacccgtgt atccaataaa gccttttgct gttgcatccg 1020
aatcgtggtc tcgctgatcc ttgggagggt ctcctcagag tgattgactg cccagcctgg 1080
gggtctttca tttgggggct cgtccgggat ttggagaccc ccgcccaggg accaccgacc 1140
caccgtcggg aggtaagctg gccagcgatc gttttgtctc cgtctctgtc tttgtgcgtg 1200
tgtgtgtgtg tgccggcatc tactttttgc gcctgcgtct gattctgtac tagttagcta 1260
actagatctg tatctggcgg ctccgtggaa gaactgacga gttcgtattc ccgaccgcag 1320
ccctgggaga cgtctcagag gcatcggggg ggggatccag agctcgagct ttgaaaaaca 1380
cgcggccgcc atgtagtcta gaacgcgttg atcagttaac gaattcgaag ggtcccaggc 1440
ctcggagatc tgggcccatg cggccgcccc ctaacgttac tggccgaagc cgcttggaat 1500
aaggccggtg tgcgtttgtc tatatgttat tttccaccat attgccgtct tttggcaatg 1560
tgagggcccg gaaacctggc cctgtcttct tgacgagcat tcctaggggt ctttcccctc 1620
tcgccaaagg aatgcaaggt ctgttgaatg tcgtgaagga agcagttcct ctggaagctt 1680
cttgaagaca aacaacgtct gtagcgaccc tttgcaggca gcggaacccc ccacctggcg 1740
acaggtgcct ctgcggccaa aagccacgtg tataagatac acctgcaaag gcggcacaac 1800
cccagtgcca cgttgtgagt tggatagttg tggaaagagt caaatggctc tcctcaagcg 1860
tattcaacaa ggggctgaag gatgcccaga aggtacccca ttgtatggga tctgatctgg 1920
ggcctcggtg cacatgcttt acatgtgttt agtcgaggtt aaaaaacgtc taggcccccc 1980
gaaccacggg gacgtggttt tcctttgaaa aacacgattg ccgcgtgtgg cctcgaacac 2040
cgagcgaccc tgcagccaat atgggatcgg ccattgaaca agatggattg cacgcaggtt 2100
ctccggccgc ttgggtggag aggctattcg gctatgactg ggcacaacag acaatcggct 2160
gctctgatgc cgccgtgttc cggctgtcag cgcaggggcg cccggttctt tttgtcaaga 2220
ccgacctgtc cggtgccctg aatgaactgc aggacgaggc agcgcggcta tcgtggctgg 2280

```
ccacgacggg cgttccttgc gcagctgtgc tcgacgttgt cactgaagcg ggaagggact 2340
ggctgctatt gggcgaagtg ccggggcagg atctcctgtc atctcacctt gctcctgccg 2400
agaaagtatc catcatggct gatgcaatgc ggcggctgca tacgcttgat ccggctacct 2460
gcccattcga ccaccaagcg aaacatcgca tcgagcgagc acgtactcgg atggaagccg 2520
gtcttgtcga tcaggatgat ctggacgaag agcatcaggg gctcgcgcca gccgaactgt 2580
tcgccaggct caaggcgcgc atgcccgacg gcgaggatct cgtcgtgacc catggcgatg 2640
cctgcttgcc gaatatcatg gtggaaaatg gccgcttttc tggattcatc gactgtggcc 2700
ggctgggtgt ggcggaccgc tatcaggaca tagcgttggc tacccgtgat attgctgaag 2760
agcttggcgg cgaatgggct gaccgcttcc tcgtgcttta cggtatcgcc gctcccgatt 2820
cgcagcgcat cgccttctat cgccttcttg acgagttctt ctgacttaag acaatagaag 2880
attgtaaatc acgtgaataa aagattttat tcagtttaca gaaagagggg ggaatgaaag 2940
accccttcat aaggcttagc cagctaactg cagtaacgcc attttgcaag gcatgggaaa 3000
ataccagagc tgatgttctc agaaaaacaa gaacaaggaa gtacagagag gctggaaagt 3060
accgggacta gggccaaaca ggatatctgt ggtcaagcac tagggccccg gcccagggcc 3120
aagaacagat ggtccccaga aacagagagg ctggaaagta ccgggactag ggccaaacag 3180
gatatctgtg gtcaagcact agggccccgg cccagggcca agaacagatg gtccccagaa 3240
atagctaaaa caacaacagt ttcaagagac ccagaaactg tctcaaggtt ccccagatga 3300
ccggggatca accccaagcc tcatttaaac taaccaatca gctcgcttct cgcttctgta 3360
cccgcgctta ttgctgccca gctctataaa aagggtaaga accccacact cggcgcgcca 3420
gtcctccgat agactgagtc gcccgggtac ccgtgtatcc aataaagcct tttgctgttg 3480
catccgaatc gtggtctcgc tgatccttgg gagggtctcc tcctctgtcg ctcgacctgc 3540
aggcatgcaa gcttggcgta atcatggtca tagctgtttc ctgtgtgaaa ttgttatccg 3600
ctcacaattc cacacaacat acgagccgga agcataaagt gtaaagcctg gggtgcctaa 3660
tgagtgagct aactcacatt aattgcgttg cgctcactgc ccgctttcca gtcgggaaac 3720
ctgtcgtgcc agctgcatta atgaatcggc caacgcgcgg ggagaggcgg tttgcgtatt 3780
gggcgctctt ccgcttcctc gctcactgac tcgctgcgct cggtcgttcg gctgcggcga 3840
gcggtatcag ctcactcaaa ggcggtaata cggttatcca cagaatcagg ggataacgca 3900
ggaaagaaca tgtgagcaaa aggccagcaa aaggccagga accgtaaaaa ggccgcgttg 3960
ctggcgtttt tccataggct ccgcccccct gacgagcatc acaaaaatcg acgctcaagt 4020
cagaggtggc gaaacccgac aggactataa agataccagg cgtttccccc tggaagctcc 4080
ctcgtgcgct ctcctgttcc gaccctgccg cttaccggat acctgtccgc ctttctccct 4140
tcgggaagcg tggcgctttc tcaatgctca cgctgtaggt atctcagttc ggtgtaggtc 4200
gttcgctcca agctgggctg tgtgcacgaa ccccccgttc agcccgaccg ctgcgcctta 4260
tccggtaact atcgtcttga gtccaacccg gtaagacacg acttatcgcc actggcagca 4320
gccactggta acaggattag cagagcgagg tatgtaggcg gtgctacaga gttcttgaag 4380
tggtggccta actacggcta cactagaagg acagtatttg gtatctgcgc tctgctgaag 4440
ccagttacct tcggaaaaag agttggtagc tcttgatccg gcaaacaaac caccgctggt 4500
agcggtggtt tttttgtttg caagcagcag attacgcgca gaaaaaaagg atctcaagaa 4560
gatcctttga tcttttctac ggggtctgac gctcagtgga acgaaaactc acgttaaggg 4620
attttggtca tgagattatc aaaaaggatc ttcacctaga tccttttaaa ttaaaaatga 4680
agttttaaat caatctaaag tatatatgag taaacttggt ctgacagtta ccaatgctta 4740
atcagtgagg cacctatctc agcgatctgt ctatttcgtt catccatagt tgcctgactc 4800
cccgtcgtgt agataactac gatacgggag ggcttaccat ctggccccag tgctgcaatg 4860
ataccgcgag acccacgctc accggctcca gatttatcag caataaacca gccagccgga 4920
agggccgagc gcagaagtgg tcctgcaact ttatccgcct ccatccagtc tattaattgt 4980
tgccgggaag ctagagtaag tagttcgcca gttaatagtt tgcgcaacgt tgttgccatt 5040
gctacaggca tcgtggtgtc acgctcgtcg tttggtatgg cttcattcag ctccggttcc 5100
caacgatcaa ggcgagttac atgatccccc atgttgtgca aaaaagcggt tagctccttc 5160
ggtcctccga tcgttgtcag aagtaagttg gccgcagtgt tatcactcat ggttatggca 5220
gcactgcata attctcttac tgtcatgcca tccgtaagat gcttttctgt gactggtgag 5280
tactcaacca agtcattctg agaatagtgt atgcggcgac cgagttgctc ttgcccggcg 5340
tcaatacggg ataataccgc gccacatagc agaactttaa aagtgctcat cattggaaaa 5400
cgttcttcgg ggcgaaaact ctcaaggatc ttaccgctgt tgagatccag ttcgatgtaa 5460
```

```
cccactcgtg cacccaactg atcttcagca tcttttactt tcaccagcgt ttctgggtga 5520
gcaaaaacag gaaggcaaaa tgccgcaaaa aagggaataa gggcgacacg gaaatgttga 5580
atactcatac tcttcctttt tcaatattat tgaagcattt atcagggtta ttgtctcatg 5640
agcggataca tatttgaatg tatttagaaa aataaacaaa taggggttcc gcgcacattt 5700
ccccgaaaag tgccacctga cgtctaagaa accattatta tcatgacatt aacctataaa 5760
aataggcgta tcacgaggcc ctttcgtc                                    5788
```

<210> 40
<211> 48
<212> DNA
<213> Artificial Sequence

<220>
<223> Description of Artificial Sequence: Synthetic DNA primer

<400> 40
```
ggtcaggaat tctccggaat tggctagcct agagtccgtt acataact              48
```

<210> 41
<211> 63
<212> DNA
<213> Artificial Sequence

<220>
<223> Description of Artificial Sequence: Synthetic DNA primer

<400> 41
```
gaggactggc gcgccgagtg tggggttctt accctttta tagacctccc accgtacacg 60
cct                                                               63
```

<210> 42
<211> 76
<212> DNA
<213> Artificial Sequence

<220>
<223> Description of Artificial Sequence: Synthetic DNA primer

<400> 42
```
agatctccga ggcctgggac ccttcgaatt cgttaactga tcaacgcgtt ctagactaca 60
tggcggccgc gtgttt                                                 76
```

<210> 43
<211> 44
<212> DNA
<213> Artificial Sequence

```
<220>
<223> Description of Artificial Sequence: Synthetic DNA
      primer

<400> 43
gggggatcca gagctcgagc tttgaaaaac acgcggccgc catg                    44
```